# EUROPEAN PATENT SPECIFICATION

(11) **EP 1 185 664 B1**
(45) Date of publication and mention of the grant of the patent: **16.05.2007**
(21) Application number: 00938146.8
(22) Date of filing: 02.06.2000
(51) Int. Cl.: C12N 15/51, C07K 14/18, C07K 16/18, A61K 38/00, A61K 39/00

(54) **CLONED GENONE OF INFECTIOUS HEPATITIS C VIRUS OF GENOTYPE 2a AND USES THEREOF**
KLONIERTE GENOME VON INFEKTIÖSEN HEPATITIS C VIREN GENOTYP 2A UND DEREN VERWENDUNGEN
GENOME CLONE DU VIRUS DE L'HEPATITE C DE GENOTYPE 2A ET SON UTILISATION

(30) Priority: 04.06.1999 US 137693 P
(43) Date of publication of application: 13.03.2002
(73) Proprietor: THE GOVERNMENT OF THE UNITED STATES OF AMERICA as represented by the SECRETARY OF THE DEPARTMENT OF HEALTH AND HUMAN SERVICES, Rockville, MD 20852-3804 (US)
(72) Inventor: YANAGI, Masayuki, Rockville, MD 20852 (US); BUKH, Jens, Rockville, MD 20851 (US); EMERSON, Suzanne, U., Kensington, MD 20895 (US); PURCELL, Robert, H., Boyds, MD 20841 (US)
(74) Representative: Gervasi, Gemma
(86) International application number: PCT/US2000/015446
(87) International publication number: WO 2000/075338

(56) References cited:
- EP-A- 0 532 167
- WO-A-00/26418
- WO-A-91/15575
- H. OKAMOTO ET AL.: "Nucleotide sequence of the genomic RNA of hepatitis C virus isolated from a human carrier: comparison with reported isolates for conserved and divergent regions." JOURNAL OF GENERAL VIROLOGY, vol. 72, 1991, pages 2697-2704, XP000911895
- HAN J H ET AL: "GROUP SPECIFIC SEQUENCES AND CONSERVED SECONDARY STRUCTURE AT THE 3' END OF HCV GENOME AND ITS IMPLICATION FOR VIRAL REPLICATION" NUCLEIC ACIDS RESEARCH,OXFORD UNIVERSITY PRESS, SURREY,GB, vol. 20, no. 13, April 1992 (1992-04), page 3520 XP000938816 ISSN: 0305-1048
- M. YANAGI ET AL.: "Transcripts of a chimeric cDNA clone of Hepatitis C virus genotype 1b are infectious in vivo." VIROLOGY, vol. 244, 1998, pages 161-172, XP002149625 cited in the application
- OHNO T. ET AL: "New hepatitis C virus (HCV) genotyping system that allows for identification of HCV genotypes 1a, 1b, 2a, 2b, 3a, 3b, 4, 5a, and 6a." JOURNAL OF CLINICAL MICROBIOLOGY, (1997) 35/1 (201-207)., XP000911892
- HASHIMOTO M. ET AL: "Typing six major hepatitis C virus genotypes by polymerase chain reaction using primers derived from nucleotide sequences of the NS5 region." INTERNATIONAL HEPATOLOGY COMMUNICATIONS, (1996) 4/5 (263-267)., XP000911896
- YONG YUAN ZHANG ET AL: "Greater diversity of hepatitis C virus genotypes found in Hong Kong than in Mainland China." JOURNAL OF CLINICAL MICROBIOLOGY, (1995) 33/11 (2931-2934)., XP000911893
- FOX S A ET AL: "Rapid genotyping of hepatitis C virus isolates by dideoxy fingerprinting." JOURNAL OF VIROLOGICAL METHODS, (1995 MAY) 53 (1) 1-9., XP000911899
- M. YANAGI ET AL.: "Hepatitis C Virus: An infectious molecular clone of a second major genotype (2a) and lack of viability of intertypic 1a and 2a chimeras." VIROLOGY, vol. 262, 1999, pages 250-263, XP000911930
- DE FRANCESCO R. ET AL: "A zinc binding site in viral serine proteinases." BIOCHEMISTRY, (1996) 35/41 (13282-13287) , XP000981213
- STEMPNIAK M. ET AL: "The NS3 proteinase domain of hepatitis C virus is a zinc-containing enzyme." JOURNAL OF VIROLOGY, (1997) 71/4 (2881-2886), XP000981212
- Y.M. PARK ET AL.: "Monitoring antibody titers to recombinant core-NS3 fusion polypeptide is useful for evaluating hepatitis C virus infection and responses to interferon-alpha therapy" J. KOREAN MED. SCI., vol. 14, April 1999 (1999-04), pages 165-170, XP000980030
- L.M. MISON ET AL.: "Prevalence of hepatitis C virus and genotype distribution in an Australian volunteer blood donor population." TRANSFUSION, vol. 37, January 1997 (1997-01), pages 73-78, XP000981247
- WRIGHT-MINOGUE J. ET AL: "Cross-genotypic interaction between hepatitis C virus NS3 protease domains and NS4A cofactors." JOURNAL OF HEPATOLOGY, (2000) 32/3 (497-504). , XP000981249
- MARTIN J. ET AL: "In vitro effect of amantadine and interferon.alpha.- 2a on hepatitis C virus markers in cultured peripheral blood mononuclear cells from hepatitis C virus-infected patients." ANTIVIRAL RESEARCH, (1999) 42/1 (59-70). , XP000980547
- URUSHIHARA A. ET AL: "Changes in antibody titers to hepatitis C virus following interferon therapy for chronic infection." JOURNAL OF MEDICAL VIROLOGY, (1994) 42/4 (348-356). , XP000980020
- D.L. SALI ET AL.: "Serine protease of Hepatitis C virus expressed in insect cells as the NS3/4A complex" BIOCHEMISTRY, vol. 37, no. 10, 1998, pages 3392-3401, XP002159433
- P.L. CALVO ET AL.: "Hepatitis C virus heteroduplex tracking assay for genotype determination reveals diverging Genotype 2 isolates in Italian hemodialysis patients." JOURNAL OF CLINICAL MICROBIOLOGY, vol. 36, no. 1, January 1998 (1998-01), pages 227-233, XP000981214
- BUKH J ET AL: "At least 12 genotypes of hepatitis C virus predicted by sequence analysis of the putative E1 gene of isolates collected worldwide." PROCEEDINGS OF THE NATIONAL ACADEMY OF SCIENCES OF USA,US,NATIONAL ACADEMY OF SCIENCE. WASHINGTON, vol. 90, September 1994 (1994-09), pages 8234-8238, XP002159434 ISSN: 0027-8424 cited in the application
- P. SIMMONDS ET AL.: "Identification of genotypes of hepatitis C virus by sequence comparisons in the core, E1 and NS-5 regions." JOURNAL OF GENERAL VIROLOGY, vol. 75, 1994, pages 1053-1061, XP000979107
- L.J. VAN DOORN ET AL.: "Sequence analysis of hepatitis C virus genotypes 1 to 5 reveals multiple novel subtypes in the Benelux countries." JOURNAL OF GENERAL VIROLOGY., vol. 76, 1995, pages 1871-1876, XP000979102
- WU CHAODONG ET AL.: "Antibody response to E2 glycoprotein induced in mice by immunization with plasmid DNA containing sequence derived from a Chinese genotype III/2a isolate of hepatitis C virus." CHINESE MEDICAL JOURNAL, vol. 112, no. 2, February 1999 (1999-02), pages 166-168, XP000980092
- N. YUKI ET AL.: "Quantitative analysis of antibody to Hepatitis C virus Envelope 2 Glycoprotein in patients with chronic Hepatitis C virus infection." HEPTOLOGY, vol. 23, no. 5, May 1996 (1996-05), pages 947-952, XP000981263
- G. LONGOMBARDO ET AL.: "Immune response to an epitope of the NS4 protein of Hepatitis C virus in HCV-related disorders." CLINICAL IMMUNOLOGY AND IMMUNOPATHOLOGY, vol. 87, May 1998 (1998-05), pages 124-129, XP000981260
- F. FABRIZI ET AL.: "Hepatitis C virus genotpyes in chronic dialysis patients." NEPHROL. DIAL. TRANSPLANT., vol. 11, 1996, pages 679-683, XP000981328
- H-H. LIN ET AL.: "Serotypes, genotypes and levels of Hepatitis C Viremia in pregnant women in Taiwan." J. FORMOS MEDL ASSOC. , vol. 95, no. 6, 1996, pages 429-434, XP000981246
- M. DEVESA ET AL.: "Reduced antibody reactivity to Hepatitis C virus antigen in Hemodialysis patients coinfected with hepatitis B virus." CLINICAL AND DIAGNOSTIC LABORATORY IMMUNOLOGY, vol. 4, no. 6, November 1997 (1997-11), pages 639-642, XP000981261
- N. YUKI ET AL.: "Hepatitis C virus replicative levels and efficiency of genotyping by specific PCR and antibody assay." JOURNAL OF CLINICAL MICROBIOLOGY, vol. 35, no. 5, May 1997 (1997-05), pages 1184-1189, XP000981255
- Z-X.ZHANG ET AL.: "Evaluation of the multiple peptide assay for typing of antibodies to the Hepatitis C Virus: Relation to genomic typing by the Polymerase Chain Reaction." JOURNAL OF MEDICAL VIROLOGY, vol. 45, 1995, pages 50-55, XP000569306
- H. NOMURA ET AL.: "Interferon therapy and Hepatitis C virus." JOURNAL OF GASTROENTEROLOGY AND HEPATOLOGY, vol. 14, no. 1, January 1999 (1999-01), pages 85-89, XP000980021
- N. FURUSYO ET AL.: "Differences between interferon-alpha and -beta treatment for patients with chronic hepatitis C virus infection." DIGESTIVE DISEASES AND SCIENCES., vol. 44, no. 3, March 1999 (1999-03), pages 608-617, XP000981254
- G.B. YAO ET AL.: "Long-term efficacy of recombinant interferon alpha 2a in the treatment of chronic Hepatitis C: A randomized prospective study comparing two dose schedules in Chinese patients." HEPATO-GASTROENTEROLOGY, vol. 46, March 1999 (1999-03) - April 1999 (1999-04), pages 1059-1064, XP000981266
- M. MARTINOT-PEIGNOUX ET AL.: "Predictors of sustained response to alpha interferon therapy in chronic hepatitis C." JOURNAL OF HEPATOLOGY, vol. 29, no. 2, August 1998 (1998-08), pages 214-223, XP000980024
- W.M. LEE: "Therapy of Hepatitis C: Interferon Alfa-2a trials." HEPATOLOGY, vol. 26, 1997, pages 89S-95S, XP000981288
- K.L. LINDSAY : "Therapy of Hepatitis C: Overview" HEPATOLOGY, vol. 26, 1997, pages 71S-77S, XP000981298
- T. MARAKAMI ET AL.: "Mutations in Nonstructural protein 5A gene and response to interferon in Hepatitis C virus genotype 2 infection." HEPATOLOGY, vol. 30, October 1999 (1999-10), pages 1045-1053, XP000981333

### SEQUENCE LISTING

<110> Yanagi, Masayuki
   Emerson, Suzanne
   Bukh, Jens
   Purcell, Robert
<120> Cloned Genome of Infectious Hepatitis C Viruses of Genotype 2a and Uses Thereof
<130> 20264302PC
<140> TBA
   <141> 2000-06-02
<150> 60/137,693
   <151> 1999-06-04
<160> 39
<170> PatentIn Ver. 2.1
<210> 1
   <211> 9711
   <212> DNA
   <213> Hepatitis C virus
<400> 1
   acccgcccct aataggggcg acactccgcc atgaatcact cccctgtgag gaactactgt 60
   cttcacgcag aaagcgtcta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gccatttggg cgtgcccccg 240
   caagactgct agccgagtag cgttgggttg cgaaaggcct tgtggtactg cctgataggg 300
   tgcttgcgag tgccccggga ggtctcgtag accgtgcacc atgagcacaa atcctaaacc 360
   tcaaagaaaa accaaaagaa acaccaaccg tcgcccacaa gacgttaagt ttccgggcgg 420
   cggccagatc gttggcggag tatacttgtt gccgcgcagg ggccccaggt tgggtgtgcg 480
   cgcgacaagg aagacttcgg agcggtccca gccacgtgga aggcgccagc ccatccctaa 540
   agatcggcgc tccactggca aatcctgggg aaaaccagga tacccctggc ccctatacgg 600
   gaatgaggga ctcggctggg caggatggct cctgtccccc cgaggttccc gtccctcttg 660
   gggccccaat gacccccggc ataggtcgcg caacgtgggt aaggtcatcg ataccctaac 720
   gtgcggcttt gccgacctca tggggtacat ccctgtcgtg ggcgccccgc tcggcggcgt 780
   cgccagagct ctcgcgcatg gcgtgagagt cctggaggac ggggttaatt ttgcaacagg 840
   gaacttaccc ggttgctcct tttctatctt cttgctggcc ctgctgtcct gcatcaccac 900
   cccggtctcc gctgccgaag tgaagaacat cagtaccggc tacatggtga ctaacgactg 960
   caccaatgac agcattacct ggcagctcca ggctgctgtc ctccacgtcc ccgggtgcgt 1020
   cccgtgcgag aaagtgggga atgcatctca gtgctggata ccggtctcac cgaatgtggc 1080
   cgtgcagcgg cccggcgccc tcacgcaggg cttgcggacg cacatcgaca tggttgtgat 1140
   gtccgccacg ctctgctctg ccctctacgt gggggacctc tgcggtgggg tgatgctcgc 1200
   agcccaaatg ttcattgtct cgccgcagca ccactggttt gtccaagact gcaattgctc 1260
   catctaccct ggtaccatca ctggacaccg catggcatgg gacatgatga tgaactggtc 1320
   gcccacggct accatgatct tggcgtacgc gatgcgtgtc cccgaggtca ttatagacat 1380
   cattagcggg gctcattggg gcgtcatgtt cggcttggcc tacttctcta tgcagggagc 1440
   gtgggcgaaa gtcgttgtca tccttctgtt ggccgccggg gtggacgcgc gcacccatac 1500
   tgttgggggt tctgccgcgc agaccaccgg gcgcctcacc agcttatttg acatgggccc 1560
   caggcagaaa atccagctcg ttaacaccaa tggcagctgg cacatcaacc gcaccgccct 1620
   gaactgcaat gactccttgc acaccggctt tatcgcgtct ctgttctaca cccacagctt 1680
   caactcgtca ggatgtcccg aacgcatgtc cgcctgccgc agtatcgagg ccttccgggt 1740
   gggatggggc gccttgcaat atgaggataa tgtcaccaat ccagaggata tgagacccta 1800
   ttgctggcac tacccaccaa ggcagtgtgg cgtggtctcc gcgaagactg tgtgtggccc 1860
   agtgtactgt ttcaccccca gcccagtggt agtgggcacg accgacaggc ttggagcgcc 1920
   cacttacacg tggggggaga atgagacaga tgtcttccta ttgaacagca ctcgaccacc 1980
   gctggggtca tggttcggct gcacgtggat gaactcttct ggctacacca agacttgcgg 2040
   cgcaccaccc tgccgtacta gagctgactt caacgccagc acggacctgt tgtgccccac 2100
   ggactgtttt aggaagcatc ctgataccac ttacctcaaa tgcggctctg ggccctggct 2160
   cacgccaagg tgcctgatcg actaccccta caggctctgg cattacccct gcacagttaa 2220
   ctataccatc ttcaaaataa ggatgtatgt gggaggggtt gagcacaggc tcacggctgc 2280
   atgcaatttc actcgtgggg atcgttgcaa cttggaggac agagacagaa gtcaactgtc 2340
   tcctttgttg cactccacca cggaatgggc cattttacct tgctcttact cggacctgcc 2400
   cgccttgtcg actggtcttc tccacctcca ccaaaacatc gtggacgtac aattcatgta 2460
   tggcctatca cctgccctca caaaatacat cgtccgatgg gagtgggtaa tactcttatt 2520
   cctgctctta gcggacgcca gggtttgcgc ctgcttatgg atgctcatct tgttgggcca 2580
   ggccgaagca gcactagaga agctggtcat cttgcacgct gcgagcgcag ctagctgcaa 2640
   tggcttccta tattttgtca tctttttcgt ggctgcttgg tacatcaagg gtcgggtagt 2700
   ccccttagct acctattccc tcactggcct gtggtccttt agcctactgc tcctagcatt 2760
   gccccaacag gcttatgctt atgacgcatc tgtgcatggc cagataggag cggctctgct 2820
   ggtaatgatc actctcttta ctctcacccc cgggtataag acccttctca gccggttttt 2880
   gtggtggttg tgctatcttc tgaccctggg ggaagctatg gtccaggagt gggcaccacc 2940
   tatgcaggtg cgcggtggcc gtgatggcat catatgggcc gtcgccatat tctacccagg 3000
   tgtggtgttt gacataacca agtggctctt ggcggtgctt gggcctgctt acctcctaaa 3060
   aggtgctttg acgcgcgtgc cgtacttcgt cagggctcac gctctactga ggatgtgcac 3120
   catggcaagg catctcgcgg ggggcaggta cgtccagatg gcgctactag cccttggcag 3180
   gtggactggc acttacatct atgaccacct cacccctatg tcggattggg ctgctagtgg 3240
   cctgcgggac ctggcggtcg ccgttgagcc tatcatcttc agtccgatgg agaagaaagt 3300
   cattgtctgg ggagcggaga cagctgcttg tggggacatt ttacacggac ttcccgtgtc 3360
   cgcccgactt ggtcgggagg tcctccttgg cccagctgat ggctatacct ccaaggggtg 3420
   gagtcttctc gcccccatca ctgcttacgc ccagcagaca cgtggccttt tgggcaccat 3480
   agtggtgagc atgacggggc gcgacaagac agaacaggct ggggaaattc aggtcctgtc 3540
   cacagtcact cagtccttcc tcggaacatc catctcgggg gttttgtgga ctgtctacca 3600
   tggagctggc aacaagactc tggccggctc acggggtccg gtcacgcaga tgtactccag 3660
   tgctgagggg gacttagtag ggtggcccag cccccctggg actaaatctt tggagccgtg 3720
   cacgtgtgga gcggtcgacc tgtacctggt cacgcggaac gctgatgtca tcccggctcg 3780
   aagacgcggg gacaaacggg gagcgctact ctccccgaga cctctttcca ccttgaaggg 3840
   gtcctcagga ggcccggtgc tatgccccag gggccacgct gtcggagtct tccgggcagc 3900
   tgtgtgctct cggggcgtgg ctaagtccat agatttcatc cccgttgaga cactcgacat 3960
   cgtcacgcgg tcccccacct ttagtgacaa cagcacacca cctgctgtgc cccagaccta 4020
   tcaggtcggg tacttgcatg ccccgactgg cagtggaaag agcaccaaag ttcctgtcgc 4080
   atatgctgct caggggtata aagtgctagt gcttaatccc tcagtggctg ccaccctggg 4140
   gtttggggcg tacttgtcta aggcacatgg catcaatccc aacattagga ctggagtcag 4200
   gactgtgacg accggggcgc ccatcacgta ctccacatat ggcaaattcc tcgccgatgg 4260
   gggctgtgcg ggcggcgcct acgacatcat catatgtgat gaatgccatg ccgtggactc 4320
   taccaccatc cttggcatcg gaacagtcct tgatcaagca gagacagctg gggtcagact 4380
   aactgtgctg gctacagcta cgccccctgg gtcagtgaca accccccacc ccaacataga 4440
   ggaggtggcc cttgggcagg agggcgagat ccccttctat gggagggcga ttcccctgtc 4500
   ttacatcaag ggaggaagac atctgatctt ctgccattca aagaaaaagt gtgacgagct 4560
   cgcggcggcc cttcggggta tgggcttgaa ctcagtggca tactacagag ggttggacgt 4620
   ctccgtaata ccaactcagg gagacgtagt ggtcgtcgcc accgacgccc tcatgacagg 4680
   gtatactggg gactttgact ccgtgatcga ctgcaacgta gcggtcactc aagttgtaga 4740
   cttcagttta gaccccacat tcaccataac cacacagatt gtccctcaag acgctgtctc 4800
   acgtagccag cgccggggtc gcacgggtag gggaagactg ggcatttata ggtatgtttc 4860
   cactggtgag cgagcctcag gaatgtttga cagtgtagtg ctctgtgagt gctacgacgc 4920
   aggggccgca tggtatgagc tcacaccatc ggagaccacc gtcaggctca gggcgtattt 4980
   caacacgccc ggtttgcctg tgtgccaaga ccatcttgag ttttgggagg cagttttcac 5040
   cggcctcaca cacatagatg cccacttcct ttcccaaaca aagcaatcgg gggaaaattt 5100
   cgcatactta acagcctacc aggctacagt gtgcgctagg gccaaagccc cccccccgtc 5160
   ctgggacgtc atgtggaagt gtttgactcg actcaagccc acactcgtgg gccccacacc 5220
   tctcctgtac cgcttgggct ctgttaccaa cgaggtcacc ctcacacatc ccgtgacgaa 5280
   atacatcgcc acctgcatgc aagccgacct tgaggtcatg accagcacat gggtcttggc 5340
   agggggagtc ttggcggccg tcgccgcgta ttgcctggcg accgggtgtg tttgcatcat 5400
   cggccgcttg cacattaacc agcgagccgt cgttgcgccg gacaaggagg tcctctatga 5460
   ggcttttgat gagatggagg aatgtgcctc tagggcggct ctcattgaag aggggcagcg 5520
   gatagccgag atgctgaagt ccaagatcca aggcttattg cagcaagctt ccaaacaagc 5580
   tcaagacata caacccactg tgcaggcttc atggcccaag gtagaacaat tctgggccaa 5640
   acacatgtgg aacttcatta gcggcatcca atacctcgca ggactatcaa cactgccagg 5700
   gaaccctgca gtagcttcca tgatggcgtt cagtgccgcc ctcaccagtc cgctgtcaac 5760
   aagcaccact atccttctca acatttgggg gggctggcta gcatcccaaa ttgcaccacc 5820
   cgcgggggcc actggcttcg ttgtcagtgg cctagtggga gctgccgtag gcagtatagg 5880
   cttaggtaag gtgctagtgg acatcctggc agggtatggt gcgggcattt cgggggctct 5940
   cgtcgcattc aagatcatgt ctggcgagaa gccctccatg gaggatgtcg tcaacttgct 6000
   gcctggaatt ctgtctccgg gtgccttggt agtgggagtc atctgcgcgg ccattctgcg 6060
   ccgacacgtg ggaccggggg aaggcgccgt ccaatggatg aatagactca ttgcctttgc 6120
   ttccagagga aatcacgtcg cccccaccca ctacgtgacg gagtcggatg cgtcgcagcg 6180
   tgtgacccaa ctacttggct cccttaccat aaccagcctg ctcagaagac tccacaactg 6240
   gattactgag gactgcccca tcccatgcgg cggctcgtgg ctccgcgatg tgtgggactg 6300
   ggtttgcacc atcctaacag actttaaaaa ttggctgacc tccaaattat tcccaaagat 6360
   gcccggcctc ccctttgtct cctgtcaaaa ggggtacaag ggcgtgtggg ccggcactgg 6420
   catcatgacc acacggtgtc cttgcggcgc caatatctct ggcaatgtcc gcttgggctc 6480
   catgagaatc acggggccta agacctgcat gaatatctgg caggggacct ttcctatcaa 6540
   ttgttacacg gagggccagt gcgtgccgaa acccgcgcca aactttaagg tcgccatctg 6600
   gagggtggcg gcctcagagt acgcggaggt gacgcagcac gggtcatacc actacataac 6660
   aggactcacc actgataact tgaaagtccc ctgccaacta ccctctcccg agttcttttc 6720
   ctgggtggac ggagtgcaga tccataggtt tgcccccaca ccgaagccgt ttttccggga 6780
   tgaggtctcg ttctgcgttg ggcttaattc atttgtcgtc gggtcccagc ttccttgcga 6840
   ccctgaaccc gacacagacg tattgatgtc catgctaaca gatccatctc atatcacggc 6900
   ggagactgca gcgcggcgtt tagcgcgggg gtcaccccca tccgaggcaa gctcctcggc 6960
   gagccagcta tcggcaccat cgctgcgagc cacctgcacc acccacggca aagcctatga 7020
   tgtggacatg gtggatgcta acctgttcat ggggggcgat gtgactcgga tagagtctgg 7080
   gtccaaagtg gtcgttctgg actctctcga cccaatggtc gaagaaagga gcgaccttga 7140
   gccttcgata ccatcagaat acatgctccc caagaagagg ttcccaccag ctttaccggc 7200
   ctgggcacgg cctgattaca acccaccgct tgtggaatcg tggaaaaggc cagattacca 7260
   accggccact gttgcgggct gtgctctccc tcctcctagg aaaaccccga cgcctccccc 7320
   aaggaggcgc cggacagtgg gcctaagtga ggactccata ggagatgccc ttcaacagct 7380
   ggccattaag tcctttggcc agcccccccc aagcggcgat tcaggccttt ccacgggggc 7440
   gggcgctgcc gattccggca gtcagacgcc tcctgatgag ttggcccttt cggagacagg 7500
   ttccatctct tccatgcccc ccctcgaggg ggagcttgga gatccagacc tggagcctga 7560
   gcaggtagag ccccaacccc ccccccaggg gggggtggca gctcccggct cggactcggg 7620
   gtcctggtct acttgctccg aggaggacga ctccgtcgtg tgctgctcca tgtcatactc 7680
   ctggaccggg gctctaataa ctccttgtag tcccgaagag gagaagttac cgattaaccc 7740
   cttgagcaac tccctgttgc gatatcacaa caaggtgtac tgtaccacaa caaagagcgc 7800
   ctcactaagg gctaaaaagg taacttttga taggatgcaa gtgctcgact cctactacga 7860
   ctcagtctta aaggacatta agctagcggc ctccaaggtc accgcaaggc tcctcaccat 7920
   ggaggaggct tgccagttaa ccccacccca ttctgcaaga tctaaatatg ggtttggggc 7980
   taaggaggtc cgcagcttgt ccgggagggc cgttaaccac atcaagtccg tgtggaagga 8040
   cctcctggag gactcagaaa caccaattcc cacaaccatt atggccaaaa atgaggtgtt 8100
   ctgcgtggac cccaccaagg ggggcaagaa agcagctcgc cttatcgttt accctgacct 8160
   cggcgtcagg gtctgcgaga agatggccct ttatgacatt acacaaaaac ttcctcaggc 8220
   ggtgatgggg gcttcttatg gattccagta ttcccccgct cagcgggtag agtttctctt 8280
   gaaagcatgg gcggaaaaga aggaccctat gggtttttcg tatgataccc gatgctttga 8340
   ctcaaccgtc actgagagag acatcaggac tgaggagtcc atatatcggg cctgctcctt 8400
   gcccgaggag gcccacactg ccatacactc gctaactgag agactttacg tgggagggcc 8460
   tatgttcaac agcaagggcc aaacctgcgg gtacaggcgt tgccgcgcca gcggggtgct 8520
   caccactagc atggggaaca ccatcacatg ctacgtgaaa gccttagcgg cttgtaaagc 8580
   tgcagggata atcgcgccca caatgctggt atgcggcgat gacttggttg tcatctcaga 8640
   aagccagggg accgaggagg acgagcggaa cctgagagcc ttcacggagg ctatgaccag 8700
   gtattctgcc cctcctggtg acccccccag accggagtat gatctggagc tgataacatc 8760
   ttgctcctca aatgtgtctg tggcgctggg cccacaaggc cgccgcagat actacctgac 8820
   cagagaccct accactccaa tcgcccgggc tgcctgggaa acagttagac actcccctgt 8880
   caattcatgg ctgggaaaca tcatccagta cgccccgacc atatgggctc gcatggtcct 8940
   gatgacacac ttcttctcca ttctcatggc tcaagacacg ctggaccaga acctcaactt 9000
   tgagatgtac ggagcggtgt actccgtgag tcccttggac ctcccagcta taattgaaag 9060
   gttacatggg cttgacgctt tttctctgca cacatacact ccccacgaac tgacacgggt 9120
   ggcttcagcc ctcagaaaac ttggggcgcc acccctcaga gcgtggaaga gccgggcacg 9180
   tgcagtcagg gcgtccctca tctcccgtgg ggggagagcg gccgtttgcg gtcgatatct 9240
   cttcaattgg gcggtgaaga ccaagctcaa actcactcca ttgccggaag cgcgcctcct 9300
   ggatttatcc agctggttca ccgtcggcgc cggcgggggc gacatttatc acagcgtgtc 9360
   gcgtgcccga ccccgcttat tgctctttgg cctactccta ctttttgtag gggtaggcct 9420
   tttcctactc cccgctcggt agagcggcac acattagcta cactccatag ctaactgtcc 9480
   cttttttttt tttttttttt tttttttttt tttttttttt tttttttttt tttttttttt 9540
   tttttttttt tttttttttt tttttctttt tttctctttt ccttctttct taccttattt 9600
   tactttcttt cctggtggct ccatcttagc cctagtcacg gctagctgtg aaaggtccgt 9660
   gagccgcatg actgcagaga gtgccgtaac tggtctctct gcagatcatg t 9711
<210> 2
   <211> 3033
   <212> PRT
   <213> Hepatitis C virus
<400> 2
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Asn Asp Pro
               100                 105                 110
   Arg His Arg Ser Arg Asn Val Gly Lys Val Ile Asp Thr Leu Thr Cys
           115                 120                 125
   Gly Phe Ala Asp Leu Met Gly Tyr Ile Pro Val Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Phe Ala Thr Gly Asn Leu Pro Gly Cys Ser Phe Ser Ile
                   165                 170                 175
   Phe Leu Leu Ala Leu Leu Ser Cys Ile Thr Thr Pro Val Ser Ala Ala
               180                 185                 190
   Glu Val Lys Asn Ile Ser Thr Gly Tyr Met Val Thr Asn Asp Cys Thr
           195                 200                 205
   Asn Asp Ser Ile Thr Trp Gln Leu Gln Ala Ala Val Leu His Val Pro
       210                 215                 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Ile Leu Leu Phe Leu Leu Leu Ala Asp Ala Arg Val Cys
   725 730 735
   Ala Cys Leu Trp Met Leu Ile Leu Leu Gly Gln Ala Glu Ala Ala Leu
   740 745 750
   Glu Lys Leu Val Ile Leu His Ala Ala Ser Ala Ala Ser Cys Asn Gly
   755 760 765
   Phe Leu Tyr Phe Val Ile Phe Phe Val Ala Ala Trp Tyr Ile Lys Gly
   770 775 780
   Arg Val Val Pro Leu Ala Thr Tyr Ser Leu Thr Gly Leu Trp Ser Phe
   785 790 795 800
   Ser Leu Leu Leu Leu Ala Leu Pro Gln Gln Ala Tyr Ala Tyr Asp Ala
   805 810 815
   Ser Val His Gly Gln Ile Gly Ala Ala Leu Leu Val Met Ile Thr Leu
   820 825 830
   Phe Thr Leu Thr Pro Gly Tyr Lys Thr Leu Leu Ser Arg Phe Leu Trp
   835 840 845
   Trp Leu Cys Tyr Leu Leu Thr Leu Gly Glu Ala Met Val Gln Glu Trp
   850 855 860
   Ala Pro Pro Met Gln Val Arg Gly Gly Arg Asp Gly Ile Ile Trp Ala
   865 870 875 880
   Val Ala Ile Phe Tyr Pro Gly Val Val Phe Asp Ile Thr Lys Trp Leu
   885 890 895
   Leu Ala Val Leu Gly Pro Ala Tyr Leu Leu Lys Gly Ala Leu Thr Arg
   900 905 910
   Val Pro Tyr Phe Val Arg Ala His Ala Leu Leu Arg Met Cys Thr Met
   915 920 925
   Ala Arg His Leu Ala Gly Gly Arg Tyr Val Gln Met Ala Leu Leu Ala
   930 935 940
   Leu Gly Arg Trp Thr Gly Thr Tyr Ile Tyr Asp His Leu Thr Pro Met
   945 950 955 960
   Ser Asp Trp Ala Ala Ser Gly Leu Arg Asp Leu Ala Val Ala Val Glu
   965 970 975
   Pro Ile Ile Phe Ser Pro Met Glu Lys Lys Val Ile Val Trp Gly Ala
   980 985 990
   Glu Thr Ala Ala Cys Gly Asp Ile Leu His Gly Leu Pro Val Ser Ala
   995 1000 1005
   Arg Leu Gly Arg Glu Val Leu Leu Gly Pro Ala Asp Gly Tyr Thr Ser
   1010 1015 1020
   Lys Gly Trp Ser Leu Leu Ala Pro Ile Thr Ala Tyr Ala Gln Gln Thr
   1025 1030 1035 1040
   Arg Gly Leu Leu Gly Thr Ile Val Val Ser Met Thr Gly Arg Asp Lys
   1045 1050 1055
   Thr Glu Gln Ala Gly Glu Ile Gln Val Leu Ser Thr Val Thr Gln Ser
   1060 1065 1070
   Phe Leu Gly Thr Ser Ile Ser Gly Val Leu Trp Thr Val Tyr His Gly
   1075 1080 1085
   Ala Gly Asn Lys Thr Leu Ala Gly Ser Arg Gly Pro Val Thr Gln Met
   1090 1095 1100
   Tyr Ser Ser Ala Glu Gly Asp Leu Val Gly Trp Pro Ser Pro Pro Gly
   1105 1110 1115 1120
   Thr Lys Ser Leu Glu Pro Cys Thr Cys Gly Ala Val Asp Leu Tyr Leu
   1125 1130 1135
   Val Thr Arg Asn Ala Asp Val Ile Pro Ala Arg Arg Arg Gly Asp Lys
   1140 1145 1150
   Arg Gly Ala Leu Leu Ser Pro Arg Pro Leu Ser Thr Leu Lys Gly Ser
   1155 1160 1165
   Ser Gly Gly Pro Val Leu Cys Pro Arg Gly His Ala Val Gly Val Phe
   1170 1175 1180
   Arg Ala Ala Val Cys Ser Arg Gly Val Ala Lys Ser Ile Asp Phe Ile
   1185 1190 1195 1200
   Pro Val Glu Thr Leu Asp Ile Val Thr Arg Ser Pro Thr Phe Ser Asp
   1205 1210 1215
   Asn Ser Thr Pro Pro Ala Val Pro Gln Thr Tyr Gln Val Gly Tyr Leu
   1220 1225 1230
   His Ala Pro Thr Gly Ser Gly Lys Ser Thr Lys Val Pro Val Ala Tyr
   1235 1240 1245
   Ala Ala Gln Gly Tyr Lys Val Leu Val Leu Asn Pro Ser Val Ala Ala
   1250 1255 1260
   Thr Leu Gly Phe Gly Ala Tyr Leu Ser Lys Ala His Gly Ile Asn Pro
   1265 1270 1275 1280
   Asn Ile Arg Thr Gly Val Arg Thr Val Thr Thr Gly Ala Pro Ile Thr
   1285 1290 1295
   Tyr Ser Thr Tyr Gly Lys Phe Leu Ala Asp Gly Gly Cys Ala Gly Gly
   1300 1305 1310
   Ala Tyr Asp Ile Ile Ile Cys Asp Glu Cys His Ala Val Asp Ser Thr
   1315 1320 1325
   Thr Ile Leu Gly Ile Gly Thr Val Leu Asp Gln Ala Glu Thr Ala Gly
   1330 1335 1340
   Val Arg Leu Thr Val Leu Ala Thr Ala Thr Pro Pro Gly Ser Val Thr
   1345 1350 1355 1360
   Thr Pro His Pro Asn Ile Glu Glu Val Ala Leu Gly Gln Glu Gly Glu
   1365 1370 1375
   Ile Pro Phe Tyr Gly Arg Ala Ile Pro Leu Ser Tyr Ile Lys Gly Gly
   1380 1385 1390
   Arg His Leu Ile Phe Cys His Ser Lys Lys Lys Cys Asp Glu Leu Ala
   1395 1400 1405
   Ala Ala Leu Arg Gly Met Gly Leu Asn Ser Val Ala Tyr Tyr Arg Gly
   1410 1415 1420
   Leu Asp Val Ser Val Ile Pro Thr Gln Gly Asp Val Val Val Val Ala
   1425 1430 1435 1440
   Thr Asp Ala Leu Met Thr Gly Tyr Thr Gly Asp Phe Asp Ser Val Ile
   1445 1450 1455
   Asp Cys Asn Val Ala Val Thr Gln Val Val Asp Phe Ser Leu Asp Pro
   1460 1465 1470
   Thr Phe Thr Ile Thr Thr Gln Ile Val Pro Gln Asp Ala Val Ser Arg
   1475 1480 1485
   Ser Gln Arg Arg Gly Arg Thr Gly Arg Gly Arg Leu Gly Ile Tyr Arg
   1490 1495 1500
   Tyr Val Ser Thr Gly Glu Arg Ala Ser Gly Met Phe Asp Ser Val Val
   1505 1510 1515 1520
   Leu Cys Glu Cys Tyr Asp Ala Gly Ala Ala Trp Tyr Glu Leu Thr Pro
   1525 1530 1535
   Ser Glu Thr Thr Val Arg Leu Arg Ala Tyr Phe Asn Thr Pro Gly Leu
   1540 1545 1550
   Pro Val Cys Gln Asp His Leu Glu Phe Trp Glu Ala Val Phe Thr Gly
   1555 1560 1565
   Leu Thr His Ile Asp Ala His Phe Leu Ser Gln Thr Lys Gln Ser Gly
   1570 1575 1580
   Glu Asn Phe Ala Tyr Leu Thr Ala Tyr Gln Ala Thr Val Cys Ala Arg
   1585 1590 1595 1600
   Ala Lys Ala Pro Pro Pro Ser Trp Asp Val Met Trp Lys Cys Leu Thr
   1605 1610 1615
   Arg Leu Lys Pro Thr Leu Val Gly Pro Thr Pro Leu Leu Tyr Arg Leu
   1620 1625 1630
   Gly Ser Val Thr Asn Glu Val Thr Leu Thr His Pro Val Thr Lys Tyr
   1635 1640 1645
   Ile Ala Thr Cys Met Gln Ala Asp Leu Glu Val Met Thr Ser Thr Trp
   1650 1655 1660
   Val Leu Ala Gly Gly Val Leu Ala Ala Val Ala Ala Tyr Cys Leu Ala
   1665 1670 1675 1680
   Thr Gly Cys Val Cys Ile Ile Gly Arg Leu His Ile Asn Gln Arg Ala
   1685 1690 1695
   Val Val Ala Pro Asp Lys Glu Val Leu Tyr Glu Ala Phe Asp Glu Met
   1700 1705 1710
   Glu Glu Cys Ala Ser Arg Ala Ala Leu Ile Glu Glu Gly Gln Arg Ile
   1715 1720 1725
   Ala Glu Met Leu Lys Ser Lys Ile Gln Gly Leu Leu Gln Gln Ala Ser
   1730 1735 1740
   Lys Gln Ala Gln Asp Ile Gln Pro Thr Val Gln Ala Ser Trp Pro Lys
   1745 1750 1755 1760
   Val Glu Gln Phe Trp Ala Lys His Met Trp Asn Phe Ile Ser Gly Ile
   1765 1770 1775
   Gln Tyr Leu Ala Gly Leu Ser Thr Leu Pro Gly Asn Pro Ala Val Ala
   1780 1785 1790
   Ser Met Met Ala Phe Ser Ala Ala Leu Thr Ser Pro Leu Ser Thr Ser
   1795 1800 1805
   Thr Thr Ile Leu Leu Asn Ile Leu Gly Gly Trp Leu Ala Ser Gln Ile
   1810 1815 1820
   Ala Pro Pro Ala Gly Ala Thr Gly Phe Val Val Ser Gly Leu Val Gly
   1825 1830 1835 1840
   Ala Ala Val Gly Ser Ile Gly Leu Gly Lys Val Leu Val Asp Ile Leu
   1845 1850 1855
   Ala Gly Tyr Gly Ala Gly Ile Ser Gly Ala Leu Val Ala Phe Lys Ile
   1860 1865 1870
   Met Ser Gly Glu Lys Pro Ser Met Glu Asp Val Val Asn Leu Leu Pro
   1875 1880 1885
   Gly Ile Leu Ser Pro Gly Ala Leu Val Val Gly Val Ile Cys Ala Ala
   1890 1895 1900
   Ile Leu Arg Arg His Val Gly Pro Gly Glu Gly Ala Val Gln Trp Met
   1905 1910 1915 1920
   Asn Arg Leu Ile Ala Phe Ala Ser Arg Gly Asn His Val Ala Pro Thr
   1925 1930 1935
   His Tyr Val Thr Glu Ser Asp Ala Ser Gln Arg Val Thr Gln Leu Leu
   1940 1945 1950
   Gly Ser Leu Thr Ile Thr Ser Leu Leu Arg Arg Leu His Asn Trp Ile
   1955 1960 1965
   Thr Glu Asp Cys Pro Ile Pro Cys Gly Gly Ser Trp Leu Arg Asp Val
   1970 1975 1980
   Trp Asp Trp Val Cys Thr Ile Leu Thr Asp Phe Lys Asn Trp Leu Thr
   1985 1990 1995 2000
   Ser Lys Leu Phe Pro Lys Met Pro Gly Leu Pro Phe Val Ser Cys Gln
   2005 2010 2015
   Lys Gly Tyr Lys Gly Val Trp Ala Gly Thr Gly Ile Met Thr Thr Arg
   2020 2025 2030
   Cys Pro Cys Gly Ala Asn Ile Ser Gly Asn Val Arg Leu Gly Ser Met
   2035 2040 2045
   Arg Ile Thr Gly Pro Lys Thr Cys Met Asn Ile Trp Gln Gly Thr Phe
   2050 2055 2060
   Pro Ile Asn Cys Tyr Thr Glu Gly Gln Cys Val Pro Lys Pro Ala Pro
   2065 2070 2075 2080
   Asn Phe Lys Val Ala Ile Trp Arg Val Ala Ala Ser Glu Tyr Ala Glu
   2085 2090 2095
   Val Thr Gln His Gly Ser Tyr His Tyr Ile Thr Gly Leu Thr Thr Asp
   2100 2105 2110
   Asn Leu Lys Val Pro Cys Gln Leu Pro Ser Pro Glu Phe Phe Ser Trp
   2115 2120 2125
   Val Asp Gly Val Gln Ile His Arg Phe Ala Pro Thr Pro Lys Pro Phe
   2130 2135 2140
   Phe Arg Asp Glu Val Ser Phe Cys Val Gly Leu Asn Ser Phe Val Val
   2145 2150 2155 2160
   Gly Ser Gln Leu Pro Cys Asp Pro Glu Pro Asp Thr Asp Val Leu Met
   2165 2170 2175
   Ser Met Leu Thr Asp Pro Ser His Ile Thr Ala Glu Thr Ala Ala Arg
   2180 2185 2190
   Arg Leu Ala Arg Gly Ser Pro Pro Ser Glu Ala Ser Ser Ser Ala Ser
   2195 2200 2205
   Gln Leu Ser Ala Pro Ser Leu Arg Ala Thr Cys Thr Thr His Gly Lys
   2210 2215 2220
   Ala Tyr Asp Val Asp Met Val Asp Ala Asn Leu Phe Met Gly Gly Asp
   2225 2230 2235 2240
   Val Thr Arg Ile Glu Ser Gly Ser Lys Val Val Val Leu Asp Ser Leu
   2245 2250 2255
   Asp Pro Met Val Glu Glu Arg Ser Asp Leu Glu Pro Ser Ile Pro Ser
   2260 2265 2270
   Glu Tyr Met Leu Pro Lys Lys Arg Phe Pro Pro Ala Leu Pro Ala Trp
   2275 2280 2285
   Ala Arg Pro Asp Tyr Asn Pro Pro Leu Val Glu Ser Trp Lys Arg Pro
   2290 2295 2300
   Asp Tyr Gln Pro Ala Thr Val Ala Gly Cys Ala Leu Pro Pro Pro Arg
   2305 2310 2315 2320
   Lys Thr Pro Thr Pro Pro Pro Arg Arg Arg Arg Thr Val Gly Leu Ser
   2325 2330 2335
   Glu Asp Ser Ile Gly Asp Ala Leu Gln Gln Leu Ala Ile Lys Ser Phe
   2340 2345 2350
   Gly Gln Pro Pro Pro Ser Gly Asp Ser Gly Leu Ser Thr Gly Ala Gly
   2355 2360 2365
   Ala Ala Asp Ser Gly Ser Gln Thr Pro Pro Asp Glu Leu Ala Leu Ser
   2370 2375 2380
   Glu Thr Gly Ser Ile Ser Ser Met Pro Pro Leu Glu Gly Glu Leu Gly
   2385 2390 2395 2400
   Asp Pro Asp Leu Glu Pro Glu Gln Val Glu Pro Gln Pro Pro Pro Gln
   2405 2410 2415
   Gly Gly Val Ala Ala Pro Gly Ser Asp Ser Gly Ser Trp Ser Thr Cys
   2420 2425 2430
   Ser Glu Glu Asp Asp Ser Val Val Cys Cys Ser Met Ser Tyr Ser Trp
   2435 2440 2445
   Thr Gly Ala Leu Ile Thr Pro Cys Ser Pro Glu Glu Glu Lys Leu Pro
   2450 2455 2460
   Ile Asn Pro Leu Ser Asn Ser Leu Leu Arg Tyr His Asn Lys Val Tyr
   2465 2470 2475 2480
   Cys Thr Thr Thr Lys Ser Ala Ser Leu Arg Ala Lys Lys Val Thr Phe
   2485 2490 2495
   Asp Arg Met Gln Val Leu Asp Ser Tyr Tyr Asp Ser Val Leu Lys Asp
   2500 2505 2510
   Ile Lys Leu Ala Ala Ser Lys Val Thr Ala Arg Leu Leu Thr Met Glu
   2515 2520 2525
   Glu Ala Cys Gln Leu Thr Pro Pro His Ser Ala Arg Ser Lys Tyr Gly
   2530 2535 2540
   Phe Gly Ala Lys Glu Val Arg Ser Leu Ser Gly Arg Ala Val Asn His
   2545 2550 2555 2560
   Ile Lys Ser Val Trp Lys Asp Leu Leu Glu Asp Ser Glu Thr Pro Ile
   2565 2570 2575
   Pro Thr Thr Ile Met Ala Lys Asn Glu Val Phe Cys Val Asp Pro Thr
   2580 2585 2590
   Lys Gly Gly Lys Lys Ala Ala Arg Leu Ile Val Tyr Pro Asp Leu Gly
   2595 2600 2605
   Val Arg Val Cys Glu Lys Met Ala Leu Tyr Asp Ile Thr Gln Lys Leu
   2610 2615 2620
   Pro Gln Ala Val Met Gly Ala Ser Tyr Gly Phe Gln Tyr Ser Pro Ala
   2625 2630 2635 2640
   Gln Arg Val Glu Phe Leu Leu Lys Ala Trp Ala Glu Lys Lys Asp Pro
   2645 2650 2655
   Met Gly Phe Ser Tyr Asp Thr Arg Cys Phe Asp Ser Thr Val Thr Glu
   2660 2665 2670
   Arg Asp Ile Arg Thr Glu Glu Ser Ile Tyr Arg Ala Cys Ser Leu Pro
   2675 2680 2685
   Glu Glu Ala His Thr Ala Ile His Ser Leu Thr Glu Arg Leu Tyr Val
   2690 2695 2700
   Gly Gly Pro Met Phe Asn Ser Lys Gly Gln Thr Cys Gly Tyr Arg Arg
   2705 2710 2715 2720
   Cys Arg Ala Ser Gly Val Leu Thr Thr Ser Met Gly Asn Thr Ile Thr
   2725 2730 2735
   Cys Tyr Val Lys Ala Leu Ala Ala Cys Lys Ala Ala Gly Ile Ile Ala
   2740 2745 2750
   Pro Thr Met Leu Val Cys Gly Asp Asp Leu Val Val Ile Ser Glu Ser
   2755 2760 2765
   Gln Gly Thr Glu Glu Asp Glu Arg Asn Leu Arg Ala Phe Thr Glu Ala
   2770 2775 2780
   Met Thr Arg Tyr Ser Ala Pro Pro Gly Asp Pro Pro Arg Pro Glu Tyr
   2785 2790 2795 2800
   Asp Leu Glu Leu Ile Thr Ser Cys Ser Ser Asn Val Ser Val Ala Leu
   2805 2810 2815
   Gly Pro Gln Gly Arg Arg Arg Tyr Tyr Leu Thr Arg Asp Pro Thr Thr
   2820 2825 2830
   Pro Ile Ala Arg Ala Ala Trp Glu Thr Val Arg His Ser Pro Val Asn
   2835 2840 2845
   Ser Trp Leu Gly Asn Ile Ile Gln Tyr Ala Pro Thr Ile Trp Ala Arg
   2850 2855 2860
   Met Val Leu Met Thr His Phe Phe Ser Ile Leu Met Ala Gln Asp Thr
   2865 2870 2875 2880
   Leu Asp Gln Asn Leu Asn Phe Glu Met Tyr Gly Ala Val Tyr Ser Val
   2885 2890 2895
   Ser Pro Leu Asp Leu Pro Ala Ile Ile Glu Arg Leu His Gly Leu Asp
   2900 2905 2910
   Ala Phe Ser Leu His Thr Tyr Thr Pro His Glu Leu Thr Arg Val Ala
   2915 2920 2925
   Ser Ala Leu Arg Lys Leu Gly Ala Pro Pro Leu Arg Ala Trp Lys Ser
   2930 2935 2940
   Arg Ala Arg Ala Val Arg Ala Ser Leu Ile Ser Arg Gly Gly Arg Ala
   2945 2950 2955 2960
   Ala Val Cys Gly Arg Tyr Leu Phe Asn Trp Ala Val Lys Thr Lys Leu
   2965 2970 2975
   Lys Leu Thr Pro Leu Pro Glu Ala Arg Leu Leu Asp Leu Ser Ser Trp
   2980 2985 2990
   Phe Thr Val Gly Ala Gly Gly Gly Asp Ile Tyr His Ser Val Ser Arg
   2995 3000 3005
   Ala Arg Pro Arg Leu Leu Leu Phe Gly Leu Leu Leu Leu Phe Val Gly
   3010 3015 3020
   Val Gly Leu Phe Leu Leu Pro Ala Arg
   3025 3030
<210> 3
   <211> 9611
   <212> DNA
   <213> Hepatitis C virus
<400> 3
<210> 4
   <211> 3015
   <212> PRT
   <213> Hepatitis C virus
<400> 4
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Asn Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
   195 200 205
   Asn Asp Ser Ile Thr Trp Gln Leu Gln Ala Ala Val Leu His Val Pro
   210 215 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Ile Leu Leu Phe Leu Leu Leu Ala Asp Ala Arg Val Cys
   725 730 735
   Ala Cys Leu Trp Met Leu Ile Leu Leu Gly Gln Ala Glu Ala Ala Leu
   740 745 750
   Glu Lys Leu Val Ile Leu His Ala Ala Ser Ala Ala Ser Cys Asn Gly
   755 760 765
   Phe Leu Tyr Phe Val Ile Phe Phe Val Ala Ala Trp Tyr Ile Lys Gly
   770 775 780
   Arg Val Val Pro Leu Ala Thr Tyr Ser Leu Thr Gly Leu Trp Ser Phe
   785 790 795 800
   Ser Leu Leu Leu Leu Ala Leu Pro Gln Gln Ala Tyr Ala Leu Asp Thr
   805 810 815
   Glu Val Ala Ala Ser Cys Gly Gly Val Val Leu Val Gly Leu Met Ala
   820 825 830
   Leu Thr Leu Ser Pro Tyr Tyr Lys Arg Tyr Ile Ser Trp Cys Met Trp
   835 840 845
   Trp Leu Gln Tyr Phe Leu Thr Arg Val Glu Ala Gln Leu His Val Trp
   850 855 860
   Val Pro Pro Leu Asn Val Arg Gly Gly Arg Asp Ala Val Ile Leu Leu
   865 870 875 880
   Met Cys Val Val His Pro Thr Leu Val Phe Asp Ile Thr Lys Leu Leu
   885 890 895
   Leu Ala Ile Phe Gly Pro Leu Trp Ile Leu Gln Ala Ser Leu Leu Lys
   900 905 910
   Val Pro Tyr Phe Val Arg Val Gln Gly Leu Leu Arg Ile Cys Ala Leu
   915 920 925
   Ala Arg Lys Ile Ala Gly Gly His Tyr Val Gln Met Ala Ile Ile Lys
   930 935 940
   Leu Gly Ala Leu Thr Gly Thr Tyr Val Tyr Asn His Leu Thr Pro Leu
   945 950 955 960
   Arg Asp Trp Ala His Asn Gly Leu Arg Asp Leu Ala Val Ala Val Glu
   965 970 975
   Pro Val Val Phe Ser Arg Met Glu Thr Lys Leu Ile Thr Trp Gly Ala
   980 985 990
   Asp Thr Ala Ala Cys Gly Asp Ile Ile Asn Gly Leu Pro Val Ser Ala
   995 1000 1005
   Arg Arg Gly Gln Glu Ile Leu Leu Gly Pro Ala Asp Gly Met Val Ser
   1010 1015 1020
   Lys Gly Trp Arg Leu Leu Ala Pro Ile Thr Ala Tyr Ala Gln Gln Thr
   1025 1030 1035 1040
   Arg Gly Leu Leu Gly Cys Ile Ile Thr Ser Leu Thr Gly Arg Asp Lys
   1045 1050 1055
   Asn Gln Val Glu Gly Glu Val Gln Ile Val Ser Thr Ala Thr Gln Thr
   1060 1065 1070
   Phe Leu Ala Thr Cys Ile Asn Gly Val Cys Trp Thr Val Tyr His Gly
   1075 1080 1085
   Ala Gly Thr Arg Thr Ile Ala Ser Pro Lys Gly Pro Val Ile Gln Met
   1090 1095 1100
   Tyr Thr Asn Val Asp Gln Asp Leu Val Gly Trp Pro Ala Pro Gln Gly
   1105 1110 1115 1120
   Ser Arg Ser Leu Thr Pro Cys Thr Cys Gly Ser Ser Asp Leu Tyr Leu
   1125 1130 1135
   Val Thr Arg His Ala Asp Val Ile Pro Val Arg Arg Arg Gly Asp Ser
   1140 1145 1150
   Arg Gly Ser Leu Leu Ser Pro Arg Pro Ile Ser Tyr Leu Lys Gly Ser
   1155 1160 1165
   Ser Gly Gly Pro Leu Leu Cys Pro Ala Gly His Ala Val Gly Leu Phe
   1170 1175 1180
   Arg Ala Ala Val Cys Thr Arg Gly Val Ala Lys Ala Val Asp Phe Ile
   1185 1190 1195 1200
   Pro Val Glu Asn Leu Gly Thr Thr Met Arg Ser Pro Val Phe Thr Asp
   1205 1210 1215
   Asn Ser Ser Pro Pro Ala Val Pro Gln Ser Phe Gln Val Ala His Leu
   1220 1225 1230
   His Ala Pro Thr Gly Ser Gly Lys Ser Thr Lys Val Pro Ala Ala Tyr
   1235 1240 1245
   Ala Ala Gln Gly Tyr Lys Val Leu Val Leu Asn Pro Ser Val Ala Ala
   1250 1255 1260
   Thr Leu Gly Phe Gly Ala Tyr Met Ser Lys Ala His Gly Val Asp Pro
   1265 1270 1275 1280
   Asn Ile Arg Thr Gly Val Arg Thr Ile Thr Thr Gly Ser Pro Ile Thr
   1285 1290 1295
   Tyr Ser Thr Tyr Gly Lys Phe Leu Ala Asp Gly Gly Cys Ser Gly Gly
   1300 1305 1310
   Ala Tyr Asp Ile Ile Ile Cys Asp Glu Cys His Ser Thr Asp Ala Thr
   1315 1320 1325
   Ser Ile Leu Gly Ile Gly Thr Val Leu Asp Gln Ala Glu Thr Ala Gly
   1330 1335 1340
   Ala Arg Leu Val Val Leu Ala Thr Ala Thr Pro Pro Gly Ser Val Thr
   1345 1350 1355 1360
   Val Ser His Pro Asn Ile Glu Glu Val Ala Leu Ser Thr Thr Gly Glu
   1365 1370 1375
   Ile Pro Phe Tyr Gly Lys Ala Ile Pro Leu Glu Val Ile Lys Gly Gly
   1380 1385 1390
   Arg His Leu Ile Phe Cys His Ser Lys Lys Lys Cys Asp Glu Leu Ala
   1395 1400 1405
   Ala Lys Leu Val Ala Leu Gly Ile Asn Ala Val Ala Tyr Tyr Arg Gly
   1410 1415 1420
   Leu Asp Val Ser Val Ile Pro Thr Ser Gly Asp Val Val Val Val Ser
   1425 1430 1435 1440
   Thr Asp Ala Leu Met Thr Gly Phe Thr Gly Asp Phe Asp Ser Val Ile
   1445 1450 1455
   Asp Cys Asn Thr Cys Val Thr Gln Thr Val Asp Phe Ser Leu Asp Pro
   1460 1465 1470
   Thr Phe Thr Ile Glu Thr Thr Thr Leu Pro Gln Asp Ala Val Ser Arg
   1475 1480 1485
   Thr Gln Arg Arg Gly Arg Thr Gly Arg Gly Lys Pro Gly Ile Tyr Arg
   1490 1495 1500
   Phe Val Ala Pro Gly Glu Arg Pro Ser Gly Met Phe Asp Ser Ser Val
   1505 1510 1515 1520
   Leu Cys Glu Cys Tyr Asp Ala Gly Cys Ala Trp Tyr Glu Leu Thr Pro
   1525 1530 1535
   Ala Glu Thr Thr Val Arg Leu Arg Ala Tyr Met Asn Thr Pro Gly Leu
   1540 1545 1550
   Pro Val Cys Gln Asp His Leu Glu Phe Trp Glu Gly Val Phe Thr Gly
   1555 1560 1565
   Leu Thr His Ile Asp Ala His Phe Leu Ser Gln Thr Lys Gln Ser Gly
   1570 1575 1580
   Glu Asn Phe Pro Tyr Leu Val Ala Tyr Gln Ala Thr Val Cys Ala Arg
   1585 1590 1595 1600
   Ala Gln Ala Pro Pro Pro Ser Trp Asp Gln Met Trp Lys Cys Leu Ile
   1605 1610 1615
   Arg Leu Lys Pro Thr Leu His Gly Pro Thr Pro Leu Leu Tyr Arg Leu
   1620 1625 1630
   Gly Ala Val Gln Asn Glu Val Thr Leu Thr His Pro Ile Thr Lys Tyr
   1635 1640 1645
   Ile Met Thr Cys Met Ser Ala Asp Leu Glu Val Val Thr Ser Thr Trp
   1650 1655 1660
   Val Leu Val Gly Gly Val Leu Ala Ala Leu Ala Ala Tyr Cys Leu Ser
   1665 1670 1675 1680
   Thr Gly Cys Val Val Ile Val Gly Arg Ile Val Leu Ser Gly Lys Pro
   1685 1690 1695
   Ala Ile Ile Pro Asp Arg Glu Val Leu Tyr Gln Glu Phe Asp Glu Met
   1700 1705 1710
   Glu Glu Cys Ser Gln His Leu Pro Tyr Ile Glu Gln Gly Met Met Leu
   1715 1720 1725
   Ala Glu Gln Phe Lys Gln Lys Ala Leu Gly Leu Leu Gln Thr Ala Ser
   1730 1735 1740
   Arg His Ala Glu Val Ile Thr Pro Ala Val Gln Thr Asn Trp Gln Lys
   1745 1750 1755 1760
   Leu Glu Val Phe Trp Ala Lys His Met Trp Asn Phe Ile Ser Gly Ile
   1765 1770 1775
   Gln Tyr Leu Ala Gly Leu Ser Thr Leu Pro Gly Asn Pro Ala Ile Ala
   1780 1785 1790
   Ser Leu Met Ala Phe Thr Ala Ala Val Thr Ser Pro Leu Thr Thr Gly
   1795 1800 1805
   Gln Thr Leu Leu Phe Asn Ile Leu Gly Gly Trp Val Ala Ala Gln Leu
   1810 1815 1820
   Ala Ala Pro Gly Ala Ala Thr Ala Phe Val Gly Ala Gly Leu Ala Gly
   1825 1830 1835 1840
   Ala Ala Ile Gly Ser Val Gly Leu Gly Lys Val Leu Val Asp Ile Leu
   1845 1850 1855
   Ala Gly Tyr Gly Ala Gly Val Ala Gly Ala Leu Val Ala Phe Lys Ile
   1860 1865 1870
   Met Ser Gly Glu Val Pro Ser Thr Glu Asp Leu Val Asn Leu Leu Pro
   1875 1880 1885
   Ala Ile Leu Ser Pro Gly Ala Leu Val Val Gly Val Val Cys Ala Ala
   1890 1895 1900
   Ile Leu Arg Arg His Val Gly Pro Gly Glu Gly Ala Val Gln Trp Met
   1905 1910 1915 1920
   Asn Arg Leu Ile Ala Phe Ala Ser Arg Gly Asn His Val Ser Pro Thr
   1925 1930 1935
   His Tyr Val Pro Glu Ser Asp Ala Ala Ala Arg Val Thr Ala Ile Leu
   1940 1945 1950
   Ser Ser Leu Thr Val Thr Gln Leu Leu Arg Arg Leu His Gln Trp Ile
   1955 1960 1965
   Ser Ser Glu Cys Thr Thr Pro Cys Ser Gly Ser Trp Leu Arg Asp Ile
   1970 1975 1980
   Trp Asp Trp Ile Cys Glu Val Leu Ser Asp Phe Lys Thr Trp Leu Lys
   1985 1990 1995 2000
   Ala Lys Leu Met Pro Gln Leu Pro Gly Ile Pro Phe Val Ser Cys Gln
   2005 2010 2015
   Arg Gly Tyr Arg Gly Val Trp Arg Gly Asp Gly Ile Met His Thr Arg
   2020 2025 2030
   Cys His Cys Gly Ala Glu Ile Thr Gly His Val Lys Asn Gly Thr Met
   2035 2040 2045
   Arg Ile Val Gly Pro Arg Thr Cys Arg Asn Met Trp Ser Gly Thr Phe
   2050 2055 2060
   Pro Ile Asn Ala Tyr Thr Thr Gly Pro Cys Thr Pro Leu Pro Ala Pro
   2065 2070 2075 2080
   Asn Tyr Lys Phe Ala Leu Trp Arg Val Ser Ala Glu Glu Tyr Val Glu
   2085 2090 2095
   Ile Arg Arg Val Gly Asp Phe His Tyr Val Ser Gly Met Thr Thr Asp
   2100 2105 2110
   Asn Leu Lys Cys Pro Cys Gln Ile Pro Ser Pro Glu Phe Phe Thr Glu
   2115 2120 2125
   Leu Asp Gly Val Arg Leu His Arg Phe Ala Pro Pro Cys Lys Pro Leu
   2130 2135 2140
   Leu Arg Glu Glu Val Ser Phe Arg Val Gly Leu His Glu Tyr Pro Val
   2145 2150 2155 2160
   Gly Ser Gln Leu Pro Cys Glu Pro Glu Pro Asp Val Ala Val Leu Thr
   2165 2170 2175
   Ser Met Leu Thr Asp Pro Ser His Ile Thr Ala Glu Ala Ala Gly Arg
   2180 2185 2190
   Arg Leu Ala Arg Gly Ser Pro Pro Ser Met Ala Ser Ser Ser Ala Ser
   2195 2200 2205
   Gln Leu Ser Ala Pro Ser Leu Lys Ala Thr Cys Thr Ala Asn His Asp
   2210 2215 2220
   Ser Pro Asp Ala Glu Leu Ile Glu Ala Asn Leu Leu Trp Arg Gln Glu
   2225 2230 2235 2240
   Met Gly Gly Asn Ile Thr Arg Val Glu Ser Glu Asn Lys Val Val Ile
   2245 2250 2255
   Leu Asp Ser Phe Asp Pro Leu Val Ala Glu Glu Asp Glu Arg Glu Val
   2260 2265 2270
   Ser Val Pro Ala Glu Ile Leu Arg Lys Ser Arg Arg Phe Ala Arg Ala
   2275 2280 2285
   Leu Pro Val Trp Ala Arg Pro Asp Tyr Asn Pro Pro Leu Val Glu Thr
   2290 2295 2300
   Trp Lys Lys Pro Asp Tyr Glu Pro Pro Val Val His Gly Cys Pro Leu
   2305 2310 2315 2320
   Pro Pro Pro Arg Ser Pro Pro Val Pro Pro Pro Arg Lys Lys Arg Thr
   2325 2330 2335
   Val Val Leu Thr Glu Ser Thr Leu Ser Thr Ala Leu Ala Glu Leu Ala
   2340 2345 2350
   Thr Lys Ser Phe Gly Ser Ser Ser Thr Ser Gly Ile Thr Gly Asp Asn
   2355 2360 2365
   Thr Thr Thr Ser Ser Glu Pro Ala Pro Ser Gly Cys Pro Pro Asp Ser
   2370 2375 2380
   Asp Val Glu Ser Tyr Ser Ser Met Pro Pro Leu Glu Gly Glu Pro Gly
   2385 2390 2395 2400
   Asp Pro Asp Leu Ser Asp Gly Ser Trp Ser Thr Val Ser Ser Gly Ala
   2405 2410 2415
   Asp Thr Glu Asp Val Val Cys Cys Ser Met Ser Tyr Ser Trp Thr Gly
   2420 2425 2430
   Ala Leu Val Thr Pro Cys Ala Ala Glu Glu Gln Lys Leu Pro Ile Asn
   2435 2440 2445
   Ala Leu Ser Asn Ser Leu Leu Arg His His Asn Leu Val Tyr Ser Thr
   2450 2455 2460
   Thr Ser Arg Ser Ala Cys Gln Arg Gln Lys Lys Val Thr Phe Asp Arg
   2465 2470 2475 2480
   Leu Gln Val Leu Asp Ser His Tyr Gln Asp Val Leu Lys Glu Val Lys
   2485 2490 2495
   Ala Ala Ala Ser Lys Val Lys Ala Asn Leu Leu Ser Val Glu Glu Ala
   2500 2505 2510
   Cys Ser Leu Thr Pro Pro His Ser Ala Lys Ser Lys Phe Gly Tyr Gly
   2515 2520 2525
   Ala Lys Asp Val Arg Cys His Ala Arg Lys Ala Val Ala His Ile Asn
   2530 2535 2540
   Ser Val Trp Lys Asp Leu Leu Glu Asp Ser Val Thr Pro Ile Asp Thr
   2545 2550 2555 2560
   Thr Ile Met Ala Lys Asn Glu Val Phe Cys Val Gln Pro Glu Lys Gly
   2565 2570 2575
   Gly Arg Lys Pro Ala Arg Leu Ile Val Phe Pro Asp Leu Gly Val Arg
   2580 2585 2590
   Val Cys Glu Lys Met Ala Leu Tyr Asp Val Val Ser Lys Leu Pro Leu
   2595 2600 2605
   Ala Val Met Gly Ser Ser Tyr Gly Phe Gln Tyr Ser Pro Gly Gln Arg
   2610 2615 2620
   Val Glu Phe Leu Val Gln Ala Trp Lys Ser Lys Lys Thr Pro Met Gly
   2625 2630 2635 2640
   Phe Ser Tyr Asp Thr Arg Cys Phe Asp Ser Thr Val Thr Glu Ser Asp
   2645 2650 2655
   Ile Arg Thr Glu Glu Ala Ile Tyr Gln Cys Cys Asp Leu Asp Pro Gln
   2660 2665 2670
   Ala Arg Val Ala Ile Lys Ser Leu Thr Glu Arg Leu Tyr Val Gly Gly
   2675 2680 2685
   Pro Leu Thr Asn Ser Arg Gly Glu Asn Cys Gly Tyr Arg Arg Cys Arg
   2690 2695 2700
   Ala Ser Gly Val Leu Thr Thr Ser Cys Gly Asn Thr Leu Thr Cys Tyr
   2705 2710 2715 2720
   Ile Lys Ala Arg Ala Ala Cys Arg Ala Ala Gly Leu Gln Asp Cys Thr
   2725 2730 2735
   Met Leu Val Cys Gly Asp Asp Leu Val Val Ile Cys Glu Ser Ala Gly
   2740 2745 2750
   Val Gln Glu Asp Ala Ala Ser Leu Arg Ala Phe Thr Glu Ala Met Thr
   2755 2760 2765
   Arg Tyr Ser Ala Pro Pro Gly Asp Pro Pro Gln Pro Glu Tyr Asp Leu
   2770 2775 2780
   Glu Leu Ile Thr Ser Cys Ser Ser Asn Val Ser Val Ala His Asp Gly
   2785 2790 2795 2800
   Ala Gly Lys Arg Val Tyr Tyr Leu Thr Arg Asp Pro Thr Thr Pro Leu
   2805 2810 2815
   Ala Arg Ala Ala Trp Glu Thr Ala Arg His Thr Pro Val Asn Ser Trp
   2820 2825 2830
   Leu Gly Asn Ile Ile Met Phe Ala Pro Thr Leu Trp Ala Arg Met Ile
   2835 2840 2845
   Leu Met Thr His Phe Phe Ser Val Leu Ile Ala Arg Asp Gln Leu Glu
   2850 2855 2860
   Gln Ala Leu Asn Cys Glu Ile Tyr Gly Ala Cys Tyr Ser Ile Glu Pro
   2865 2870 2875 2880
   Leu Asp Leu Pro Pro Ile Ile Gln Arg Leu His Gly Leu Ser Ala Phe
   2885 2890 2895
   Ser Leu His Ser Tyr Ser Pro Gly Glu Ile Asn Arg Val Ala Ala Cys
   2900 2905 2910
   Leu Arg Lys Leu Gly Val Pro Pro Leu Arg Ala Trp Arg His Arg Ala
   2915 2920 2925
   Arg Ser Val Arg Ala Arg Leu Leu Ser Arg Gly Gly Arg Ala Ala Ile
   2930 2935 2940
   Cys Gly Lys Tyr Leu Phe Asn Trp Ala Val Arg Thr Lys Leu Lys Leu
   2945 2950 2955 2960
   Thr Pro Ile Ala Ala Ala Gly Arg Leu Asp Leu Ser Gly Trp Phe Thr
   2965 2970 2975
   Ala Gly Tyr Ser Gly Gly Asp Ile Tyr His Ser Val Ser His Ala Arg
   2980 2985 2990
   Pro Arg Trp Phe Trp Phe Cys Leu Leu Leu Leu Ala Ala Gly Val Gly
   2995 3000 3005
   Ile Tyr Leu Leu Pro Asn Arg
   3010 3015
<210> 5
   <211> 9611
   <212> DNA
   <213> Hepatitis C virus
<400> 5
   gccagccccc tgatgggggc gacactccac catgaatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccgg 180
   gaagactggg tcctttcttg gataaaccca ctctatgccc ggccatttgg gcgtgccccc 240
   gcaagactgc tagccgagta gcgttgggtt gcgaaaggcc ttgtggtact gcctgatagg 300
   gtgcttgcga gtgccccggg aggtctcgta gaccgtgcac catgagcaca aatcctaaac 360
   ctcaaagaaa aaccaaaaga aacaccaacc gtcgcccaca agacgttaag tttccgggcg 420
   gcggccagat cgttggcgga gtatacttgt tgccgcgcag gggccccagg ttgggtgtgc 480
   gcgcgacaag gaagacttcg gagcggtccc agccacgtgg aaggcgccag cccatcccta 540
   aagatcggcg ctccactggc aaatcctggg gaaaaccagg atacccctgg cccctatacg 600
   ggaatgaggg actcggctgg gcaggatggc tcctgtcccc ccgaggttcc cgtccctctt 660
   ggggccccaa tgacccccgg cataggtcgc gcaacgtggg taaggtcatc gataccctaa 720
   cgtgcggctt tgccgacctc atggggtaca tccctgtcgt gggcgccccg ctcggcggcg 780
   tcgccagagc tctcgcgcat ggcgtgagag tcctggagga cggggttaat tttgcaacag 840
   ggaacttacc cggttgctcc ttttctatct tcttgctggc cctgctgtcc tgcatcacca 900
   ccccggtctc cgctgccgaa gtgaagaaca tcagtaccgg ctacatggtg actaacgact 960
   gcaccaatga cagcattacc tggcagctcc aggctgctgt cctccacgtc cccgggtgcg 1020
   tcccgtgcga gaaagtgggg aatgcatctc agtgctggat accggtctca ccgaatgtgg 1080
   ccgtgcagcg gcccggcgcc ctcacgcagg gcttgcggac gcacatcgac atggttgtga 1140
   tgtccgccac gctctgctct gccctctacg tgggggacct ctgcggtggg gtgatgctcg 1200
   cagcccaaat gttcattgtc tcgccgcagc accactggtt tgtccaagac tgcaattgct 1260
   ccatctaccc tggtaccatc actggacacc gcatggcatg ggacatgatg atgaactggt 1320
   cgcccacggc taccatgatc ttggcgtacg cgatgcgtgt ccccgaggtc attatagaca 1380
   tcattagcgg ggctcattgg ggcgtcatgt tcggcttggc ctacttctct atgcagggag 1440
   cgtgggcgaa agtcgttgtc atccttctgt tggccgccgg ggtggacgcg cgcacccata 1500
   ctgttggggg ttctgccgcg cagaccaccg ggcgcctcac cagcttattt gacatgggcc 1560
   ccaggcagaa aatccagctc gttaacacca atggcagctg gcacatcaac cgcaccgccc 1620
   tgaactgcaa tgactccttg cacaccggct ttatcgcgtc tctgttctac acccacagct 1680
   tcaactcgtc aggatgtccc gaacgcatgt ccgcctgccg cagtatcgag gccttccggg 1740
   tgggatgggg cgccttgcaa tatgaggata atgtcaccaa tccagaggat atgagaccct 1800
   attgctggca ctacccacca aggcagtgtg gcgtggtctc cgcgaagact gtgtgtggcc 1860
   cagtgtactg tttcaccccc agcccagtgg tagtgggcac gaccgacagg cttggagcgc 1920
   ccacttacac gtggggggag aatgagacag atgtcttcct attgaacagc actcgaccac 1980
   cgctggggtc atggttcggc tgcacgtgga tgaactcttc tggctacacc aagacttgcg 2040
   gcgcaccacc ctgccgtact agagctgact tcaacgccag cacggacctg ttgtgcccca 2100
   cggactgttt taggaagcat cctgatacca cttacctcaa atgcggctct gggccctggc 2160
   tcacgccaag gtgcctgatc gactacccct acaggctctg gcattacccc tgcacagtta 2220
   actataccat cttcaaaata aggatgtatg tgggaggggt tgagcacagg ctcacggctg 2280
   catgcaattt cactcgtggg gatcgttgca acttggagga cagagacaga agtcaactgt 2340
   ctcctttgtt gcactccacc acggaatggg ccattttacc ttgctcttac tcggacctgc 2400
   ccgccttgtc gactggtctt ctccacctcc accaaaacat cgtggacgta caattcatgt 2460
   atggcctatc acctgccctc acaaaataca tcgtccgatg ggagtgggta atactcttat 2520
   tcctgctctt agcggacgcc agggtttgcg cctgcttatg gatgctcatc ttgttgggcc 2580
   aggccgaagc agctttggag aacctcgtaa tactcaatgc agcatccctg gccgggacgc 2640
   acggtcttgt gtccttcctc gtgttcttct gctttgcgtg gtatctgaag ggtaggtggg 2700
   tgcccggagc ggtctacgcc ctctacggga tgtggcctct cctcctgctc ctgctggcgt 2760
   tgcctcagcg ggcatatgca ctggacacgg aggtggccgc gtcgtgtggc ggcgttgttc 2820
   ttgtcgggtt aatggcgctg actctgtcgc catattacaa gcgctatatc agctggtgca 2880
   tgtggtggct tcagtatttt ctgaccagag tagaagcgca actgcacgtg tgggttcccc 2940
   ccctcaacgt ccgggggggg cgcgatgccg tcatcttact catgtgtgta gtacacccga 3000
   ccctggtatt tgacatcacc aaactactcc tggccatctt cggacccctt tggattcttc 3060
   aagccagttt gcttaaagtc ccctacttcg tgcgcgttca aggccttctc cggatctgcg 3120
   cgctagcgcg gaagatagcc ggaggtcatt acgtgcaaat ggccatcatc aagttagggg 3180
   cgcttactgg cacctatgtg tataaccatc tcacccctct tcgagactgg gcgcacaacg 3240
   gcctgcgaga tctggccgtg gctgtggaac cagtcgtctt ctcccgaatg gagaccaagc 3300
   tcatcacgtg gggggcagat accgccgcgt gcggtgacat catcaacggc ttgcccgtct 3360
   ctgcccgtag ggccaggagg atactgcttg ggccagccga cggaatggtc tccaaggggt 3420
   ggaggttgct ggcgcccatc acggcgtacg cccagcagac gagaggcctc ctagggtgta 3480
   taatcaccag cctgactggc cgggacaaaa accaagtgga gggtgaggtc cagatcgtgt 3540
   caactgctac ccaaaccttc ctggcaacgt gcatcaatgg ggtatgctgg actgtctacc 3600
   acggggccgg aacgaggacc atcgcatcac ccaagggtcc tgtcatccag atgtatacca 3660
   atgtggacca agaccttgtg ggctggcccg ctcctcaagg ttcccgctca ttgacaccct 3720
   gtacctgcgg ctcctcggac ctttacctgg tcacgaggca cgccgatgtc attcccgtgc 3780
   gccggcgagg tgatagcagg ggtagcctgc tttcgccccg gcccatttcc tacttgaaag 3840
   gctcctcggg gggtccgctg ttgtgccccg cgggacacgc cgtgggccta ttcagggccg 3900
   cggtgtgcac ccgtggagtg gctaaagcgg tggactttat ccctgtggag aacctaggga 3960
   caaccatgag atccccggtg ttcacggaca actcctctcc accagcagtg ccccagagct 4020
   tccaggtggc ccacctgcat gctcccaccg gcagcggtaa gagcaccaag gtcccggctg 4080
   cgtacgcagc ccagggctac aaggtgttgg tgctcaaccc ctctgttgct gcaacgctgg 4140
   gctttggtgc ttacatgtcc aaggcccatg gggttgatcc taatatcagg accggggtga 4200
   gaacaattac cactggcagc cccatcacgt actccaccta cggcaagttc cttgccgacg 4260
   gcgggtgctc aggaggtgct tatgacataa taatttgtga cgagtgccac tccacggatg 4320
   ccacatccat cttgggcatc ggcactgtcc ttgaccaagc agagactgcg ggggcgagac 4380
   tggttgtgct cgccactgct acccctccgg gctccgtcac tgtgtcccat cctaacatcg 4440
   aggaggttgc tctgtccacc accggagaga tcccctttta cggcaaggct atccccctcg 4500
   aggtgatcaa ggggggaaga catctcatct tctgccactc aaagaagaag tgcgacgagc 4560
   tcgccgcgaa gctggtcgca ttgggcatca atgccgtggc ctactaccgc ggtcttgacg 4620
   tgtctgtcat cccgaccagc ggcgatgttg tcgtcgtgtc gaccgatgct ctcatgactg 4680
   gctttaccgg cgacttcgac tctgtgatag actgcaacac gtgtgtcact cagacagtcg 4740
   atttcagcct tgaccctacc tttaccattg agacaaccac gctcccccag gatgctgtct 4800
   ccaggactca acgccggggc aggactggca gggggaagcc aggcatctat agatttgtgg 4860
   caccggggga gcgcccctcc ggcatgttcg actcgtccgt cctctgtgag tgctatgacg 4920
   cgggctgtgc ttggtatgag ctcacgcccg ccgagactac agttaggcta cgagcgtaca 4980
   tgaacacccc ggggcttccc gtgtgccagg accatcttga attttgggag ggcgtcttta 5040
   cgggcctcac tcatatagat gcccactttt tatcccagac aaagcagagt ggggagaact 5100
   ttccttacct ggtagcgtac caagccaccg tgtgcgctag ggctcaagcc cctcccccat 5160
   cgtgggacca gatgtggaag tgtttgatcc gccttaaacc caccctccat gggccaacac 5220
   ccctgctata cagactgggc gctgttcaga atgaagtcac cctgacgcac ccaatcacca 5280
   aatacatcat gacatgcatg tcggccgacc tggaggtcgt cacgagcacc tgggtgctcg 5340
   ttggcggcgt cctggctgct ctggccgcgt attgcctgtc aacaggctgc gtggtcatag 5400
   tgggcaggat cgtcttgtcc gggaagccgg caattatacc tgacagggag gttctctacc 5460
   aggagttcga tgagatggaa gagtgctctc agcacttacc gtacatcgag caagggatga 5520
   tgctcgctga gcagttcaag cagaaggccc tcggcctcct gcagaccgcg tcccgccatg 5580
   cagaggttat cacccctgct gtccagacca actggcagaa actcgaggtc ttttgggcga 5640
   agcacatgtg gaatttcatc agtgggatac aatacttggc gggcctgtca acgctgcctg 5700
   gtaaccccgc cattgcttca ttgatggctt ttacagctgc cgtcaccagc ccactaacca 5760
   ctggccaaac cctcctcttc aacatattgg gggggtgggt ggctgcccag ctcgccgccc 5820
   ccggtgccgc tactgccttt gtgggtgctg gcctagctgg cgccgccatc ggcagcgttg 5880
   gactggggaa ggtcctcgtg gacattcttg cagggtatgg cgcgggcgtg gcgggagctc 5940
   ttgtagcatt caagatcatg agcggtgagg tcccctccac ggaggacctg gtcaatctgc 6000
   tgcccgccat cctctcgcct ggagcccttg tagtcggtgt ggtctgcgca gcaatactgc 6060
   gccggcacgt tggcccgggc gagggggcag tgcaatggat gaaccggcta atagccttcg 6120
   cctcccgggg gaaccatgtt tcccccacgc actacgtgcc ggagagcgat gcagccgccc 6180
   gcgtcactgc catactcagc agcctcactg taacccagct cctgaggcga ctgcatcagt 6240
   ggataagctc ggagtgtacc actccatgct ccggttcctg gctaagggac atctgggact 6300
   ggatatgcga ggtgctgagc gactttaaga cctggctgaa agccaagctc atgccacaac 6360
   tgcctgggat tccctttgtg tcctgccagc gcgggtatag gggggtctgg cgaggagacg 6420
   gcattatgca cactcgctgc cactgtggag ctgagatcac tggacatgtc aaaaacggga 6480
   cgatgaggat cgtcggtcct aggacctgca ggaacatgtg gagtgggacg ttccccatta 6540
   acgcctacac cacgggcccc tgtactcccc ttcctgcgcc gaactataag ttcgcgctgt 6600
   ggagggtgtc tgcagaggaa tacgtggaga taaggcgggt gggggacttc cactacgtat 6660
   cgggtatgac tactgacaat cttaaatgcc cgtgccagat cccatcgccc gaatttttca 6720
   cagaattgga cggggtgcgc ctacacaggt ttgcgccccc ttgcaagccc ttgctgcggg 6780
   aggaggtatc attcagagta ggactccacg agtacccggt ggggtcgcaa ttaccttgcg 6840
   agcccgaacc ggacgtagcc gtgttgacgt ccatgctcac tgatccctcc catataacag 6900
   cagaggcggc cgggagaagg ttggcgagag ggtcaccccc ttctatggcc agctcctcgg 6960
   ctagccagct gtccgctcca tctctcaagg caacttgcac cgccaaccat gactcccctg 7020
   acgccgagct catagaggct aacctcctgt ggaggcagga gatgggcggc aacatcacca 7080
   gggttgagtc agagaacaaa gtggtgattc tggactcctt cgatccgctt gtggcagagg 7140
   aggatgagcg ggaggtctcc gtacctgcag aaattctgcg gaagtctcgg agattcgccc 7200
   gggccctgcc cgtctgggcg cggccggact acaacccccc gctagtagag acgtggaaaa 7260
   agcctgacta cgaaccacct gtggtccatg gctgcccgct accacctcca cggtcccctc 7320
   ctgtgcctcc gcctcggaaa aagcgtacgg tggtcctcac cgaatcaacc ctatctactg 7380
   ccttggccga gcttgccacc aaaagttttg gcagctcctc aacttccggc attacgggcg 7440
   acaatacgac aacatcctct gagcccgccc cttctggctg cccccccgac tccgacgttg 7500
   agtcctattc ttccatgccc cccctggagg gggagcctgg ggatccggat ctcagcgacg 7560
   ggtcatggtc gacggtcagt agtggggccg acacggaaga tgtcgtgtgc tgctcaatgt 7620
   cttattcctg gacaggcgca ctcgtcaccc cgtgcgctgc ggaagaacaa aaactgccca 7680
   tcaacgcact gagcaactcg ttgctacgcc atcacaatct ggtgtattcc accacttcac 7740
   gcagtgcttg ccaaaggcag aagaaagtca catttgacag actgcaagtt ctggacagcc 7800
   attaccagga cgtgctcaag gaggtcaaag cagcggcgtc aaaagtgaag gctaacttgc 7860
   tatccgtaga ggaagcttgc agcctgacgc ccccacattc agccaaatcc aagtttggct 7920
   atggggcaaa agacgtccgt tgccatgcca gaaaggccgt agcccacatc aactccgtgt 7980
   ggaaagacct tctggaagac agtgtaacac caatagacac taccatcatg gccaagaacg 8040
   aggttttctg cgttcagcct gagaaggggg gtcgtaagcc agctcgtctc atcgtgttcc 8100
   ccgacctggg cgtgcgcgtg tgcgagaaga tggccctgta cgacgtggtt agcaagctcc 8160
   ccctggccgt gatgggaagc tcctacggat tccaatactc accaggacag cgggttgaat 8220
   tcctcgtgca agcgtggaag tccaagaaga ccccgatggg gttctcgtat gatacccgct 8280
   gttttgactc cacagtcact gagagcgaca tccgtacgga ggaggcaatt taccaatgtt 8340
   gtgacctgga cccccaagcc cgcgtggcca tcaagtccct cactgagagg ctttatgttg 8400
   ggggccctct taccaattca aggggggaaa actgcggcta ccgcaggtgc cgcgcgagcg 8460
   gcgtactgac aactagctgt ggtaacaccc tcacttgcta catcaaggcc cgggcagcct 8520
   gtcgagccgc agggctccag gactgcacca tgctcgtgtg tggcgacgac ttagtcgtta 8580
   tctgtgaaag tgcgggggtc caggaggacg cggcgagcct gagagccttc acggaggcta 8640
   tgaccaggta ctccgccccc cccggggacc ccccacaacc agaatacgac ttggagctta 8700
   taacatcatg ctcctccaac gtgtcagtcg cccacgacgg cgctggaaag agggtctact 8760
   accttacccg tgaccctaca acccccctcg cgagagccgc gtgggagaca gcaagacaca 8820
   ctccagtcaa ttcctggcta ggcaacataa tcatgtttgc ccccacactg tgggcgagga 8880
   tgatactgat gacccatttc tttagcgtcc tcatagccag ggatcagctt gaacaggctc 8940
   ttaactgtga gatctacgga gcctgctact ccatagaacc actggatcta cctccaatca 9000
   ttcaaagact ccatggcctc agcgcatttt cactccacag ttactctcca ggtgaaatca 9060
   atagggtggc cgcatgcctc agaaaacttg gggtcccgcc cttgcgagct tggagacacc 9120
   gggcccggag cgtccgcgct aggcttctgt ccagaggagg cagggctgct atatgtggca 9180
   agtacctctt caactgggca gtaagaacaa agctcaaact cactccaata gcggccgctg 9240
   gccggctgga cttgtccggt tggttcacgg ctggctacag cgggggagac atttatcaca 9300
   gcgtgtctca tgcccggccc cgctggttct ggttttgcct actcctgctc gctgcagggg 9360
   taggcatcta cctcctcccc aaccgatgaa ggttggggta aacactccgg cctcttaagc 9420
   catttcctgt tttttttttt tttttttttt tttttttctt tttttttttc tttcctttcc 9480
   ttcttttttt cctttctttt tcccttcttt aatggtggct ccatcttagc cctagtcacg 9540
   gctagctgtg aaaggtccgt gagccgcatg actgcagaga gtgctgatac tggcctctct 9600
   gcagatcatg t 9611
<210> 6
   <211> 3015
   <212> PRT
   <213> Hepatitis C virus
<400> 6
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Asn Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
   195 200 205
   Asn Asp Ser Ile Thr Trp Gln Leu Gln Ala Ala Val Leu His Val Pro
   210 215 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Ile Leu Leu Phe Leu Leu Leu Ala Asp Ala Arg Val Cys
   725 730 735
   Ala Cys Leu Trp Met Leu Ile Leu Leu Gly Gln Ala Glu Ala Ala Leu
   740 745 750
   Glu Asn Leu Val Ile Leu Asn Ala Ala Ser Leu Ala Gly Thr His Gly
   755 760 765
   Leu Val Ser Phe Leu Val Phe Phe Cys Phe Ala Trp Tyr Leu Lys Gly
   770 775 780
   Arg Trp Val Pro Gly Ala Val Tyr Ala Leu Tyr Gly Met Trp Pro Leu
   785 790 795 800
   Leu Leu Leu Leu Leu Ala Leu Pro Gln Arg Ala Tyr Ala Leu Asp Thr
   805 810 815
   Glu Val Ala Ala Ser Cys Gly Gly Val Val Leu Val Gly Leu Met Ala
   820 825 830
   Leu Thr Leu Ser Pro Tyr Tyr Lys Arg Tyr Ile Ser Trp Cys Met Trp
   835 840 845
   Trp Leu Gln Tyr Phe Leu Thr Arg Val Glu Ala Gln Leu His Val Trp
   850 855 860
   Val Pro Pro Leu Asn Val Arg Gly Gly Arg Asp Ala Val Ile Leu Leu
   865 870 875 880
   Met Cys Val Val His Pro Thr Leu Val Phe Asp Ile Thr Lys Leu Leu
   885 890 895
   Leu Ala Ile Phe Gly Pro Leu Trp Ile Leu Gln Ala Ser Leu Leu Lys
   900 905 910
   Val Pro Tyr Phe Val Arg Val Gln Gly Leu Leu Arg Ile Cys Ala Leu
   915 920 925
   Ala Arg Lys Ile Ala Gly Gly His Tyr Val Gln Met Ala Ile Ile Lys
   930 935 940
   Leu Gly Ala Leu Thr Gly Thr Tyr Val Tyr Asn His Leu Thr Pro Leu
   945 950 955 960
   Arg Asp Trp Ala His Asn Gly Leu Arg Asp Leu Ala Val Ala Val Glu
   965 970 975
   Pro Val Val Phe Ser Arg Met Glu Thr Lys Leu Ile Thr Trp Gly Ala
   980 985 990
   Asp Thr Ala Ala Cys Gly Asp Ile Ile Asn Gly Leu Pro Val Ser Ala
   995 1000 1005
   Arg Arg Gly Gln Glu Ile Leu Leu Gly Pro Ala Asp Gly Met Val Ser
   1010 1015 1020
   Lys Gly Trp Arg Leu Leu Ala Pro Ile Thr Ala Tyr Ala Gln Gln Thr
   1025 1030 1035 1040
   Arg Gly Leu Leu Gly Cys Ile Ile Thr Ser Leu Thr Gly Arg Asp Lys
   1045 1050 1055
   Asn Gln Val Glu Gly Glu Val Gln Ile Val Ser Thr Ala Thr Gln Thr
   1060 1065 1070
   Phe Leu Ala Thr Cys Ile Asn Gly Val Cys Trp Thr Val Tyr His Gly
   1075 1080 1085
   Ala Gly Thr Arg Thr Ile Ala Ser Pro Lys Gly Pro Val Ile Gln Met
   1090 1095 1100
   Tyr Thr Asn Val Asp Gln Asp Leu Val Gly Trp Pro Ala Pro Gln Gly
   1105 1110 1115 1120
   Ser Arg Ser Leu Thr Pro Cys Thr Cys Gly Ser Ser Asp Leu Tyr Leu
   1125 1130 1135
   Val Thr Arg His Ala Asp Val Ile Pro Val Arg Arg Arg Gly Asp Ser
   1140 1145 1150
   Arg Gly Ser Leu Leu Ser Pro Arg Pro Ile Ser Tyr Leu Lys Gly Ser
   1155 1160 1165
   Ser Gly Gly Pro Leu Leu Cys Pro Ala Gly His Ala Val Gly Leu Phe
   1170 1175 1180
   Arg Ala Ala Val Cys Thr Arg Gly Val Ala Lys Ala Val Asp Phe Ile
   1185 1190 1195 1200
   Pro Val Glu Asn Leu Gly Thr Thr Met Arg Ser Pro Val Phe Thr Asp
   1205 1210 1215
   Asn Ser Ser Pro Pro Ala Val Pro Gln Ser Phe Gln Val Ala His Leu
   1220 1225 1230
   His Ala Pro Thr Gly Ser Gly Lys Ser Thr Lys Val Pro Ala Ala Tyr
   1235 1240 1245
   Ala Ala Gln Gly Tyr Lys Val Leu Val Leu Asn Pro Ser Val Ala Ala
   1250 1255 1260
   Thr Leu Gly Phe Gly Ala Tyr Met Ser Lys Ala His Gly Val Asp Pro
   1265 1270 1275 1280
   Asn Ile Arg Thr Gly Val Arg Thr Ile Thr Thr Gly Ser Pro Ile Thr
   1285 1290 1295
   Tyr Ser Thr Tyr Gly Lys Phe Leu Ala Asp Gly Gly Cys Ser Gly Gly
   1300 1305 1310
   Ala Tyr Asp Ile Ile Ile Cys Asp Glu Cys His Ser Thr Asp Ala Thr
   1315 1320 1325
   Ser Ile Leu Gly Ile Gly Thr Val Leu Asp Gln Ala Glu Thr Ala Gly
   1330 1335 1340
   Ala Arg Leu Val Val Leu Ala Thr Ala Thr Pro Pro Gly Ser Val Thr
   1345 1350 1355 1360
   Val Ser His Pro Asn Ile Glu Glu Val Ala Leu Ser Thr Thr Gly Glu
   1365 1370 1375
   Ile Pro Phe Tyr Gly Lys Ala Ile Pro Leu Glu Val Ile Lys Gly Gly
   1380 1385 1390
   Arg His Leu Ile Phe Cys His Ser Lys Lys Lys Cys Asp Glu Leu Ala
   1395 1400 1405
   Ala Lys Leu Val Ala Leu Gly Ile Asn Ala Val Ala Tyr Tyr Arg Gly
   1410 1415 1420
   Leu Asp Val Ser Val Ile Pro Thr Ser Gly Asp Val Val Val Val Ser
   1425 1430 1435 1440
   Thr Asp Ala Leu Met Thr Gly Phe Thr Gly Asp Phe Asp Ser Val Ile
   1445 1450 1455
   Asp Cys Asn Thr Cys Val Thr Gln Thr Val Asp Phe Ser Leu Asp Pro
   1460 1465 1470
   Thr Phe Thr Ile Glu Thr Thr Thr Leu Pro Gln Asp Ala Val Ser Arg
   1475 1480 1485
   Thr Gln Arg Arg Gly Arg Thr Gly Arg Gly Lys Pro Gly Ile Tyr Arg
   1490 1495 1500
   Phe Val Ala Pro Gly Glu Arg Pro Ser Gly Met Phe Asp Ser Ser Val
   1505 1510 1515 1520
   Leu Cys Glu Cys Tyr Asp Ala Gly Cys Ala Trp Tyr Glu Leu Thr Pro
   1525 1530 1535
   Ala Glu Thr Thr Val Arg Leu Arg Ala Tyr Met Asn Thr Pro Gly Leu
   1540 1545 1550
   Pro Val Cys Gln Asp His Leu Glu Phe Trp Glu Gly Val Phe Thr Gly
   1555 1560 1565
   Leu Thr His Ile Asp Ala His Phe Leu Ser Gln Thr Lys Gln Ser Gly
   1570 1575 1580
   Glu Asn Phe Pro Tyr Leu Val Ala Tyr Gln Ala Thr Val Cys Ala Arg
   1585 1590 1595 1600
   Ala Gln Ala Pro Pro Pro Ser Trp Asp Gln Met Trp Lys Cys Leu Ile
   1605 1610 1615
   Arg Leu Lys Pro Thr Leu His Gly Pro Thr Pro Leu Leu Tyr Arg Leu
   1620 1625 1630
   Gly Ala Val Gln Asn Glu Val Thr Leu Thr His Pro Ile Thr Lys Tyr
   1635 1640 1645
   Ile Met Thr Cys Met Ser Ala Asp Leu Glu Val Val Thr Ser Thr Trp
   1650 1655 1660
   Val Leu Val Gly Gly Val Leu Ala Ala Leu Ala Ala Tyr Cys Leu Ser
   1665 1670 1675 1680
   Thr Gly Cys Val Val Ile Val Gly Arg Ile Val Leu Ser Gly Lys Pro
   1685 1690 1695
   Ala Ile Ile Pro Asp Arg Glu Val Leu Tyr Gln Glu Phe Asp Glu Met
   1700 1705 1710
   Glu Glu Cys Ser Gln His Leu Pro Tyr Ile Glu Gln Gly Met Met Leu
   1715 1720 1725
   Ala Glu Gln Phe Lys Gln Lys Ala Leu Gly Leu Leu Gln Thr Ala Ser
   1730 1735 1740
   Arg His Ala Glu Val Ile Thr Pro Ala Val Gln Thr Asn Trp Gln Lys
   1745 1750 1755 1760
   Leu Glu Val Phe Trp Ala Lys His Met Trp Asn Phe Ile Ser Gly Ile
   1765 1770 1775
   Gln Tyr Leu Ala Gly Leu Ser Thr Leu Pro Gly Asn Pro Ala Ile Ala
   1780 1785 1790
   Ser Leu Met Ala Phe Thr Ala Ala Val Thr Ser Pro Leu Thr Thr Gly
   1795 1800 1805
   Gln Thr Leu Leu Phe Asn Ile Leu Gly Gly Trp Val Ala Ala Gln Leu
   1810 1815 1820
   Ala Ala Pro Gly Ala Ala Thr Ala Phe Val Gly Ala Gly Leu Ala Gly
   1825 1830 1835 1840
   Ala Ala Ile Gly Ser Val Gly Leu Gly Lys Val Leu Val Asp Ile Leu
   1845 1850 1855
   Ala Gly Tyr Gly Ala Gly Val Ala Gly Ala Leu Val Ala Phe Lys Ile
   1860 1865 1870
   Met Ser Gly Glu Val Pro Ser Thr Glu Asp Leu Val Asn Leu Leu Pro
   1875 1880 1885
   Ala Ile Leu Ser Pro Gly Ala Leu Val Val Gly Val Val Cys Ala Ala
   1890 1895 1900
   Ile Leu Arg Arg His Val Gly Pro Gly Glu Gly Ala Val Gln Trp Met
   1905 1910 1915 1920
   Asn Arg Leu Ile Ala Phe Ala Ser Arg Gly Asn His Val Ser Pro Thr
   1925 1930 1935
   His Tyr Val Pro Glu Ser Asp Ala Ala Ala Arg Val Thr Ala Ile Leu
   1940 1945 1950
   Ser Ser Leu Thr Val Thr Gln Leu Leu Arg Arg Leu His Gln Trp Ile
   1955 1960 1965
   Ser Ser Glu Cys Thr Thr Pro Cys Ser Gly Ser Trp Leu Arg Asp Ile
   1970 1975 1980
   Trp Asp Trp Ile Cys Glu Val Leu Ser Asp Phe Lys Thr Trp Leu Lys
   1985 1990 1995 2000
   Ala Lys Leu Met Pro Gln Leu Pro Gly Ile Pro Phe Val Ser Cys Gln
   2005 2010 2015
   Arg Gly Tyr Arg Gly Val Trp Arg Gly Asp Gly Ile Met His Thr Arg
   2020 2025 2030
   Cys His Cys Gly Ala Glu Ile Thr Gly His Val Lys Asn Gly Thr Met
   2035 2040 2045
   Arg Ile Val Gly Pro Arg Thr Cys Arg Asn Met Trp Ser Gly Thr Phe
   2050 2055 2060
   Pro Ile Asn Ala Tyr Thr Thr Gly Pro Cys Thr Pro Leu Pro Ala Pro
   2065 2070 2075 2080
   Asn Tyr Lys Phe Ala Leu Trp Arg Val Ser Ala Glu Glu Tyr Val Glu
   2085 2090 2095
   Ile Arg Arg Val Gly Asp Phe His Tyr Val Ser Gly Met Thr Thr Asp
   2100 2105 2110
   Asn Leu Lys Cys Pro Cys Gln Ile Pro Ser Pro Glu Phe Phe Thr Glu
   2115 2120 2125
   Leu Asp Gly Val Arg Leu His Arg Phe Ala Pro Pro Cys Lys Pro Leu
   2130 2135 2140
   Leu Arg Glu Glu Val Ser Phe Arg Val Gly Leu His Glu Tyr Pro Val
   2145 2150 2155 2160
   Gly Ser Gln Leu Pro Cys Glu Pro Glu Pro Asp Val Ala Val Leu Thr
   2165 2170 2175
   Ser Met Leu Thr Asp Pro Ser His Ile Thr Ala Glu Ala Ala Gly Arg
   2180 2185 2190
   Arg Leu Ala Arg Gly Ser Pro Pro Ser Met Ala Ser Ser Ser Ala Ser
   2195 2200 2205
   Gln Leu Ser Ala Pro Ser Leu Lys Ala Thr Cys Thr Ala Asn His Asp
   2210 2215 2220
   Ser Pro Asp Ala Glu Leu Ile Glu Ala Asn Leu Leu Trp Arg Gln Glu
   2225 2230 2235 2240
   Met Gly Gly Asn Ile Thr Arg Val Glu Ser Glu Asn Lys Val Val Ile
   2245 2250 2255
   Leu Asp Ser Phe Asp Pro Leu Val Ala Glu Glu Asp Glu Arg Glu Val
   2260 2265 2270
   Ser Val Pro Ala Glu Ile Leu Arg Lys Ser Arg Arg Phe Ala Arg Ala
   2275 2280 2285
   Leu Pro Val Trp Ala Arg Pro Asp Tyr Asn Pro Pro Leu Val Glu Thr
   2290 2295 2300
   Trp Lys Lys Pro Asp Tyr Glu Pro Pro Val Val His Gly Cys Pro Leu
   2305 2310 2315 2320
   Pro Pro Pro Arg Ser Pro Pro Val Pro Pro Pro Arg Lys Lys Arg Thr
   2325 2330 2335
   Val Val Leu Thr Glu Ser Thr Leu Ser Thr Ala Leu Ala Glu Leu Ala
   2340 2345 2350
   Thr Lys Ser Phe Gly Ser Ser Ser Thr Ser Gly Ile Thr Gly Asp Asn
   2355 2360 2365
   Thr Thr Thr Ser Ser Glu Pro Ala Pro Ser Gly Cys Pro Pro Asp Ser
   2370 2375 2380
   Asp Val Glu Ser Tyr Ser Ser Met Pro Pro Leu Glu Gly Glu Pro Gly
   2385 2390 2395 2400
   Asp Pro Asp Leu Ser Asp Gly Ser Trp Ser Thr Val Ser Ser Gly Ala
   2405 2410 2415
   Asp Thr Glu Asp Val Val Cys Cys Ser Met Ser Tyr Ser Trp Thr Gly
   2420 2425 2430
   Ala Leu Val Thr Pro Cys Ala Ala Glu Glu Gln Lys Leu Pro Ile Asn
   2435 2440 2445
   Ala Leu Ser Asn Ser Leu Leu Arg His His Asn Leu Val Tyr Ser Thr
   2450 2455 2460
   Thr Ser Arg Ser Ala Cys Gln Arg Gln Lys Lys Val Thr Phe Asp Arg
   2465 2470 2475 2480
   Leu Gln Val Leu Asp Ser His Tyr Gln Asp Val Leu Lys Glu Val Lys
   2485 2490 2495
   Ala Ala Ala Ser Lys Val Lys Ala Asn Leu Leu Ser Val Glu Glu Ala
   2500 2505 2510
   Cys Ser Leu Thr Pro Pro His Ser Ala Lys Ser Lys Phe Gly Tyr Gly
   2515 2520 2525
   Ala Lys Asp Val Arg Cys His Ala Arg Lys Ala Val Ala His Ile Asn
   2530 2535 2540
   Ser Val Trp Lys Asp Leu Leu Glu Asp Ser Val Thr Pro Ile Asp Thr
   2545 2550 2555 2560
   Thr Ile Met Ala Lys Asn Glu Val Phe Cys Val Gln Pro Glu Lys Gly
   2565 2570 2575
   Gly Arg Lys Pro Ala Arg Leu Ile Val Phe Pro Asp Leu Gly Val Arg
   2580 2585 2590
   Val Cys Glu Lys Met Ala Leu Tyr Asp Val Val Ser Lys Leu Pro Leu
   2595 2600 2605
   Ala Val Met Gly Ser Ser Tyr Gly Phe Gln Tyr Ser Pro Gly Gln Arg
   2610 2615 2620
   Val Glu Phe Leu Val Gln Ala Trp Lys Ser Lys Lys Thr Pro Met Gly
   2625 2630 2635 2640
   Phe Ser Tyr Asp Thr Arg Cys Phe Asp Ser Thr Val Thr Glu Ser Asp
   2645 2650 2655
   Ile Arg Thr Glu Glu Ala Ile Tyr Gln Cys Cys Asp Leu Asp Pro Gln
   2660 2665 2670
   Ala Arg Val Ala Ile Lys Ser Leu Thr Glu Arg Leu Tyr Val Gly Gly
   2675 2680 2685
   Pro Leu Thr Asn Ser Arg Gly Glu Asn Cys Gly Tyr Arg Arg Cys Arg
   2690 2695 2700
   Ala Ser Gly Val Leu Thr Thr Ser Cys Gly Asn Thr Leu Thr Cys Tyr
   2705 2710 2715 2720
   Ile Lys Ala Arg Ala Ala Cys Arg Ala Ala Gly Leu Gln Asp Cys Thr
   2725 2730 2735
   Met Leu Val Cys Gly Asp Asp Leu Val Val Ile Cys Glu Ser Ala Gly
   2740 2745 2750
   Val Gln Glu Asp Ala Ala Ser Leu Arg Ala Phe Thr Glu Ala Met Thr
   2755 2760 2765
   Arg Tyr Ser Ala Pro Pro Gly Asp Pro Pro Gln Pro Glu Tyr Asp Leu
   2770 2775 2780
   Glu Leu Ile Thr Ser Cys Ser Ser Asn Val Ser Val Ala His Asp Gly
   2785 2790 2795 2800
   Ala Gly Lys Arg Val Tyr Tyr Leu Thr Arg Asp Pro Thr Thr Pro Leu
   2805 2810 2815
   Ala Arg Ala Ala Trp Glu Thr Ala Arg His Thr Pro Val Asn Ser Trp
   2820 2825 2830
   Leu Gly Asn Ile Ile Met Phe Ala Pro Thr Leu Trp Ala Arg Met Ile
   2835 2840 2845
   Leu Met Thr His Phe Phe Ser Val Leu Ile Ala Arg Asp Gln Leu Glu
   2850 2855 2860
   Gln Ala Leu Asn Cys Glu Ile Tyr Gly Ala Cys Tyr Ser Ile Glu Pro
   2865 2870 2875 2880
   Leu Asp Leu Pro Pro Ile Ile Gln Arg Leu His Gly Leu Ser Ala Phe
   2885 2890 2895
   Ser Leu His Ser Tyr Ser Pro Gly Glu Ile Asn Arg Val Ala Ala Cys
   2900 2905 2910
   Leu Arg Lys Leu Gly Val Pro Pro Leu Arg Ala Trp Arg His Arg Ala
   2915 2920 2925
   Arg Ser Val Arg Ala Arg Leu Leu Ser Arg Gly Gly Arg Ala Ala Ile
   2930 2935 2940
   Cys Gly Lys Tyr Leu Phe Asn Trp Ala Val Arg Thr Lys Leu Lys Leu
   2945 2950 2955 2960
   Thr Pro Ile Ala Ala Ala Gly Arg Leu Asp Leu Ser Gly Trp Phe Thr
   2965 2970 2975
   Ala Gly Tyr Ser Gly Gly Asp Ile Tyr His Ser Val Ser His Ala Arg
   2980 2985 2990
   Pro Arg Trp Phe Trp Phe Cys Leu Leu Leu Leu Ala Ala Gly Val Gly
   2995 3000 3005
   Ile Tyr Leu Leu Pro Asn Arg
   3010 3015
<210> 7
   <211> 9611
   <212> DNA
   <213> Hepatitis C virus
<400> 7
   gccagccccc tgatgggggc gacactccac catgaatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gataaacccg ctcaatgcct ggagatttgg gcgtgccccc 240
   gcaagactgc tagccgagta gtgttgggtc gcgaaaggcc ttgtggtact gcctgatagg 300
   gtgcttgcga gtgccccggg aggtctcgta gaccgtgcac catgagcaca aatcctaaac 360
   ctcaaagaaa aaccaaaaga aacaccaacc gtcgcccaca agacgttaag tttccgggcg 420
   gcggccagat cgttggcgga gtatacttgt tgccgcgcag gggccccagg ttgggtgtgc 480
   gcgcgacaag gaagacttcg gagcggtccc agccacgtgg aaggcgccag cccatcccta 540
   aagatcggcg ctccactggc aaatcctggg gaaaaccagg atacccctgg cccctatacg 600
   ggaatgaggg actcggctgg gcaggatggc tcctgtcccc ccgaggttcc cgtccctctt 660
   ggggccccaa tgacccccgg cataggtcgc gcaacgtggg taaggtcatc gataccctaa 720
   cgtgcggctt tgccgacctc atggggtaca tccctgtcgt gggcgccccg ctcggcggcg 780
   tcgccagagc tctcgcgcat ggcgtgagag tcctggagga cggggttaat tttgcaacag 840
   ggaacttacc cggttgctcc ttttctatct tcttgctggc cctgctgtcc tgcatcacca 900
   ccccggtctc cgctgccgaa gtgaagaaca tcagtaccgg ctacatggtg actaacgact 960
   gcaccaatga cagcattacc tggcagctcc aggctgctgt cctccacgtc cccgggtgcg 1020
   tcccgtgcga gaaagtgggg aatgcatctc agtgctggat accggtctca ccgaatgtgg 1080
   ccgtgcagcg gcccggcgcc ctcacgcagg gcttgcggac gcacatcgac atggttgtga 1140
   tgtccgccac gctctgctct gccctctacg tgggggacct ctgcggtggg gtgatgctcg 1200
   cagcccaaat gttcattgtc tcgccgcagc accactggtt tgtccaagac tgcaattgct 1260
   ccatctaccc tggtaccatc actggacacc gcatggcatg ggacatgatg atgaactggt 1320
   cgcccacggc taccatgatc ttggcgtacg cgatgcgtgt ccccgaggtc attatagaca 1380
   tcattagcgg ggctcattgg ggcgtcatgt tcggcttggc ctacttctct atgcagggag 1440
   cgtgggcgaa agtcgttgtc atccttctgt tggccgccgg ggtggacgcg cgcacccata 1500
   ctgttggggg ttctgccgcg cagaccaccg ggcgcctcac cagcttattt gacatgggcc 1560
   ccaggcagaa aatccagctc gttaacacca atggcagctg gcacatcaac cgcaccgccc 1620
   tgaactgcaa tgactccttg cacaccggct ttatcgcgtc tctgttctac acccacagct 1680
   tcaactcgtc aggatgtccc gaacgcatgt ccgcctgccg cagtatcgag gccttccggg 1740
   tgggatgggg cgccttgcaa tatgaggata atgtcaccaa tccagaggat atgagaccct 1800
   attgctggca ctacccacca aggcagtgtg gcgtggtctc cgcgaagact gtgtgtggcc 1860
   cagtgtactg tttcaccccc agcccagtgg tagtgggcac gaccgacagg cttggagcgc 1920
   ccacttacac gtggggggag aatgagacag atgtcttcct attgaacagc actcgaccac 1980
   cgctggggtc atggttcggc tgcacgtgga tgaactcttc tggctacacc aagacttgcg 2040
   gcgcaccacc ctgccgtact agagctgact tcaacgccag cacggacctg ttgtgcccca 2100
   cggactgttt taggaagcat cctgatacca cttacctcaa atgcggctct gggccctggc 2160
   tcacgccaag gtgcctgatc gactacccct acaggctctg gcattacccc tgcacagtta 2220
   actataccat cttcaaaata aggatgtatg tgggaggggt tgagcacagg ctcacggctg 2280
   catgcaattt cactcgtggg gatcgttgca acttggagga cagagacaga agtcaactgt 2340
   ctcctttgtt gcactccacc acggaatggg ccattttacc ttgctcttac tcggacctgc 2400
   ccgccttgtc gactggtctt ctccacctcc accaaaacat cgtggacgta caattcatgt 2460
   atggcctatc acctgccctc acaaaataca tcgtccgatg ggagtgggta atactcttat 2520
   tcctgctctt agcggacgcc agggtttgcg cctgcttatg gatgctcatc ttgttgggcc 2580
   aggccgaagc agcactagag aagctggtca tcttgcacgc tgcgagcgca gctagctgca 2640
   atggcttcct atattttgtc atctttttcg tggctgcttg gtacatcaag ggtcgggtag 2700
   tccccttagc tacctattcc ctcactggcc tgtggtcctt tagcctactg ctcctagcat 2760
   tgccccaaca ggcatatgca ctggacacgg aggtggccgc gtcgtgtggc ggcgttgttc 2820
   ttgtcgggtt aatggcgctg actctgtcgc catattacaa gcgctatatc agctggtgca 2880
   tgtggtggct tcagtatttt ctgaccagag tagaagcgca actgcacgtg tgggttcccc 2940
   ccctcaacgt ccgggggggg cgcgatgccg tcatcttact catgtgtgta gtacacccga 3000
   ccctggtatt tgacatcacc aaactactcc tggccatctt cggacccctt tggattcttc 3060
   aagccagttt gcttaaagtc ccctacttcg tgcgcgttca aggccttctc cggatctgcg 3120
   cgctagcgcg gaagatagcc ggaggtcatt acgtgcaaat ggccatcatc aagttagggg 3180
   cgcttactgg cacctatgtg tataaccatc tcacccctct tcgagactgg gcgcacaacg 3240
   gcctgcgaga tctggccgtg gctgtggaac cagtcgtctt ctcccgaatg gagaccaagc 3300
   tcatcacgtg gggggcagat accgccgcgt gcggtgacat catcaacggc ttgcccgtct 3360
   ctgcccgtag gggccaggag atactgcttg ggccagccga cggaatggtc tccaaggggt 3420
   ggaggttgct ggcgcccatc acggcgtacg cccagcagac gagaggcctc ctagggtgta 3480
   taatcaccag cctgactggc cgggacaaaa accaagtgga gggtgaggtc cagatcgtgt 3540
   caactgctac ccaaaccttc ctggcaacgt gcatcaatgg ggtatgctgg actgtctacc 3600
   acggggccgg aacgaggacc atcgcatcac ccaagggtcc tgtcatccag atgtatacca 3660
   atgtggacca agaccttgtg ggctggcccg ctcctcaagg ttcccgctca ttgacaccct 3720
   gtacctgcgg ctcctcggac ctttacctgg tcacgaggca cgccgatgtc attcccgtgc 3780
   gccggcgagg tgatagcagg ggtagcctgc tttcgccccg gcccatttcc tacttgaaag 3840
   gctcctcggg gggtccgctg ttgtgccccg cgggacacgc cgtgggccta ttcagggccg 3900
   cggtgtgcac ccgtggagtg gctaaagcgg tggactttat ccctgtggag aacctaggga 3960
   caaccatgag atccccggtg ttcacggaca actcctctcc accagcagtg ccccagagct 4020
   tccaggtggc ccacctgcat gctcccaccg gcagcggtaa gagcaccaag gtcccggctg 4080
   cgtacgcagc ccagggctac aaggtgttgg tgctcaaccc ctctgttgct gcaacgctgg 4140
   gctttggtgc ttacatgtcc aaggcccatg gggttgatcc taatatcagg accggggtga 4200
   gaacaattac cactggcagc cccatcacgt actccaccta cggcaagttc cttgccgacg 4260
   gcgggtgctc aggaggtgct tatgacataa taatttgtga cgagtgccac tccacggatg 4320
   ccacatccat cttgggcatc ggcactgtcc ttgaccaagc agagactgcg ggggcgagac 4380
   tggttgtgct cgccactgct acccctccgg gctccgtcac tgtgtcccat cctaacatcg 4440
   aggaggttgc tctgtccacc accggagaga tcccctttta cggcaaggct atccccctcg 4500
   aggtgatcaa ggggggaaga catctcatct tctgccactc aaagaagaag tgcgacgagc 4560
   tcgccgcgaa gctggtcgca ttgggcatca atgccgtggc ctactaccgc ggtcttgacg 4620
   tgtctgtcat cccgaccagc ggcgatgttg tcgtcgtgtc gaccgatgct ctcatgactg 4680
   gctttaccgg cgacttcgac tctgtgatag actgcaacac gtgtgtcact cagacagtcg 4740
   atttcagcct tgaccctacc tttaccattg agacaaccac gctcccccag gatgctgtct 4800
   ccaggactca acgccggggc aggactggca gggggaagcc aggcatctat agatttgtgg 4860
   caccggggga gcgcccctcc ggcatgttcg actcgtccgt cctctgtgag tgctatgacg 4920
   cgggctgtgc ttggtatgag ctcacgcccg ccgagactac agttaggcta cgagcgtaca 4980
   tgaacacccc ggggcttccc gtgtgccagg accatcttga attttgggag ggcgtcttta 5040
   cgggcctcac tcatatagat gcccactttt tatcccagac aaagcagagt ggggagaact 5100
   ttccttacct ggtagcgtac caagccaccg tgtgcgctag ggctcaagcc cctcccccat 5160
   cgtgggacca gatgtggaag tgtttgatcc gccttaaacc caccctccat gggccaacac 5220
   ccctgctata cagactgggc gctgttcaga atgaagtcac cctgacgcac ccaatcacca 5280
   aatacatcat gacatgcatg tcggccgacc tggaggtcgt cacgagcacc tgggtgctcg 5340
   ttggcggcgt cctggctgct ctggccgcgt attgcctgtc aacaggctgc gtggtcatag 5400
   tgggcaggat cgtcttgtcc gggaagccgg caattatacc tgacagggag gttctctacc 5460
   aggagttcga tgagatggaa gagtgctctc agcacttacc gtacatcgag caagggatga 5520
   tgctcgctga gcagttcaag cagaaggccc tcggcctcct gcagaccgcg tcccgccatg 5580
   cagaggttat cacccctgct gtccagacca actggcagaa actcgaggtc ttttgggcga 5640
   agcacatgtg gaatttcatc agtgggatac aatacttggc gggcctgtca acgctgcctg 5700
   gtaaccccgc cattgcttca ttgatggctt ttacagctgc cgtcaccagc ccactaacca 5760
   ctggccaaac cctcctcttc aacatattgg gggggtgggt ggctgcccag ctcgccgccc 5820
   ccggtgccgc tactgccttt gtgggtgctg gcctagctgg cgccgccatc ggcagcgttg 5880
   gactggggaa ggtcctcgtg gacattcttg cagggtatgg cgcgggcgtg gcgggagctc 5940
   ttgtagcatt caagatcatg agcggtgagg tcccctccac ggaggacctg gtcaatctgc 6000
   tgcccgccat cctctcgcct ggagcccttg tagtcggtgt ggtctgcgca gcaatactgc 6060
   gccggcacgt tggcccgggc gagggggcag tgcaatggat gaaccggcta atagccttcg 6120
   cctcccgggg gaaccatgtt tcccccacgc actacgtgcc ggagagcgat gcagccgccc 6180
   gcgtcactgc catactcagc agcctcactg taacccagct cctgaggcga ctgcatcagt 6240
   ggataagctc ggagtgtacc actccatgct ccggttcctg gctaagggac atctgggact 6300
   ggatatgcga ggtgctgagc gactttaaga cctggctgaa agccaagctc atgccacaac 6360
   tgcctgggat tccctttgtg tcctgccagc gcgggtatag gggggtctgg cgaggagacg 6420
   gcattatgca cactcgctgc cactgtggag ctgagatcac tggacatgtc aaaaacggga 6480
   cgatgaggat cgtcggtcct aggacctgca ggaacatgtg gagtgggacg ttccccatta 6540
   acgcctacac cacgggcccc tgtactcccc ttcctgcgcc gaactataag ttcgcgctgt 6600
   ggagggtgtc tgcagaggaa tacgtggaga taaggcgggt gggggacttc cactacgtat 6660
   cgggtatgac tactgacaat cttaaatgcc cgtgccagat cccatcgccc gaatttttca 6720
   cagaattgga cggggtgcgc ctacacaggt ttgcgccccc ttgcaagccc ttgctgcggg 6780
   aggaggtatc attcagagta ggactccacg agtacccggt ggggtcgcaa ttaccttgcg 6840
   agcccgaacc ggacgtagcc gtgttgacgt ccatgctcac tgatccctcc catataacag 6900
   cagaggcggc cgggagaagg ttggcgagag ggtcaccccc ttctatggcc agctcctcgg 6960
   ctagccagct gtccgctcca tctctcaagg caacttgcac cgccaaccat gactcccctg 7020
   acgccgagct catagaggct aacctcctgt ggaggcagga gatgggcggc aacatcacca 7080
   gggttgagtc agagaacaaa gtggtgattc tggactcctt cgatccgctt gtggcagagg 7140
   aggatgagcg ggaggtctcc gtacctgcag aaattctgcg gaagtctcgg agattcgccc 7200
   gggccctgcc cgtctgggcg cggccggact acaacccccc gctagtagag acgtggaaaa 7260
   agcctgacta cgaaccacct gtggtccatg gctgcccgct accacctcca cggtcccctc 7320
   ctgtgcctcc gcctcggaaa aagcgtacgg tggtcctcac cgaatcaacc ctatctactg 7380
   ccttggccga gcttgccacc aaaagttttg gcagctcctc aacttccggc attacgggcg 7440
   acaatacgac aacatcctct gagcccgccc cttctggctg cccccccgac tccgacgttg 7500
   agtcctattc ttccatgccc cccctggagg gggagcctgg ggatccggat ctcagcgacg 7560
   ggtcatggtc gacggtcagt agtggggccg acacggaaga tgtcgtgtgc tgctcaatgt 7620
   cttattcctg gacaggcgca ctcgtcaccc cgtgcgctgc ggaagaacaa aaactgccca 7680
   tcaacgcact gagcaactcg ttgctacgcc atcacaatct ggtgtattcc accacttcac 7740
   gcagtgcttg ccaaaggcag aagaaagtca catttgacag actgcaagtt ctggacagcc 7800
   attaccagga cgtgctcaag gaggtcaaag cagcggcgtc aaaagtgaag gctaacttgc 7860
   tatccgtaga ggaagcttgc agcctgacgc ccccacattc agccaaatcc aagtttggct 7920
   atggggcaaa agacgtccgt tgccatgcca gaaaggccgt agcccacatc aactccgtgt 7980
   ggaaagacct tctggaagac agtgtaacac caatagacac taccatcatg gccaagaacg 8040
   aggttttctg cgttcagcct gagaaggggg gtcgtaagcc agctcgtctc atcgtgttcc 8100
   ccgacctggg cgtgcgcgtg tgcgagaaga tggccctgta cgacgtggtt agcaagctcc 8160
   ccctggccgt gatgggaagc tcctacggat tccaatactc accaggacag cgggttgaat 8220
   tcctcgtgca agcgtggaag tccaagaaga ccccgatggg gttctcgtat gatacccgct 8280
   gttttgactc cacagtcact gagagcgaca tccgtacgga ggaggcaatt taccaatgtt 8340
   gtgacctgga cccccaagcc cgcgtggcca tcaagtccct cactgagagg ctttatgttg 8400
   ggggccctct taccaattca aggggggaaa actgcggcta ccgcaggtgc cgcgcgagcg 8460
   gcgtactgac aactagctgt ggtaacaccc tcacttgcta catcaaggcc cgggcagcct 8520
   gtcgagccgc agggctccag gactgcacca tgctcgtgtg tggcgacgac ttagtcgtta 8580
   tctgtgaaag tgcgggggtc caggaggacg cggcgagcct gagagccttc acggaggcta 8640
   tgaccaggta ctccgccccc cccggggacc ccccacaacc agaatacgac ttggagctta 8700
   taacatcatg ctcctccaac gtgtcagtcg cccacgacgg cgctggaaag agggtctact 8760
   accttacccg tgaccctaca acccccctcg cgagagccgc gtgggagaca gcaagacaca 8820
   ctccagtcaa ttcctggcta ggcaacataa tcatgtttgc ccccacactg tgggcgagga 8880
   tgatactgat gacccatttc tttagcgtcc tcatagccag ggatcagctt gaacaggctc 8940
   ttaactgtga gatctacgga gcctgctact ccatagaacc actggatcta cctccaatca 9000
   ttcaaagact ccatggcctc agcgcatttt cactccacag ttactctcca ggtgaaatca 9060
   atagggtggc cgcatgcctc agaaaacttg gggtcccgcc cttgcgagct tggagacacc 9120
   gggcccggag cgtccgcgct aggcttctgt ccagaggagg cagggctgct atatgtggca 9180
   agtacctctt caactgggca gtaagaacaa agctcaaact cactccaata gcggccgctg 9240
   gccggctgga cttgtccggt tggttcacgg ctggctacag cgggggagac atttatcaca 9300
   gcgtgtctca tgcccggccc cgctggttct ggttttgcct actcctgctc gctgcagggg 9360
   taggcatcta cctcctcccc aaccgatgaa ggttggggta aacactccgg cctcttaagc 9420
   catttcctgt tttttttttt tttttttttt tttttttctt tttttttttc tttcctttcc 9480
   ttcttttttt cctttctttt tcccttcttt aatggtggct ccatcttagc cctagtcacg 9540
   gctagctgtg aaaggtccgt gagccgcatg actgcagaga gtgctgatac tggcctctct 9600
   gcagatcatg t 9611
<210> 8
   <211> 3015
   <212> PRT
   <213> Hepatitis C virus
<400> 8
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Asn Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
   195 200 205
   Asn Asp Ser Ile Thr Trp Gln Leu Gln Ala Ala Val Leu His Val Pro
   210 215 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Ile Leu Leu Phe Leu Leu Leu Ala Asp Ala Arg Val Cys
   725 730 735
   Ala Cys Leu Trp Met Leu Ile Leu Leu Gly Gln Ala Glu Ala Ala Leu
   740 745 750
   Glu Lys Leu Val Ile Leu His Ala Ala Ser Ala Ala Ser Cys Asn Gly
   755 760 765
   Phe Leu Tyr Phe Val Ile Phe Phe Val Ala Ala Trp Tyr Ile Lys Gly
   770 775 780
   Arg Val Val Pro Leu Ala Thr Tyr Ser Leu Thr Gly Leu Trp Ser Phe
   785 790 795 800
   Ser Leu Leu Leu Leu Ala Leu Pro Gln Gln Ala Tyr Ala Leu Asp Thr
   805 810 815
   Glu Val Ala Ala Ser Cys Gly Gly Val Val Leu Val Gly Leu Met Ala
   820 825 830
   Leu Thr Leu Ser Pro Tyr Tyr Lys Arg Tyr Ile Ser Trp Cys Met Trp
   835 840 845
   Trp Leu Gln Tyr Phe Leu Thr Arg Val Glu Ala Gln Leu His Val Trp
   850 855 860
   Val Pro Pro Leu Asn Val Arg Gly Gly Arg Asp Ala Val Ile Leu Leu
   865 870 875 880
   Met Cys Val Val His Pro Thr Leu Val Phe Asp Ile Thr Lys Leu Leu
   885 890 895
   Leu Ala Ile Phe Gly Pro Leu Trp Ile Leu Gln Ala Ser Leu Leu Lys
   900 905 910
   Val Pro Tyr Phe Val Arg Val Gln Gly Leu Leu Arg Ile Cys Ala Leu
   915 920 925
   Ala Arg Lys Ile Ala Gly Gly His Tyr Val Gln Met Ala Ile Ile Lys
   930 935 940
   Leu Gly Ala Leu Thr Gly Thr Tyr Val Tyr Asn His Leu Thr Pro Leu
   945 950 955 960
   Arg Asp Trp Ala His Asn Gly Leu Arg Asp Leu Ala Val Ala Val Glu
   965 970 975
   Pro Val Val Phe Ser Arg Met Glu Thr Lys Leu Ile Thr Trp Gly Ala
   980 985 990
   Asp Thr Ala Ala Cys Gly Asp Ile Ile Asn Gly Leu Pro Val Ser Ala
   995 1000 1005
   Arg Arg Gly Gln Glu Ile Leu Leu Gly Pro Ala Asp Gly Met Val Ser
   1010 1015 1020
   Lys Gly Trp Arg Leu Leu Ala Pro Ile Thr Ala Tyr Ala Gln Gln Thr
   1025 1030 1035 1040
   Arg Gly Leu Leu Gly Cys Ile Ile Thr Ser Leu Thr Gly Arg Asp Lys
   1045 1050 1055
   Asn Gln Val Glu Gly Glu Val Gln Ile Val Ser Thr Ala Thr Gln Thr
   1060 1065 1070
   Phe Leu Ala Thr Cys Ile Asn Gly Val Cys Trp Thr Val Tyr His Gly
   1075 1080 1085
   Ala Gly Thr Arg Thr Ile Ala Ser Pro Lys Gly Pro Val Ile Gln Met
   1090 1095 1100
   Tyr Thr Asn Val Asp Gln Asp Leu Val Gly Trp Pro Ala Pro Gln Gly
   1105 1110 1115 1120
   Ser Arg Ser Leu Thr Pro Cys Thr Cys Gly Ser Ser Asp Leu Tyr Leu
   1125 1130 1135
   Val Thr Arg His Ala Asp Val Ile Pro Val Arg Arg Arg Gly Asp Ser
   1140 1145 1150
   Arg Gly Ser Leu Leu Ser Pro Arg Pro Ile Ser Tyr Leu Lys Gly Ser
   1155 1160 1165
   Ser Gly Gly Pro Leu Leu Cys Pro Ala Gly His Ala Val Gly Leu Phe
   1170 1175 1180
   Arg Ala Ala Val Cys Thr Arg Gly Val Ala Lys Ala Val Asp Phe Ile
   1185 1190 1195 1200
   Pro Val Glu Asn Leu Gly Thr Thr Met Arg Ser Pro Val Phe Thr Asp
   1205 1210 1215
   Asn Ser Ser Pro Pro Ala Val Pro Gln Ser Phe Gln Val Ala His Leu
   1220 1225 1230
   His Ala Pro Thr Gly Ser Gly Lys Ser Thr Lys Val Pro Ala Ala Tyr
   1235 1240 1245
   Ala Ala Gln Gly Tyr Lys Val Leu Val Leu Asn Pro Ser Val Ala Ala
   1250 1255 1260
   Thr Leu Gly Phe Gly Ala Tyr Met Ser Lys Ala His Gly Val Asp Pro
   1265 1270 1275 1280
   Asn Ile Arg Thr Gly Val Arg Thr Ile Thr Thr Gly Ser Pro Ile Thr
   1285 1290 1295
   Tyr Ser Thr Tyr Gly Lys Phe Leu Ala Asp Gly Gly Cys Ser Gly Gly
   1300 1305 1310
   Ala Tyr Asp Ile Ile Ile Cys Asp Glu Cys His Ser Thr Asp Ala Thr
   1315 1320 1325
   Ser Ile Leu Gly Ile Gly Thr Val Leu Asp Gln Ala Glu Thr Ala Gly
   1330 1335 1340
   Ala Arg Leu Val Val Leu Ala Thr Ala Thr Pro Pro Gly Ser Val Thr
   1345 1350 1355 1360
   Val Ser His Pro Asn Ile Glu Glu Val Ala Leu Ser Thr Thr Gly Glu
   1365 1370 1375
   Ile Pro Phe Tyr Gly Lys Ala Ile Pro Leu Glu Val Ile Lys Gly Gly
   1380 1385 1390
   Arg His Leu Ile Phe Cys His Ser Lys Lys Lys Cys Asp Glu Leu Ala
   1395 1400 1405
   Ala Lys Leu Val Ala Leu Gly Ile Asn Ala Val Ala Tyr Tyr Arg Gly
   1410 1415 1420
   Leu Asp Val Ser Val Ile Pro Thr Ser Gly Asp Val Val Val Val Ser
   1425 1430 1435 1440
   Thr Asp Ala Leu Met Thr Gly Phe Thr Gly Asp Phe Asp Ser Val Ile
   1445 1450 1455
   Asp Cys Asn Thr Cys Val Thr Gln Thr Val Asp Phe Ser Leu Asp Pro
   1460 1465 1470
   Thr Phe Thr Ile Glu Thr Thr Thr Leu Pro Gln Asp Ala Val Ser Arg
   1475 1480 1485
   Thr Gln Arg Arg Gly Arg Thr Gly Arg Gly Lys Pro Gly Ile Tyr Arg
   1490 1495 1500
   Phe Val Ala Pro Gly Glu Arg Pro Ser Gly Met Phe Asp Ser Ser Val
   1505 1510 1515 1520
   Leu Cys Glu Cys Tyr Asp Ala Gly Cys Ala Trp Tyr Glu Leu Thr Pro
   1525 1530 1535
   Ala Glu Thr Thr Val Arg Leu Arg Ala Tyr Met Asn Thr Pro Gly Leu
   1540 1545 1550
   Pro Val Cys Gln Asp His Leu Glu Phe Trp Glu Gly Val Phe Thr Gly
   1555 1560 1565
   Leu Thr His Ile Asp Ala His Phe Leu Ser Gln Thr Lys Gln Ser Gly
   1570 1575 1580
   Glu Asn Phe Pro Tyr Leu Val Ala Tyr Gln Ala Thr Val Cys Ala Arg
   1585 1590 1595 1600
   Ala Gln Ala Pro Pro Pro Ser Trp Asp Gln Met Trp Lys Cys Leu Ile
   1605 1610 1615
   Arg Leu Lys Pro Thr Leu His Gly Pro Thr Pro Leu Leu Tyr Arg Leu
   1620 1625 1630
   Gly Ala Val Gln Asn Glu Val Thr Leu Thr His Pro Ile Thr Lys Tyr
   1635 1640 1645
   Ile Met Thr Cys Met Ser Ala Asp Leu Glu Val Val Thr Ser Thr Trp
   1650 1655 1660
   Val Leu Val Gly Gly Val Leu Ala Ala Leu Ala Ala Tyr Cys Leu Ser
   1665 1670 1675 1680
   Thr Gly Cys Val Val Ile Val Gly Arg Ile Val Leu Ser Gly Lys Pro
   1685 1690 1695
   Ala Ile Ile Pro Asp Arg Glu Val Leu Tyr Gln Glu Phe Asp Glu Met
   1700 1705 1710
   Glu Glu Cys Ser Gln His Leu Pro Tyr Ile Glu Gln Gly Met Met Leu
   1715 1720 1725
   Ala Glu Gln Phe Lys Gln Lys Ala Leu Gly Leu Leu Gln Thr Ala Ser
   1730 1735 1740
   Arg His Ala Glu Val Ile Thr Pro Ala Val Gln Thr Asn Trp Gln Lys
   1745 1750 1755 1760
   Leu Glu Val Phe Trp Ala Lys His Met Trp Asn Phe Ile Ser Gly Ile
   1765 1770 1775
   Gln Tyr Leu Ala Gly Leu Ser Thr Leu Pro Gly Asn Pro Ala Ile Ala
   1780 1785 1790
   Ser Leu Met Ala Phe Thr Ala Ala Val Thr Ser Pro Leu Thr Thr Gly
   1795 1800 1805
   Gln Thr Leu Leu Phe Asn Ile Leu Gly Gly Trp Val Ala Ala Gln Leu
   1810 1815 1820
   Ala Ala Pro Gly Ala Ala Thr Ala Phe Val Gly Ala Gly Leu Ala Gly
   1825 1830 1835 1840
   Ala Ala Ile Gly Ser Val Gly Leu Gly Lys Val Leu Val Asp Ile Leu
   1845 1850 1855
   Ala Gly Tyr Gly Ala Gly Val Ala Gly Ala Leu Val Ala Phe Lys Ile
   1860 1865 1870
   Met Ser Gly Glu Val Pro Ser Thr Glu Asp Leu Val Asn Leu Leu Pro
   1875 1880 1885
   Ala Ile Leu Ser Pro Gly Ala Leu Val Val Gly Val Val Cys Ala Ala
   1890 1895 1900
   Ile Leu Arg Arg His Val Gly Pro Gly Glu Gly Ala Val Gln Trp Met
   1905 1910 1915 1920
   Asn Arg Leu Ile Ala Phe Ala Ser Arg Gly Asn His Val Ser Pro Thr
   1925 1930 1935
   His Tyr Val Pro Glu Ser Asp Ala Ala Ala Arg Val Thr Ala Ile Leu
   1940 1945 1950
   Ser Ser Leu Thr Val Thr Gln Leu Leu Arg Arg Leu His Gln Trp Ile
   1955 1960 1965
   Ser Ser Glu Cys Thr Thr Pro Cys Ser Gly Ser Trp Leu Arg Asp Ile
   1970 1975 1980
   Trp Asp Trp Ile Cys Glu Val Leu Ser Asp Phe Lys Thr Trp Leu Lys
   1985 1990 1995 2000
   Ala Lys Leu Met Pro Gln Leu Pro Gly Ile Pro Phe Val Ser Cys Gln
   2005 2010 2015
   Arg Gly Tyr Arg Gly Val Trp Arg Gly Asp Gly Ile Met His Thr Arg
   2020 2025 2030
   Cys His Cys Gly Ala Glu Ile Thr Gly His Val Lys Asn Gly Thr Met
   2035 2040 2045
   Arg Ile Val Gly Pro Arg Thr Cys Arg Asn Met Trp Ser Gly Thr Phe
   2050 2055 2060
   Pro Ile Asn Ala Tyr Thr Thr Gly Pro Cys Thr Pro Leu Pro Ala Pro
   2065 2070 2075 2080
   Asn Tyr Lys Phe Ala Leu Trp Arg Val Ser Ala Glu Glu Tyr Val Glu
   2085 2090 2095
   Ile Arg Arg Val Gly Asp Phe His Tyr Val Ser Gly Met Thr Thr Asp
   2100 2105 2110
   Asn Leu Lys Cys Pro Cys Gln Ile Pro Ser Pro Glu Phe Phe Thr Glu
   2115 2120 2125
   Leu Asp Gly Val Arg Leu His Arg Phe Ala Pro Pro Cys Lys Pro Leu
   2130 2135 2140
   Leu Arg Glu Glu Val Ser Phe Arg Val Gly Leu His Glu Tyr Pro Val
   2145 2150 2155 2160
   Gly Ser Gln Leu Pro Cys Glu Pro Glu Pro Asp Val Ala Val Leu Thr
   2165 2170 2175
   Ser Met Leu Thr Asp Pro Ser His Ile Thr Ala Glu Ala Ala Gly Arg
   2180 2185 2190
   Arg Leu Ala Arg Gly Ser Pro Pro Ser Met Ala Ser Ser Ser Ala Ser
   2195 2200 2205
   Gln Leu Ser Ala Pro Ser Leu Lys Ala Thr Cys Thr Ala Asn His Asp
   2210 2215 2220
   Ser Pro Asp Ala Glu Leu Ile Glu Ala Asn Leu Leu Trp Arg Gln Glu
   2225 2230 2235 2240
   Met Gly Gly Asn Ile Thr Arg Val Glu Ser Glu Asn Lys Val Val Ile
   2245 2250 2255
   Leu Asp Ser Phe Asp Pro Leu Val Ala Glu Glu Asp Glu Arg Glu Val
   2260 2265 2270
   Ser Val Pro Ala Glu Ile Leu Arg Lys Ser Arg Arg Phe Ala Arg Ala
   2275 2280 2285
   Leu Pro Val Trp Ala Arg Pro Asp Tyr Asn Pro Pro Leu Val Glu Thr
   2290 2295 2300
   Trp Lys Lys Pro Asp Tyr Glu Pro Pro Val Val His Gly Cys Pro Leu
   2305 2310 2315 2320
   Pro Pro Pro Arg Ser Pro Pro Val Pro Pro Pro Arg Lys Lys Arg Thr
   2325 2330 2335
   Val Val Leu Thr Glu Ser Thr Leu Ser Thr Ala Leu Ala Glu Leu Ala
   2340 2345 2350
   Thr Lys Ser Phe Gly Ser Ser Ser Thr Ser Gly Ile Thr Gly Asp Asn
   2355 2360 2365
   Thr Thr Thr Ser Ser Glu Pro Ala Pro Ser Gly Cys Pro Pro Asp Ser
   2370 2375 2380
   Asp Val Glu Ser Tyr Ser Ser Met Pro Pro Leu Glu Gly Glu Pro Gly
   2385 2390 2395 2400
   Asp Pro Asp Leu Ser Asp Gly Ser Trp Ser Thr Val Ser Ser Gly Ala
   2405 2410 2415
   Asp Thr Glu Asp Val Val Cys Cys Ser Met Ser Tyr Ser Trp Thr Gly
   2420 2425 2430
   Ala Leu Val Thr Pro Cys Ala Ala Glu Glu Gln Lys Leu Pro Ile Asn
   2435 2440 2445
   Ala Leu Ser Asn Ser Leu Leu Arg His His Asn Leu Val Tyr Ser Thr
   2450 2455 2460
   Thr Ser Arg Ser Ala Cys Gln Arg Gln Lys Lys Val Thr Phe Asp Arg
   2465 2470 2475 2480
   Leu Gln Val Leu Asp Ser His Tyr Gln Asp Val Leu Lys Glu Val Lys
   2485 2490 2495
   Ala Ala Ala Ser Lys Val Lys Ala Asn Leu Leu Ser Val Glu Glu Ala
   2500 2505 2510
   Cys Ser Leu Thr Pro Pro His Ser Ala Lys Ser Lys Phe Gly Tyr Gly
   2515 2520 2525
   Ala Lys Asp Val Arg Cys His Ala Arg Lys Ala Val Ala His Ile Asn
   2530 2535 2540
   Ser Val Trp Lys Asp Leu Leu Glu Asp Ser Val Thr Pro Ile Asp Thr
   2545 2550 2555 2560
   Thr Ile Met Ala Lys Asn Glu Val Phe Cys Val Gln Pro Glu Lys Gly
   2565 2570 2575
   Gly Arg Lys Pro Ala Arg Leu Ile Val Phe Pro Asp Leu Gly Val Arg
   2580 2585 2590
   Val Cys Glu Lys Met Ala Leu Tyr Asp Val Val Ser Lys Leu Pro Leu
   2595 2600 2605
   Ala Val Met Gly Ser Ser Tyr Gly Phe Gln Tyr Ser Pro Gly Gln Arg
   2610 2615 2620
   Val Glu Phe Leu Val Gln Ala Trp Lys Ser Lys Lys Thr Pro Met Gly
   2625 2630 2635 2640
   Phe Ser Tyr Asp Thr Arg Cys Phe Asp Ser Thr Val Thr Glu Ser Asp
   2645 2650 2655
   Ile Arg Thr Glu Glu Ala Ile Tyr Gln Cys Cys Asp Leu Asp Pro Gln
   2660 2665 2670
   Ala Arg Val Ala Ile Lys Ser Leu Thr Glu Arg Leu Tyr Val Gly Gly
   2675 2680 2685
   Pro Leu Thr Asn Ser Arg Gly Glu Asn Cys Gly Tyr Arg Arg Cys Arg
   2690 2695 2700
   Ala Ser Gly Val Leu Thr Thr Ser Cys Gly Asn Thr Leu Thr Cys Tyr
   2705 2710 2715 2720
   Ile Lys Ala Arg Ala Ala Cys Arg Ala Ala Gly Leu Gln Asp Cys Thr
   2725 2730 2735
   Met Leu Val Cys Gly Asp Asp Leu Val Val Ile Cys Glu Ser Ala Gly
   2740 2745 2750
   Val Gln Glu Asp Ala Ala Ser Leu Arg Ala Phe Thr Glu Ala Met Thr
   2755 2760 2765
   Arg Tyr Ser Ala Pro Pro Gly Asp Pro Pro Gln Pro Glu Tyr Asp Leu
   2770 2775 2780
   Glu Leu Ile Thr Ser Cys Ser Ser Asn Val Ser Val Ala His Asp Gly
   2785 2790 2795 2800
   Ala Gly Lys Arg Val Tyr Tyr Leu Thr Arg Asp Pro Thr Thr Pro Leu
   2805 2810 2815
   Ala Arg Ala Ala Trp Glu Thr Ala Arg His Thr Pro Val Asn Ser Trp
   2820 2825 2830
   Leu Gly Asn Ile Ile Met Phe Ala Pro Thr Leu Trp Ala Arg Met Ile
   2835 2840 2845
   Leu Met Thr His Phe Phe Ser Val Leu Ile Ala Arg Asp Gln Leu Glu
   2850 2855 2860
   Gln Ala Leu Asn Cys Glu Ile Tyr Gly Ala Cys Tyr Ser Ile Glu Pro
   2865 2870 2875 2880
   Leu Asp Leu Pro Pro Ile Ile Gln Arg Leu His Gly Leu Ser Ala Phe
   2885 2890 2895
   Ser Leu His Ser Tyr Ser Pro Gly Glu Ile Asn Arg Val Ala Ala Cys
   2900 2905 2910
   Leu Arg Lys Leu Gly Val Pro Pro Leu Arg Ala Trp Arg His Arg Ala
   2915 2920 2925
   Arg Ser Val Arg Ala Arg Leu Leu Ser Arg Gly Gly Arg Ala Ala Ile
   2930 2935 2940
   Cys Gly Lys Tyr Leu Phe Asn Trp Ala Val Arg Thr Lys Leu Lys Leu
   2945 2950 2955 2960
   Thr Pro Ile Ala Ala Ala Gly Arg Leu Asp Leu Ser Gly Trp Phe Thr
   2965 2970 2975
   Ala Gly Tyr Ser Gly Gly Asp Ile Tyr His Ser Val Ser His Ala Arg
   2980 2985 2990
   Pro Arg Trp Phe Trp Phe Cys Leu Leu Leu Leu Ala Ala Gly Val Gly
   2995 3000 3005
   Ile Tyr Leu Leu Pro Asn Arg
   3010 3015
<210> 9
   <211> 9611
   <212> DNA
   <213> Hepatitis C virus
<400> 9
   gccagccccc tgatgggggc gacactccac catgaatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gataaacccg ctcaatgcct ggagatttgg gcgtgccccc 240
   gcaagactgc tagccgagta gtgttgggtc gcgaaaggcc ttgtggtact gcctgatagg 300
   gtgcttgcga gtgccccggg aggtctcgta gaccgtgcac catgagcaca aatcctaaac 360
   ctcaaagaaa aaccaaaaga aacaccaacc gtcgcccaca agacgttaag tttccgggcg 420
   gcggccagat cgttggcgga gtatacttgt tgccgcgcag gggccccagg ttgggtgtgc 480
   gcgcgacaag gaagacttcg gagcggtccc agccacgtgg aaggcgccag cccatcccta 540
   aagatcggcg ctccactggc aaatcctggg gaaaaccagg atacccctgg cccctatacg 600
   ggaatgaggg actcggctgg gcaggatggc tcctgtcccc ccgaggttcc cgtccctctt 660
   ggggccccaa tgacccccgg cataggtcgc gcaacgtggg taaggtcatc gataccctaa 720
   cgtgcggctt tgccgacctc atggggtaca tccctgtcgt gggcgccccg ctcggcggcg 780
   tcgccagagc tctcgcgcat ggcgtgagag tcctggagga cggggttaat tttgcaacag 840
   ggaacttacc cggttgctcc ttttctatct tcttgctggc cctgctgtcc tgcatcacca 900
   ccccggtctc cgctgccgaa gtgaagaaca tcagtaccgg ctacatggtg actaacgact 960
   gcaccaatga cagcattacc tggcagctcc aggctgctgt cctccacgtc cccgggtgcg 1020
   tcccgtgcga gaaagtgggg aatgcatctc agtgctggat accggtctca ccgaatgtgg 1080
   ccgtgcagcg gcccggcgcc ctcacgcagg gcttgcggac gcacatcgac atggttgtga 1140
   tgtccgccac gctctgctct gccctctacg tgggggacct ctgcggtggg gtgatgctcg 1200
   cagcccaaat gttcattgtc tcgccgcagc accactggtt tgtccaagac tgcaattgct 1260
   ccatctaccc tggtaccatc actggacacc gcatggcatg ggacatgatg atgaactggt 1320
   cgcccacggc taccatgatc ttggcgtacg cgatgcgtgt ccccgaggtc attatagaca 1380
   tcattagcgg ggctcattgg ggcgtcatgt tcggcttggc ctacttctct atgcagggag 1440
   cgtgggcgaa agtcgttgtc atccttctgt tggccgccgg ggtggacgcg cgcacccata 1500
   ctgttggggg ttctgccgcg cagaccaccg ggcgcctcac cagcttattt gacatgggcc 1560
   ccaggcagaa aatccagctc gttaacacca atggcagctg gcacatcaac cgcaccgccc 1620
   tgaactgcaa tgactccttg cacaccggct ttatcgcgtc tctgttctac acccacagct 1680
   tcaactcgtc aggatgtccc gaacgcatgt ccgcctgccg cagtatcgag gccttccggg 1740
   tgggatgggg cgccttgcaa tatgaggata atgtcaccaa tccagaggat atgagaccct 1800
   attgctggca ctacccacca aggcagtgtg gcgtggtctc cgcgaagact gtgtgtggcc 1860
   cagtgtactg tttcaccccc agcccagtgg tagtgggcac gaccgacagg cttggagcgc 1920
   ccacttacac gtggggggag aatgagacag atgtcttcct attgaacagc actcgaccac 1980
   cgctggggtc atggttcggc tgcacgtgga tgaactcttc tggctacacc aagacttgcg 2040
   gcgcaccacc ctgccgtact agagctgact tcaacgccag cacggacctg ttgtgcccca 2100
   cggactgttt taggaagcat cctgatacca cttacctcaa atgcggctct gggccctggc 2160
   tcacgccaag gtgcctgatc gactacccct acaggctctg gcattacccc tgcacagtta 2220
   actataccat cttcaaaata aggatgtatg tgggaggggt tgagcacagg ctcacggctg 2280
   catgcaattt cactcgtggg gatcgttgca acttggagga cagagacaga agtcaactgt 2340
   ctcctttgtt gcactccacc acggaatggg ccattttacc ttgctcttac tcggacctgc 2400
   ccgccttgtc gactggtctt ctccacctcc accaaaacat cgtggacgta caattcatgt 2460
   atggcctatc acctgccctc acaaaataca tcgtccgatg ggagtgggta atactcttat 2520
   tcctgctctt agcggacgcc agggtttgcg cctgcttatg gatgctcatc ttgttgggcc 2580
   aggccgaagc agctttggag aacctcgtaa tactcaatgc agcatccctg gccgggacgc 2640
   acggtcttgt gtccttcctc gtgttcttct gctttgcgtg gtatctgaag ggtaggtggg 2700
   tgcccggagc ggtctacgcc ctctacggga tgtggcctct cctcctgctc ctgctggcgt 2760
   tgcctcagcg ggcatatgca ctggacacgg aggtggccgc gtcgtgtggc ggcgttgttc 2820
   ttgtcgggtt aatggcgctg actctgtcgc catattacaa gcgctatatc agctggtgca 2880
   tgtggtggct tcagtatttt ctgaccagag tagaagcgca actgcacgtg tgggttcccc 2940
   ccctcaacgt ccgggggggg cgcgatgccg tcatcttact catgtgtgta gtacacccga 3000
   ccctggtatt tgacatcacc aaactactcc tggccatctt cggacccctt tggattcttc 3060
   aagccagttt gcttaaagtc ccctacttcg tgcgcgttca aggccttctc cggatctgcg 3120
   cgctagcgcg gaagatagcc ggaggtcatt acgtgcaaat ggccatcatc aagttagggg 3180
   cgcttactgg cacctatgtg tataaccatc tcacccctct tcgagactgg gcgcacaacg 3240
   gcctgcgaga tctggccgtg gctgtggaac cagtcgtctt ctcccgaatg gagaccaagc 3300
   tcatcacgtg gggggcagat accgccgcgt gcggtgacat catcaacggc ttgcccgtct 3360
   ctgcccgtag ggccaggagg atactgcttg ggccagccga cggaatggtc tccaaggggt 3420
   ggaggttgct ggcgcccatc acggcgtacg cccagcagac gagaggcctc ctagggtgta 3480
   taatcaccag cctgactggc cgggacaaaa accaagtgga gggtgaggtc cagatcgtgt 3540
   caactgctac ccaaaccttc ctggcaacgt gcatcaatgg ggtatgctgg actgtctacc 3600
   acggggccgg aacgaggacc atcgcatcac ccaagggtcc tgtcatccag atgtatacca 3660
   atgtggacca agaccttgtg ggctggcccg ctcctcaagg ttcccgctca ttgacaccct 3720
   gtacctgcgg ctcctcggac ctttacctgg tcacgaggca cgccgatgtc attcccgtgc 3780
   gccggcgagg tgatagcagg ggtagcctgc tttcgccccg gcccatttcc tacttgaaag 3840
   gctcctcggg gggtccgctg ttgtgccccg cgggacacgc cgtgggccta ttcagggccg 3900
   cggtgtgcac ccgtggagtg gctaaagcgg tggactttat ccctgtggag aacctaggga 3960
   caaccatgag atccccggtg ttcacggaca actcctctcc accagcagtg ccccagagct 4020
   tccaggtggc ccacctgcat gctcccaccg gcagcggtaa gagcaccaag gtcccggctg 4080
   cgtacgcagc ccagggctac aaggtgttgg tgctcaaccc ctctgttgct gcaacgctgg 4140
   gctttggtgc ttacatgtcc aaggcccatg gggttgatcc taatatcagg accggggtga 4200
   gaacaattac cactggcagc cccatcacgt actccaccta cggcaagttc cttgccgacg 4260
   gcgggtgctc aggaggtgct tatgacataa taatttgtga cgagtgccac tccacggatg 4320
   ccacatccat cttgggcatc ggcactgtcc ttgaccaagc agagactgcg ggggcgagac 4380
   tggttgtgct cgccactgct acccctccgg gctccgtcac tgtgtcccat cctaacatcg 4440
   aggaggttgc tctgtccacc accggagaga tcccctttta cggcaaggct atccccctcg 4500
   aggtgatcaa ggggggaaga catctcatct tctgccactc aaagaagaag tgcgacgagc 4560
   tcgccgcgaa gctggtcgca ttgggcatca atgccgtggc ctactaccgc ggtcttgacg 4620
   tgtctgtcat cccgaccagc ggcgatgttg tcgtcgtgtc gaccgatgct ctcatgactg 4680
   gctttaccgg cgacttcgac tctgtgatag actgcaacac gtgtgtcact cagacagtcg 4740
   atttcagcct tgaccctacc tttaccattg agacaaccac gctcccccag gatgctgtct 4800
   ccaggactca acgccggggc aggactggca gggggaagcc aggcatctat agatttgtgg 4860
   caccggggga gcgcccctcc ggcatgttcg actcgtccgt cctctgtgag tgctatgacg 4920
   cgggctgtgc ttggtatgag ctcacgcccg ccgagactac agttaggcta cgagcgtaca 4980
   tgaacacccc ggggcttccc gtgtgccagg accatcttga attttgggag ggcgtcttta 5040
   cgggcctcac tcatatagat gcccactttt tatcccagac aaagcagagt ggggagaact 5100
   ttccttacct ggtagcgtac caagccaccg tgtgcgctag ggctcaagcc cctcccccat 5160
   cgtgggacca gatgtggaag tgtttgatcc gccttaaacc caccctccat gggccaacac 5220
   ccctgctata cagactgggc gctgttcaga atgaagtcac cctgacgcac ccaatcacca 5280
   aatacatcat gacatgcatg tcggccgacc tggaggtcgt cacgagcacc tgggtgctcg 5340
   ttggcggcgt cctggctgct ctggccgcgt attgcctgtc aacaggctgc gtggtcatag 5400
   tgggcaggat cgtcttgtcc gggaagccgg caattatacc tgacagggag gttctctacc 5460
   aggagttcga tgagatggaa gagtgctctc agcacttacc gtacatcgag caagggatga 5520
   tgctcgctga gcagttcaag cagaaggccc tcggcctcct gcagaccgcg tcccgccatg 5580
   cagaggttat cacccctgct gtccagacca actggcagaa actcgaggtc ttttgggcga 5640
   agcacatgtg gaatttcatc agtgggatac aatacttggc gggcctgtca acgctgcctg 5700
   gtaaccccgc cattgcttca ttgatggctt ttacagctgc cgtcaccagc ccactaacca 5760
   ctggccaaac cctcctcttc aacatattgg gggggtgggt ggctgcccag ctcgccgccc 5820
   ccggtgccgc tactgccttt gtgggtgctg gcctagctgg cgccgccatc ggcagcgttg 5880
   gactggggaa ggtcctcgtg gacattcttg cagggtatgg cgcgggcgtg gcgggagctc 5940
   ttgtagcatt caagatcatg agcggtgagg tcccctccac ggaggacctg gtcaatctgc 6000
   tgcccgccat cctctcgcct ggagcccttg tagtcggtgt ggtctgcgca gcaatactgc 6060
   gccggcacgt tggcccgggc gagggggcag tgcaatggat gaaccggcta atagccttcg 6120
   cctcccgggg gaaccatgtt tcccccacgc actacgtgcc ggagagcgat gcagccgccc 6180
   gcgtcactgc catactcagc agcctcactg taacccagct cctgaggcga ctgcatcagt 6240
   ggataagctc ggagtgtacc actccatgct ccggttcctg gctaagggac atctgggact 6300
   ggatatgcga ggtgctgagc gactttaaga cctggctgaa agccaagctc atgccacaac 6360
   tgcctgggat tccctttgtg tcctgccagc gcgggtatag gggggtctgg cgaggagacg 6420
   gcattatgca cactcgctgc cactgtggag ctgagatcac tggacatgtc aaaaacggga 6480
   cgatgaggat cgtcggtcct aggacctgca ggaacatgtg gagtgggacg ttccccatta 6540
   acgcctacac cacgggcccc tgtactcccc ttcctgcgcc gaactataag ttcgcgctgt 6600
   ggagggtgtc tgcagaggaa tacgtggaga taaggcgggt gggggacttc cactacgtat 6660
   cgggtatgac tactgacaat cttaaatgcc cgtgccagat cccatcgccc gaatttttca 6720
   cagaattgga cggggtgcgc ctacacaggt ttgcgccccc ttgcaagccc ttgctgcggg 6780
   aggaggtatc attcagagta ggactccacg agtacccggt ggggtcgcaa ttaccttgcg 6840
   agcccgaacc ggacgtagcc gtgttgacgt ccatgctcac tgatccctcc catataacag 6900
   cagaggcggc cgggagaagg ttggcgagag ggtcaccccc ttctatggcc agctcctcgg 6960
   ctagccagct gtccgctcca tctctcaagg caacttgcac cgccaaccat gactcccctg 7020
   acgccgagct catagaggct aacctcctgt ggaggcagga gatgggcggc aacatcacca 7080
   gggttgagtc agagaacaaa gtggtgattc tggactcctt cgatccgctt gtggcagagg 7140
   aggatgagcg ggaggtctcc gtacctgcag aaattctgcg gaagtctcgg agattcgccc 7200
   gggccctgcc cgtctgggcg cggccggact acaacccccc gctagtagag acgtggaaaa 7260
   agcctgacta cgaaccacct gtggtccatg gctgcccgct accacctcca cggtcccctc 7320
   ctgtgcctcc gcctcggaaa aagcgtacgg tggtcctcac cgaatcaacc ctatctactg 7380
   ccttggccga gcttgccacc aaaagttttg gcagctcctc aacttccggc attacgggcg 7440
   acaatacgac aacatcctct gagcccgccc cttctggctg cccccccgac tccgacgttg 7500
   agtcctattc ttccatgccc cccctggagg gggagcctgg ggatccggat ctcagcgacg 7560
   ggtcatggtc gacggtcagt agtggggccg acacggaaga tgtcgtgtgc tgctcaatgt 7620
   cttattcctg gacaggcgca ctcgtcaccc cgtgcgctgc ggaagaacaa aaactgccca 7680
   tcaacgcact gagcaactcg ttgctacgcc atcacaatct ggtgtattcc accacttcac 7740
   gcagtgcttg ccaaaggcag aagaaagtca catttgacag actgcaagtt ctggacagcc 7800
   attaccagga cgtgctcaag gaggtcaaag cagcggcgtc aaaagtgaag gctaacttgc 7860
   tatccgtaga ggaagcttgc agcctgacgc ccccacattc agccaaatcc aagtttggct 7920
   atggggcaaa agacgtccgt tgccatgcca gaaaggccgt agcccacatc aactccgtgt 7980
   ggaaagacct tctggaagac agtgtaacac caatagacac taccatcatg gccaagaacg 8040
   aggttttctg cgttcagcct gagaaggggg gtcgtaagcc agctcgtctc atcgtgttcc 8100
   ccgacctggg cgtgcgcgtg tgcgagaaga tggccctgta cgacgtggtt agcaagctcc 8160
   ccctggccgt gatgggaagc tcctacggat tccaatactc accaggacag cgggttgaat 8220
   tcctcgtgca agcgtggaag tccaagaaga ccccgatggg gttctcgtat gatacccgct 8280
   gttttgactc cacagtcact gagagcgaca tccgtacgga ggaggcaatt taccaatgtt 8340
   gtgacctgga cccccaagcc cgcgtggcca tcaagtccct cactgagagg ctttatgttg 8400
   ggggccctct taccaattca aggggggaaa actgcggcta ccgcaggtgc cgcgcgagcg 8460
   gcgtactgac aactagctgt ggtaacaccc tcacttgcta catcaaggcc cgggcagcct 8520
   gtcgagccgc agggctccag gactgcacca tgctcgtgtg tggcgacgac ttagtcgtta 8580
   tctgtgaaag tgcgggggtc caggaggacg cggcgagcct gagagccttc acggaggcta 8640
   tgaccaggta ctccgccccc cccggggacc ccccacaacc agaatacgac ttggagctta 8700
   taacatcatg ctcctccaac gtgtcagtcg cccacgacgg cgctggaaag agggtctact 8760
   accttacccg tgaccctaca acccccctcg cgagagccgc gtgggagaca gcaagacaca 8820
   ctccagtcaa ttcctggcta ggcaacataa tcatgtttgc ccccacactg tgggcgagga 8880
   tgatactgat gacccatttc tttagcgtcc tcatagccag ggatcagctt gaacaggctc 8940
   ttaactgtga gatctacgga gcctgctact ccatagaacc actggatcta cctccaatca 9000
   ttcaaagact ccatggcctc agcgcatttt cactccacag ttactctcca ggtgaaatca 9060
   atagggtggc cgcatgcctc agaaaacttg gggtcccgcc cttgcgagct tggagacacc 9120
   gggcccggag cgtccgcgct aggcttctgt ccagaggagg cagggctgct atatgtggca 9180
   agtacctctt caactgggca gtaagaacaa agctcaaact cactccaata gcggccgctg 9240
   gccggctgga cttgtccggt tggttcacgg ctggctacag cgggggagac atttatcaca 9300
   gcgtgtctca tgcccggccc cgctggttct ggttttgcct actcctgctc gctgcagggg 9360
   taggcatcta cctcctcccc aaccgatgaa ggttggggta aacactccgg cctcttaagc 9420
   catttcctgt tttttttttt tttttttttt tttttttctt tttttttttc tttcctttcc 9480
   ttcttttttt cctttctttt tcccttcttt aatggtggct ccatcttagc cctagtcacg 9540
   gctagctgtg aaaggtccgt gagccgcatg actgcagaga gtgctgatac tggcctctct 9600
   gcagatcatg t 9611
<210> 10
   <211> 3015
   <212> PRT
   <213> Hepatitis C virus
<400> 10
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Asn Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
   195 200 205
   Asn Asp Ser Ile Thr Trp Gln Leu Gln Ala Ala Val Leu His Val Pro
   210 215 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Ile Leu Leu Phe Leu Leu Leu Ala Asp Ala Arg Val Cys
   725 730 735
   Ala Cys Leu Trp Met Leu Ile Leu Leu Gly Gln Ala Glu Ala Ala Leu
   740 745 750
   Glu Asn Leu Val Ile Leu Asn Ala Ala Ser Leu Ala Gly Thr His Gly
   755 760 765
   Leu Val Ser Phe Leu Val Phe Phe Cys Phe Ala Trp Tyr Leu Lys Gly
   770 775 780
   Arg Trp Val Pro Gly Ala Val Tyr Ala Leu Tyr Gly Met Trp Pro Leu
   785 790 795 800
   Leu Leu Leu Leu Leu Ala Leu Pro Gln Arg Ala Tyr Ala Leu Asp Thr
   805 810 815
   Glu Val Ala Ala Ser Cys Gly Gly Val Val Leu Val Gly Leu Met Ala
   820 825 830
   Leu Thr Leu Ser Pro Tyr Tyr Lys Arg Tyr Ile Ser Trp Cys Met Trp
   835 840 845
   Trp Leu Gln Tyr Phe Leu Thr Arg Val Glu Ala Gln Leu His Val Trp
   850 855 860
   Val Pro Pro Leu Asn Val Arg Gly Gly Arg Asp Ala Val Ile Leu Leu
   865 870 875 880
   Met Cys Val Val His Pro Thr Leu Val Phe Asp Ile Thr Lys Leu Leu
   885 890 895
   Leu Ala Ile Phe Gly Pro Leu Trp Ile Leu Gln Ala Ser Leu Leu Lys
   900 905 910
   Val Pro Tyr Phe Val Arg Val Gln Gly Leu Leu Arg Ile Cys Ala Leu
   915 920 925
   Ala Arg Lys Ile Ala Gly Gly His Tyr Val Gln Met Ala Ile Ile Lys
   930 935 940
   Leu Gly Ala Leu Thr Gly Thr Tyr Val Tyr Asn His Leu Thr Pro Leu
   945 950 955 960
   Arg Asp Trp Ala His Asn Gly Leu Arg Asp Leu Ala Val Ala Val Glu
   965 970 975
   Pro Val Val Phe Ser Arg Met Glu Thr Lys Leu Ile Thr Trp Gly Ala
   980 985 990
   Asp Thr Ala Ala Cys Gly Asp Ile Ile Asn Gly Leu Pro Val Ser Ala
   995 1000 1005
   Arg Arg Gly Gln Glu Ile Leu Leu Gly Pro Ala Asp Gly Met Val Ser
   1010 1015 1020
   Lys Gly Trp Arg Leu Leu Ala Pro Ile Thr Ala Tyr Ala Gln Gln Thr
   1025 1030 1035 1040
   Arg Gly Leu Leu Gly Cys Ile Ile Thr Ser Leu Thr Gly Arg Asp Lys
   1045 1050 1055
   Asn Gln Val Glu Gly Glu Val Gln Ile Val Ser Thr Ala Thr Gln Thr
   1060 1065 1070
   Phe Leu Ala Thr Cys Ile Asn Gly Val Cys Trp Thr Val Tyr His Gly
   1075 1080 1085
   Ala Gly Thr Arg Thr Ile Ala Ser Pro Lys Gly Pro Val Ile Gln Met
   1090 1095 1100
   Tyr Thr Asn Val Asp Gln Asp Leu Val Gly Trp Pro Ala Pro Gln Gly
   1105 1110 1115 1120
   Ser Arg Ser Leu Thr Pro Cys Thr Cys Gly Ser Ser Asp Leu Tyr Leu
   1125 1130 1135
   Val Thr Arg His Ala Asp Val Ile Pro Val Arg Arg Arg Gly Asp Ser
   1140 1145 1150
   Arg Gly Ser Leu Leu Ser Pro Arg Pro Ile Ser Tyr Leu Lys Gly Ser
   1155 1160 1165
   Ser Gly Gly Pro Leu Leu Cys Pro Ala Gly His Ala Val Gly Leu Phe
   1170 1175 1180
   Arg Ala Ala Val Cys Thr Arg Gly Val Ala Lys Ala Val Asp Phe Ile
   1185 1190 1195 1200
   Pro Val Glu Asn Leu Gly Thr Thr Met Arg Ser Pro Val Phe Thr Asp
   1205 1210 1215
   Asn Ser Ser Pro Pro Ala Val Pro Gln Ser Phe Gln Val Ala His Leu
   1220 1225 1230
   His Ala Pro Thr Gly Ser Gly Lys Ser Thr Lys Val Pro Ala Ala Tyr
   1235 1240 1245
   Ala Ala Gln Gly Tyr Lys Val Leu Val Leu Asn Pro Ser Val Ala Ala
   1250 1255 1260
   Thr Leu Gly Phe Gly Ala Tyr Met Ser Lys Ala His Gly Val Asp Pro
   1265 1270 1275 1280
   Asn Ile Arg Thr Gly Val Arg Thr Ile Thr Thr Gly Ser Pro Ile Thr
   1285 1290 1295
   Tyr Ser Thr Tyr Gly Lys Phe Leu Ala Asp Gly Gly Cys Ser Gly Gly
   1300 1305 1310
   Ala Tyr Asp Ile Ile Ile Cys Asp Glu Cys His Ser Thr Asp Ala Thr
   1315 1320 1325
   Ser Ile Leu Gly Ile Gly Thr Val Leu Asp Gln Ala Glu Thr Ala Gly
   1330 1335 1340
   Ala Arg Leu Val Val Leu Ala Thr Ala Thr Pro Pro Gly Ser Val Thr
   1345 1350 1355 1360
   Val Ser His Pro Asn Ile Glu Glu Val Ala Leu Ser Thr Thr Gly Glu
   1365 1370 1375
   Ile Pro Phe Tyr Gly Lys Ala Ile Pro Leu Glu Val Ile Lys Gly Gly
   1380 1385 1390
   Arg His Leu Ile Phe Cys His Ser Lys Lys Lys Cys Asp Glu Leu Ala
   1395 1400 1405
   Ala Lys Leu Val Ala Leu Gly Ile Asn Ala Val Ala Tyr Tyr Arg Gly
   1410 1415 1420
   Leu Asp Val Ser Val Ile Pro Thr Ser Gly Asp Val Val Val Val Ser
   1425 1430 1435 1440
   Thr Asp Ala Leu Met Thr Gly Phe Thr Gly Asp Phe Asp Ser Val Ile
   1445 1450 1455
   Asp Cys Asn Thr Cys Val Thr Gln Thr Val Asp Phe Ser Leu Asp Pro
   1460 1465 1470
   Thr Phe Thr Ile Glu Thr Thr Thr Leu Pro Gln Asp Ala Val Ser Arg
   1475 1480 1485
   Thr Gln Arg Arg Gly Arg Thr Gly Arg Gly Lys Pro Gly Ile Tyr Arg
   1490 1495 1500
   Phe Val Ala Pro Gly Glu Arg Pro Ser Gly Met Phe Asp Ser Ser Val
   1505 1510 1515 1520
   Leu Cys Glu Cys Tyr Asp Ala Gly Cys Ala Trp Tyr Glu Leu Thr Pro
   1525 1530 1535
   Ala Glu Thr Thr Val Arg Leu Arg Ala Tyr Met Asn Thr Pro Gly Leu
   1540 1545 1550
   Pro Val Cys Gln Asp His Leu Glu Phe Trp Glu Gly Val Phe Thr Gly
   1555 1560 1565
   Leu Thr His Ile Asp Ala His Phe Leu Ser Gln Thr Lys Gln Ser Gly
   1570 1575 1580
   Glu Asn Phe Pro Tyr Leu Val Ala Tyr Gln Ala Thr Val Cys Ala Arg
   1585 1590 1595 1600
   Ala Gln Ala Pro Pro Pro Ser Trp Asp Gln Met Trp Lys Cys Leu Ile
   1605 1610 1615
   Arg Leu Lys Pro Thr Leu His Gly Pro Thr Pro Leu Leu Tyr Arg Leu
   1620 1625 1630
   Gly Ala Val Gln Asn Glu Val Thr Leu Thr His Pro Ile Thr Lys Tyr
   1635 1640 1645
   Ile Met Thr Cys Met Ser Ala Asp Leu Glu Val Val Thr Ser Thr Trp
   1650 1655 1660
   Val Leu Val Gly Gly Val Leu Ala Ala Leu Ala Ala Tyr Cys Leu Ser
   1665 1670 1675 1680
   Thr Gly Cys Val Val Ile Val Gly Arg Ile Val Leu Ser Gly Lys Pro
   1685 1690 1695
   Ala Ile Ile Pro Asp Arg Glu Val Leu Tyr Gln Glu Phe Asp Glu Met
   1700 1705 1710
   Glu Glu Cys Ser Gln His Leu Pro Tyr Ile Glu Gln Gly Met Met Leu
   1715 1720 1725
   Ala Glu Gln Phe Lys Gln Lys Ala Leu Gly Leu Leu Gln Thr Ala Ser
   1730 1735 1740
   Arg His Ala Glu Val Ile Thr Pro Ala Val Gln Thr Asn Trp Gln Lys
   1745 1750 1755 1760
   Leu Glu Val Phe Trp Ala Lys His Met Trp Asn Phe Ile Ser Gly Ile
   1765 1770 1775
   Gln Tyr Leu Ala Gly Leu Ser Thr Leu Pro Gly Asn Pro Ala Ile Ala
   1780 1785 1790
   Ser Leu Met Ala Phe Thr Ala Ala Val Thr Ser Pro Leu Thr Thr Gly
   1795 1800 1805
   Gln Thr Leu Leu Phe Asn Ile Leu Gly Gly Trp Val Ala Ala Gln Leu
   1810 1815 1820
   Ala Ala Pro Gly Ala Ala Thr Ala Phe Val Gly Ala Gly Leu Ala Gly
   1825 1830 1835 1840
   Ala Ala Ile Gly Ser Val Gly Leu Gly Lys Val Leu Val Asp Ile Leu
   1845 1850 1855
   Ala Gly Tyr Gly Ala Gly Val Ala Gly Ala Leu Val Ala Phe Lys Ile
   1860 1865 1870
   Met Ser Gly Glu Val Pro Ser Thr Glu Asp Leu Val Asn Leu Leu Pro
   1875 1880 1885
   Ala Ile Leu Ser Pro Gly Ala Leu Val Val Gly Val Val Cys Ala Ala
   1890 1895 1900
   Ile Leu Arg Arg His Val Gly Pro Gly Glu Gly Ala Val Gln Trp Met
   1905 1910 1915 1920
   Asn Arg Leu Ile Ala Phe Ala Ser Arg Gly Asn His Val Ser Pro Thr
   1925 1930 1935
   His Tyr Val Pro Glu Ser Asp Ala Ala Ala Arg Val Thr Ala Ile Leu
   1940 1945 1950
   Ser Ser Leu Thr Val Thr Gln Leu Leu Arg Arg Leu His Gln Trp Ile
   1955 1960 1965
   Ser Ser Glu Cys Thr Thr Pro Cys Ser Gly Ser Trp Leu Arg Asp Ile
   1970 1975 1980
   Trp Asp Trp Ile Cys Glu Val Leu Ser Asp Phe Lys Thr Trp Leu Lys
   1985 1990 1995 2000
   Ala Lys Leu Met Pro Gln Leu Pro Gly Ile Pro Phe Val Ser Cys Gln
   2005 2010 2015
   Arg Gly Tyr Arg Gly Val Trp Arg Gly Asp Gly Ile Met His Thr Arg
   2020 2025 2030
   Cys His Cys Gly Ala Glu Ile Thr Gly His Val Lys Asn Gly Thr Met
   2035 2040 2045
   Arg Ile Val Gly Pro Arg Thr Cys Arg Asn Met Trp Ser Gly Thr Phe
   2050 2055 2060
   Pro Ile Asn Ala Tyr Thr Thr Gly Pro Cys Thr Pro Leu Pro Ala Pro
   2065 2070 2075 2080
   Asn Tyr Lys Phe Ala Leu Trp Arg Val Ser Ala Glu Glu Tyr Val Glu
   2085 2090 2095
   Ile Arg Arg Val Gly Asp Phe His Tyr Val Ser Gly Met Thr Thr Asp
   2100 2105 2110
   Asn Leu Lys Cys Pro Cys Gln Ile Pro Ser Pro Glu Phe Phe Thr Glu
   2115 2120 2125
   Leu Asp Gly Val Arg Leu His Arg Phe Ala Pro Pro Cys Lys Pro Leu
   2130 2135 2140
   Leu Arg Glu Glu Val Ser Phe Arg Val Gly Leu His Glu Tyr Pro Val
   2145 2150 2155 2160
   Gly Ser Gln Leu Pro Cys Glu Pro Glu Pro Asp Val Ala Val Leu Thr
   2165 2170 2175
   Ser Met Leu Thr Asp Pro Ser His Ile Thr Ala Glu Ala Ala Gly Arg
   2180 2185 2190
   Arg Leu Ala Arg Gly Ser Pro Pro Ser Met Ala Ser Ser Ser Ala Ser
   2195 2200 2205
   Gln Leu Ser Ala Pro Ser Leu Lys Ala Thr Cys Thr Ala Asn His Asp
   2210 2215 2220
   Ser Pro Asp Ala Glu Leu Ile Glu Ala Asn Leu Leu Trp Arg Gln Glu
   2225 2230 2235 2240
   Met Gly Gly Asn Ile Thr Arg Val Glu Ser Glu Asn Lys Val Val Ile
   2245 2250 2255
   Leu Asp Ser Phe Asp Pro Leu Val Ala Glu Glu Asp Glu Arg Glu Val
   2260 2265 2270
   Ser Val Pro Ala Glu Ile Leu Arg Lys Ser Arg Arg Phe Ala Arg Ala
   2275 2280 2285
   Leu Pro Val Trp Ala Arg Pro Asp Tyr Asn Pro Pro Leu Val Glu Thr
   2290 2295 2300
   Trp Lys Lys Pro Asp Tyr Glu Pro Pro Val Val His Gly Cys Pro Leu
   2305 2310 2315 2320
   Pro Pro Pro Arg Ser Pro Pro Val Pro Pro Pro Arg Lys Lys Arg Thr
   2325 2330 2335
   Val Val Leu Thr Glu Ser Thr Leu Ser Thr Ala Leu Ala Glu Leu Ala
   2340 2345 2350
   Thr Lys Ser Phe Gly Ser Ser Ser Thr Ser Gly Ile Thr Gly Asp Asn
   2355 2360 2365
   Thr Thr Thr Ser Ser Glu Pro Ala Pro Ser Gly Cys Pro Pro Asp Ser
   2370 2375 2380
   Asp Val Glu Ser Tyr Ser Ser Met Pro Pro Leu Glu Gly Glu Pro Gly
   2385 2390 2395 2400
   Asp Pro Asp Leu Ser Asp Gly Ser Trp Ser Thr Val Ser Ser Gly Ala
   2405 2410 2415
   Asp Thr Glu Asp Val Val Cys Cys Ser Met Ser Tyr Ser Trp Thr Gly
   2420 2425 2430
   Ala Leu Val Thr Pro Cys Ala Ala Glu Glu Gln Lys Leu Pro Ile Asn
   2435 2440 2445
   Ala Leu Ser Asn Ser Leu Leu Arg His His Asn Leu Val Tyr Ser Thr
   2450 2455 2460
   Thr Ser Arg Ser Ala Cys Gln Arg Gln Lys Lys Val Thr Phe Asp Arg
   2465 2470 2475 2480
   Leu Gln Val Leu Asp Ser His Tyr Gln Asp Val Leu Lys Glu Val Lys
   2485 2490 2495
   Ala Ala Ala Ser Lys Val Lys Ala Asn Leu Leu Ser Val Glu Glu Ala
   2500 2505 2510
   Cys Ser Leu Thr Pro Pro His Ser Ala Lys Ser Lys Phe Gly Tyr Gly
   2515 2520 2525
   Ala Lys Asp Val Arg Cys His Ala Arg Lys Ala Val Ala His Ile Asn
   2530 2535 2540
   Ser Val Trp Lys Asp Leu Leu Glu Asp Ser Val Thr Pro Ile Asp Thr
   2545 2550 2555 2560
   Thr Ile Met Ala Lys Asn Glu Val Phe Cys Val Gln Pro Glu Lys Gly
   2565 2570 2575
   Gly Arg Lys Pro Ala Arg Leu Ile Val Phe Pro Asp Leu Gly Val Arg
   2580 2585 2590
   Val Cys Glu Lys Met Ala Leu Tyr Asp Val Val Ser Lys Leu Pro Leu
   2595 2600 2605
   Ala Val Met Gly Ser Ser Tyr Gly Phe Gln Tyr Ser Pro Gly Gln Arg
   2610 2615 2620
   Val Glu Phe Leu Val Gln Ala Trp Lys Ser Lys Lys Thr Pro Met Gly
   2625 2630 2635 2640
   Phe Ser Tyr Asp Thr Arg Cys Phe Asp Ser Thr Val Thr Glu Ser Asp
   2645 2650 2655
   Ile Arg Thr Glu Glu Ala Ile Tyr Gln Cys Cys Asp Leu Asp Pro Gln
   2660 2665 2670
   Ala Arg Val Ala Ile Lys Ser Leu Thr Glu Arg Leu Tyr Val Gly Gly
   2675 2680 2685
   Pro Leu Thr Asn Ser Arg Gly Glu Asn Cys Gly Tyr Arg Arg Cys Arg
   2690 2695 2700
   Ala Ser Gly Val Leu Thr Thr Ser Cys Gly Asn Thr Leu Thr Cys Tyr
   2705 2710 2715 2720
   Ile Lys Ala Arg Ala Ala Cys Arg Ala Ala Gly Leu Gln Asp Cys Thr
   2725 2730 2735
   Met Leu Val Cys Gly Asp Asp Leu Val Val Ile Cys Glu Ser Ala Gly
   2740 2745 2750
   Val Gln Glu Asp Ala Ala Ser Leu Arg Ala Phe Thr Glu Ala Met Thr
   2755 2760 2765
   Arg Tyr Ser Ala Pro Pro Gly Asp Pro Pro Gln Pro Glu Tyr Asp Leu
   2770 2775 2780
   Glu Leu Ile Thr Ser Cys Ser Ser Asn Val Ser Val Ala His Asp Gly
   2785 2790 2795 2800
   Ala Gly Lys Arg Val Tyr Tyr Leu Thr Arg Asp Pro Thr Thr Pro Leu
   2805 2810 2815
   Ala Arg Ala Ala Trp Glu Thr Ala Arg His Thr Pro Val Asn Ser Trp
   2820 2825 2830
   Leu Gly Asn Ile Ile Met Phe Ala Pro Thr Leu Trp Ala Arg Met Ile
   2835 2840 2845
   Leu Met Thr His Phe Phe Ser Val Leu Ile Ala Arg Asp Gln Leu Glu
   2850 2855 2860
   Gln Ala Leu Asn Cys Glu Ile Tyr Gly Ala Cys Tyr Ser Ile Glu Pro
   2865 2870 2875 2880
   Leu Asp Leu Pro Pro Ile Ile Gln Arg Leu His Gly Leu Ser Ala Phe
   2885 2890 2895
   Ser Leu His Ser Tyr Ser Pro Gly Glu Ile Asn Arg Val Ala Ala Cys
   2900 2905 2910
   Leu Arg Lys Leu Gly Val Pro Pro Leu Arg Ala Trp Arg His Arg Ala
   2915 2920 2925
   Arg Ser Val Arg Ala Arg Leu Leu Ser Arg Gly Gly Arg Ala Ala Ile
   2930 2935 2940
   Cys Gly Lys Tyr Leu Phe Asn Trp Ala Val Arg Thr Lys Leu Lys Leu
   2945 2950 2955 2960
   Thr Pro Ile Ala Ala Ala Gly Arg Leu Asp Leu Ser Gly Trp Phe Thr
   2965 2970 2975
   Ala Gly Tyr Ser Gly Gly Asp Ile Tyr His Ser Val Ser His Ala Arg
   2980 2985 2990
   Pro Arg Trp Phe Trp Phe Cys Leu Leu Leu Leu Ala Ala Gly Val Gly
   2995 3000 3005
   Ile Tyr Leu Leu Pro Asn Arg
   3010 3015
<210> 11
   <211> 24
   <212> DNA
   <213> Hepatitis C virus
<400> 11
   actggacacg gaggtggccg cgtc 24
<210> 12
   <211> 24
   <212> DNA
   <213> Hepatitis C virus
<400> 12
   ttgttcttgt cgggttaatg gcgc 24
<210> 13
   <211> 24
   <212> DNA
   <213> Hepatitis C virus
<400> 13
   gggtgtacta cacacatgag taag 24
<210> 14
   <211> 22
   <212> DNA
   <213> Hepatitis C virus
<400> 14
   aagcgcccct aacttgatga tg 22
<210> 15
   <211> 40
   <212> DNA
   <213> Hepatitis C virus
<400> 15
   cgtcatcgat acctcagcgg gcatatgcac tggacacgga 40
<210> 16
   <211> 24
   <212> DNA
   <213> Hepatitis C virus
<400> 16
   gtccagtgca tatgcccgct gagg 24
<210> 17
   <211> 32
   <212> DNA
   <213> Hepatitis C virus
<400> 17
   catgcaccag ctgatatagc gcttgtaata tg 32
<210> 18
   <211> 30
   <212> DNA
   <213> Hepatitis C virus
<400> 18
   tccgtagagg aagcttgcag cctgacgccc 30
<210> 19
   <211> 34
   <212> DNA
   <213> Hepatitis C virus
<400> 19
   cagaggaggc agggctgcta tatgtggcaa gtac 34
<210> 20
   <211> 34
   <212> DNA
   <213> Hepatitis C virus
<400> 20
   gtacttgcca catatagcag ccctgcctcc tctg 34
<210> 21
   <211> 43
   <212> DNA
   <213> Hepatitis C virus
<400> 21
   cgtctctaga caggaaatgg cttaagaggc cggagtgttt ace 43
<210> 22
   <211> 65
   <212> DNA
   <213> Hepatitis C virus
<400> 22
   ttatggatgc tcatcttgtt gggccaggcc gaagcagctt tggagaacct cgtaatactc 60
   aatgc 65
<210> 23
   <211> 32
   <212> DNA
   <213> Hepatitis C virus
<400> 23
   aggatttgtg ctcatggtgc acggtctacg ag 32
<210> 24
   <211> 50
   <212> DNA
   <213> Hepatitis C virus
<400> 24
   ttttttttgc ggccgctaat acgactcact atagacccgc ccctaatagg 50
<210> 25
   <211> 31
   <212> DNA
   <213> Hepatitis C virus
<400> 25
   ccgtgcacca tgagcacaaa tcctaaacct c 31
<210> 26
   <211> 26
   <212> DNA
   <213> Hepatitis C virus
<400> 26
   ggatgtaccc catgaggtcg gcaaag 26
<210> 27
   <211> 30
   <212> DNA
   <213> Hepatitis C virus
<400> 27
   gtttgcgcct gcttatggat gctcatcttg 30
<210> 28
   <211> 26
   <212> DNA
   <213> Hepatitis C virus
<400> 28
   gcgtcataag catatgcctg ttgggg 26
<210> 29
   <211> 23
   <212> DNA
   <213> Hepatitis C virus
<400> 29
   ccctcagcac tggagtacat ctg 23
<210> 30
   <211> 39
   <212> DNA
   <213> Hepatitis C virus
<400> 30
   cgtcatgcat acccctaggg cggctctcat tgaagaggg 39
<210> 31
   <211> 30
   <212> DNA
   <213> Hepatitis C virus
<400> 31
   cgtcccctct tcaatgagag ccgctctaga 30
<210> 32
   <211> 28
   <212> DNA
   <213> Hepatitis C virus
<400> 32
   gcggtgaaga ccaagctcaa actcactc 28
<210> 33
   <211> 41
   <212> DNA
   <213> Hepatitis C virus
<400> 33
   aatctagaag gcgcgcttcc ggcaatggag tgagtttgag c 41
<210> 34
   <211> 38
   <212> DNA
   <213> Hepatitis C virus
<400> 34
   cgtctctaga ggataaatcc aggaggcgcg cttccggc 38
<210> 35
   <211> 27
   <212> DNA
   <213> Hepatitis C virus
<400> 35
   tactttttgt aggggtaggc cttttcc 27
<210> 36
   <211> 34
   <212> DNA
   <213> Hepatitis C virus
<400> 36
   cgtctctaga gtgtagctaa tgtgtgccgc tcta 34
<210> 37
   <211> 24
   <212> DNA
   <213> Hepatitis C virus
<400> 37
   ctatggagtg tagctaatgt gtgc 24
<210> 38
   <211> 66
   <212> DNA
   <213> Hepatitis C virus
<400> 38
   cgtctctaga catgatctgc agagagacca gttacggcac tctctgcagt catgcggctc 60
   acggac 66
<210> 39
   <211> 41
   <212> DNA
   <213> Hepatitis C virus
<400> 39
   ctttcacagc tagccgtgac tagggctaag atggagccac c 41

## Description

### Field Of Invention

The present invention relates to molecular approaches to the production of nucleic acid sequence which comprises the genome of infectious hepatitis C virus. In particular, the invention provides a nucleic acid sequence which comprises the genome of an infectious hepatitis C virus of genotype 2a. The invention therefore relates to the use of the nucleic acid sequence and polypeptides encoded by all or part of the sequence in the development of vaccines and diagnostic assays for HCV and in the development of screening assays for the identification of antiviral agents for HCV.

### Background Of Invention

Hepatitis C virus (HCV) has a positive-sense single-strand RNA genome and is a member of the genus Hepacivirus within the *Flaviviridae* family of viruses (Rice, 1996). As for all positive-stranded RNA viruses, the genome of HCV functions as mRNA from which all viral proteins necessary for propagation are translated.

The viral genome of HCV is approximately 9600 nucleotides (nts) in length and consists of a highly conserved 5' untranslated region (UTR), a single long open reading frame (ORF) of approximately 9,000 nts and a complex 3' UTR. The 5' UTR contains an internal ribosomal entry site (Tsukiyama-Kohara et al., 1992; Honda et al., 1996). The 3' UTR consists of a short variable region, a polypyrimidine tract of variable length and, at the 3' end, a highly conserved region of approximately 100 nucleotides (Kolykhalov et al., 1996; Tanaka et al., 1995; Tanaka et al., 1996; Yamada et al., 1996). The last 46 nucleotides of this conserved region were predicted to form a stable stem-loop structure thought to be critical for viral replication (Blight and Rice, 1997; Ito and Lai, 1997; Tsuchihara et al., 1997). The ORF encodes a large polypeptide precursor that is cleaved into at least 10 proteins by host and viral proteinases (Rice, 1996). The predicted envelope proteins contain several conserved N-linked glycosylation sites and cysteine residues (Okamoto et al., 1992a). The NS3 gene encodes a serine protease and an RNA helicase and the NS5B gene encodes an RNA-dependent RNA polymerase.

A remarkable characteristic of HCV is its genetic heterogeneity, which is manifested throughout the genome (Bukh et al., 1995). The most heterogeneous regions of the genome are found in the envelope genes, in particular the hypervariable region 1 (HVR1) at the N-terminus of E2 (Hijikata *et al*., 1991; Weiner *et al.*, 1991). HCV circulates as a quasispecies of closely related genomes in an infected individual. Globally, six major HCV genotypes (genotypes 1-6) and multiple subtypes (a, b, c, etc.) have been identified (Bukh et al., 1993; Simmonds et al., 1993).

The nucleotide and deduced amino acid sequences among isolates within a quasispecies generally differ by < 2%, whereas those between isolates of different genotypes vary by as much as 35%. Genotypes 1, 2 and 3 are found worldwide and constitute more than 90% of the HCV infections in North and South America, Europe, Russia, China, Japan and Australia (Forns and Bukh, 1998). Throughout these regions genotype 1 accounts for the majority of HCV infections but genotypes 2 and 3 each account for 5-15%.

At present, more than 80% of individuals infected with HCV become chronically infected and these chronically infected individuals have a relatively high risk of developing chronic hepatitis, liver cirrhosis and hepatocellular carcinoma (Hoofnagle, 1997). The only effective therapy for chronic hepatitis C, interferon (IFN), alone or in combination with ribavirin, induces a sustained response in less than 50% of treated patients (Davis et al., 1998; McHutchinson et al., 1998). Consequently, HCV is currently the most common cause of end stage liver failure and the reason for about 30% of liver transplants performed in the U.S. (Hoofnagle, 1997). In addition, a number of recent studies suggested that the severity of liver disease and the outcome of therapy may be genotype-dependent (reviewed in Bukh et al., 1997). In particular, these studies suggested that infection with HCV genotype 1b was associated with more severe liver disease (Brechot, 1997) and a poorer response to IFN therapy (Fried and Hoofnagle, 1995). As a result of the inability to develop a universally effective therapy against HCV infection, it is estimated that there are still more than 25,000 new infections yearly in the U.S. (Alter 1997) Moreover, since there is no vaccine for HCV, HCV remains a serious public health problem.

Despite the intense interest in the development of vaccines and therapies for HCV, progress has been hindered by the absence of a useful cell culture system and the lack of any small animal model for laboratory study. For example, while replication of HCV in several cell lines has been reported, such observations have turned out not to be highly reproducible. In addition, the chimpanzee is the only animal model, other than man, for this disease. Consequently, HCV has been studied only by using clinical materials obtained from patients or experimentally infected chimpanzees, an animal model whose availability is very limited.

However, several researchers have recently reported the construction of infectious cDNA clones of HCV, the identification of which would permit a more effective search for susceptible cell lines and facilitate molecular analysis of the viral genes and their function. For example, Yoo et al., and Dash et al., (1997) (1995) reported that RNA transcripts from cDNA clones of HCV-1 (genotype 1a) and HCV-N (genotype 1b), respectively, resulted in viral replication after transfection into human hepatoma cell lines. Unfortunately, the viability of these clones was not tested in vivo and concerns were raised about the infectivity of these cDNA clones in vitro (Fausto, 1997). In addition, both clones did not contain the terminal 98 conserved nucleotides at the very 3' end of the UTR.

Kolykhalov et al., (1997) and Yanagi et al. (1997, 1998) reported the derivation from HCV strains H77 (genotype 1a) and HC-J4 (genotype 1b) of cDNA clones of HCV that are infectious for chimpanzees. However, while these infectious clones will aid in studying HCV replication and pathogenesis and will provide an important tool for development of in vitro replication and propagation systems, it is important to have infectious clones of more than one genotype, given the extensive genetic heterogeneity of HCV and the potential impact of such heterogeneity on the development of effective therapies and vaccines for HCV.

In addition, synthetic chimeric viruses can be used to map the functional regions of viruses with different phenotypes. In flaviviruses and pestiviruses, infectious chimeric viruses have been successfully engineered to express different functional units of related viruses (Bray and Lai, 1991; Pletnev *et al.*, 1992, 1998; Vassilev *et al*., 1997) and in some cases it has been possible to make chimeras between non-related or distantly related viruses. For instance, the IRES element of poliovirus or bovine viral diarrhea virus has been replaced with IRES sequences from HCV (Frolov et al., 1998; Lu and Wimmer, 1996; Zhao *et al.*, 1999). Recently, the construction of an infectious chimera of two closely related HCV subtypes has been reported. The chimera contained the complete ORF of a genotype 1b strain but had the 5' and 3' termini of a genotype 1a strain (Yanagi *et al.*, 1998).

It is important to determine whether chimeras constructed from more divergent HCV strains are infectious because such chimeras could be used to define the functions of viral units and to dissect the immune response.

### Summary Of The Invention

The present invention relates to nucleic acid sequence which comprises the genome of infectious hepatitis C virus and in particular, nucleic acid sequence which comprises the genome of infectious hepatitis C virus of genotype 2a. It is therefore an object of the invention to provide nucleic acid sequence which encodes infectious hepatitis C virus. Such nucleic acid sequence is referred to throughout the application as "infectious nucleic acid sequence".

For the purposes of this application, nucleic acid sequence refers to RNA, DNA, cDNA or any variant thereof capable of directing host organism synthesis of a hepatitis C virus polypeptide. It is understood that nucleic acid sequence encompasses nucleic acid sequences, which due to degeneracy, encode the same polypeptide sequence as the nucleic acid sequences described herein.

Infectious nucleic acid sequence of the 2a strain HC-J6, described herein can be used to produce chimeras with sequences from the genomes of other strains of HCV from different genotypes or subtypes. Nucleic acid sequences which comprise sequences from two or more HCV genotypes or subtypes are designated "chimeric nucleic acid sequences".

The invention further relates to mutations of the infectious nucleic acid sequence of the invention where mutation includes, but is not limited to, point mutations, deletions and insertions. In one embodiment, a gene or fragment thereof can be deleted to determine the effect of the deleted gene or genes on the properties of the encoded virus such as its virulence and its.ability to replicate. Alternatively a mutation may be introduced into the infectious nucleic acid sequences to examine the effect of the mutation on the properties of the virus.

Mutations or deletions can be introduced into the infectious nucleic acid sequence in order to produce an attenuated hepatitis C virus suitable for vaccine development.

The invention further relates to the use of the infectious nucleic acid sequence to produce attenuated viruses via passage in vitro or in vivo of the viruses produced by transfection of a host cell with the infectious nucleic acid sequence.

The present invention also relates to the use of the nucleic acid sequence of the invention or fragments thereof in the production of polypeptides where "nucleic acid sequence of the invention" refers to infectious nucleic acid sequence, mutations of infectious nucleic acid sequence, chimeric nucleic acid sequence and sequences which comprise the genome of attenuated viruses produced from the infectious nucleic acid sequence of the invention. In one embodiment, said polypeptide or polypeptides are fully or partially purified from hepatitis C virus produced by cells transfected with nucleic acid sequence of the invention. In another embodiment, the polypeptide or polypeptides are produced recombinantly from a fragment of the nucleic acid sequences of the invention.

The polypeptides of the invention, especially structural polypeptides, can serve as immunogens in the development of vaccines or as antigens in the development of diagnostic assays for detecting the presence of HCV in biological samples.

The invention therefore also relates to vaccines for use in immunizing mammals especially humans against hepatitis C. In one embodiment, the vaccine comprises one or more polypeptides made from the nucleic acid sequence of the invention or fragment thereof. In a second embodiment, the vaccine comprises a hepatitis C virus produced by transfection of host cells with the nucleic acid sequences of the invention.

The invention also relates to hepatitis C viruses produced by host cells transfected with the nucleic acid sequence of the present invention.

The invention therefore also provides pharmaceutical compositions comprising the nucleic acid sequence of the invention and/or the encoded hepatitis C viruses. The invention further provides pharmaceutical compositions comprising polypeptides encoded by the nucleic acid sequence of the invention or fragments thereof. The pharmaceutical compositions of the invention may be used prophylactically or therapeutically.

The invention also relates to antibodies to the hepatitis C virus of the invention or their encoded polypeptides and to pharmaceutical compositions comprising these antibodies.

The invention also relates to the use of the nucleic acid sequences of the invention to identify cell lines capable of supporting the replication of HCV in vitro.

The invention further relates to the use of the nucleic acid sequences of the invention or their encoded viral enzymes (e.g. NS3 serine protease, NS3 helicase, NS5B RNA polymerase) to develop screening assays to identify antiviral agents for HCV.

### Brief Description Of Figures

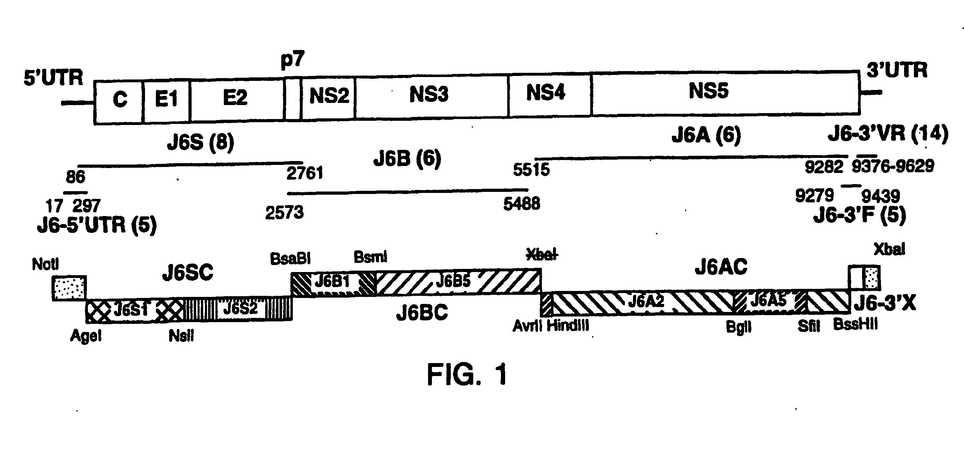

Figure 1 shows the amplification and cloning of hepatitis C virus genotype 2a (strain HC-J6_{Ch)}. The nucleotide positions correspond to the sequence of PJ6CF, a full length cDNA clone of hepatitis C virus, genotype 2a, strain HC-J6_{CH}. Products from polymerase chain reaction are also shown. The names of the clones obtained from these products are indicated (number of clones sequenced are shown in parenthesis). The composition of the full-length cDNA clone is shown at the bottom. The restriction enzymes used for cloning are indicated. An *Xba*I site in HC-J6_{CH} was eliminated by a silent substitution at position 5494.

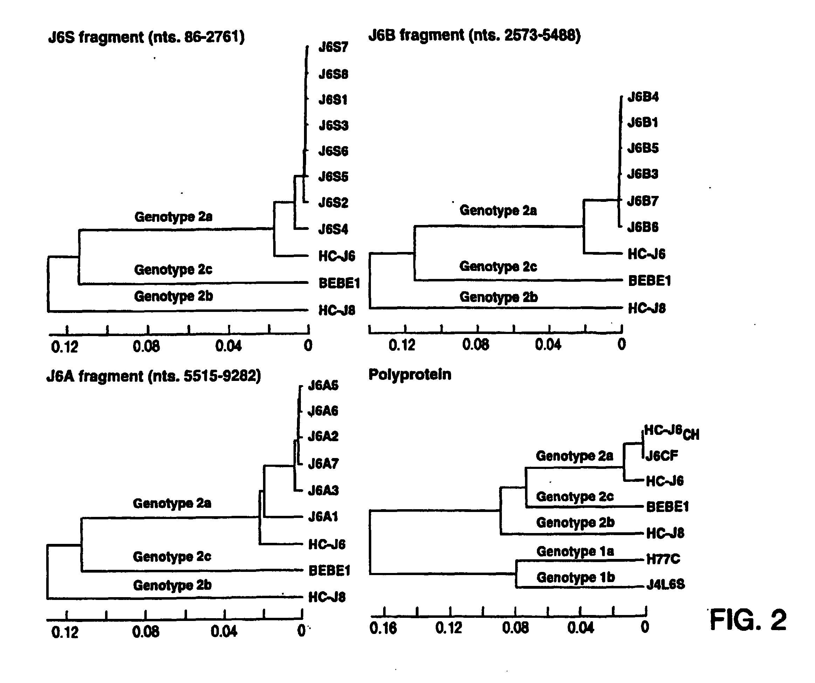

Figure 2 shows tree analysis of clones amplified from an infectious acute phase plasma pool generated in a chimpanzee inoculated with human plasma containing strain HC-J6 (Okamoto *et al*., 1991) as well as a tree of the predicted polyprotein sequence of HC-J6_{CH} and the infectious HC-J6_{CH} cDNA clone (pJ6CF). The nucleotide positions with deletions or insertions were stripped in the analysis of the clones. Multiple sequence alignments and tree analyses were performed with GeneWorks (Oxford Molecular Group) (Bukh *et al.*, 1995). Genotype designations are indicated. Other sequences included in the analysis are HC-J8 (Okamoto *et al*., 1992), genotype 1a infectious clone BEBE1 (Nakao *et al*., 1996), H77C (Yanagi *et al*., 1997); genotype 1b infectious clone J4L6S (Yanagi et al., 1998). The scale in each tree indicates the calculated genetic distance.

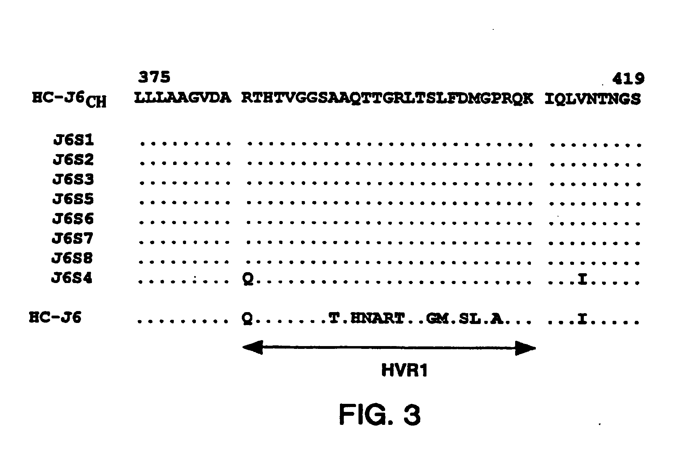

Figure 3 shows the alignment of the hypervariable region 1 sequences from 8 J6S clones of strain HC-J6_{CH}. HC-J6_{CH} represents the consensus amino acid sequence of the infectious plasma pool from an experimentally infected chimpanzee. HC-J6 is the published amino acid sequence of the original inoculum (Okamoto *et al.*, 1991).

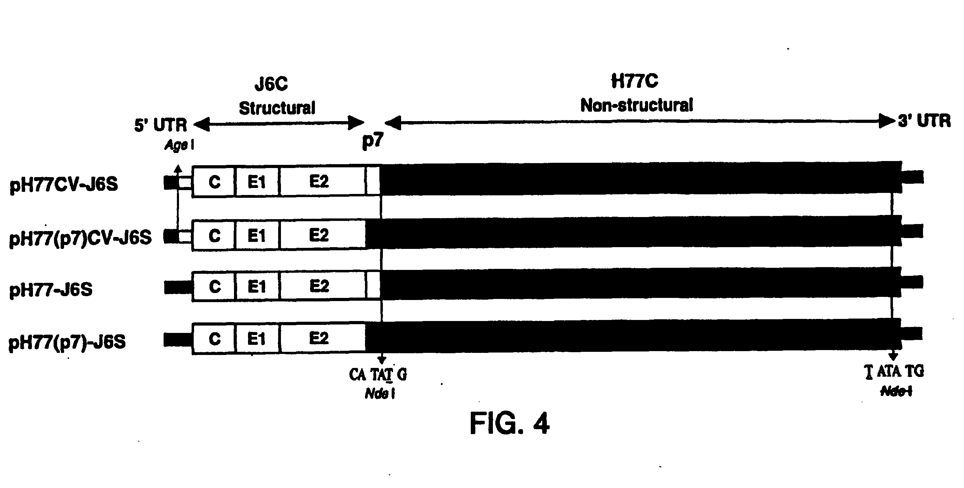

Figure 4 shows the construction of four intertypic chimeric cDNA clones. White boxes are sequences derived from genotype 2a clone pJ6CF, and black boxes are sequences derived from genotype 1a clone pCV-H77C (Yanagi *et al.,* 1997). An *Nde*I site (mutation at position 9158 of pCV-H77C) was eliminated and an artificial *Nde*I site (mutation at position 2765 of pCV-H77C) was created by site-directed mutagenesis; silent mutations are underlined.

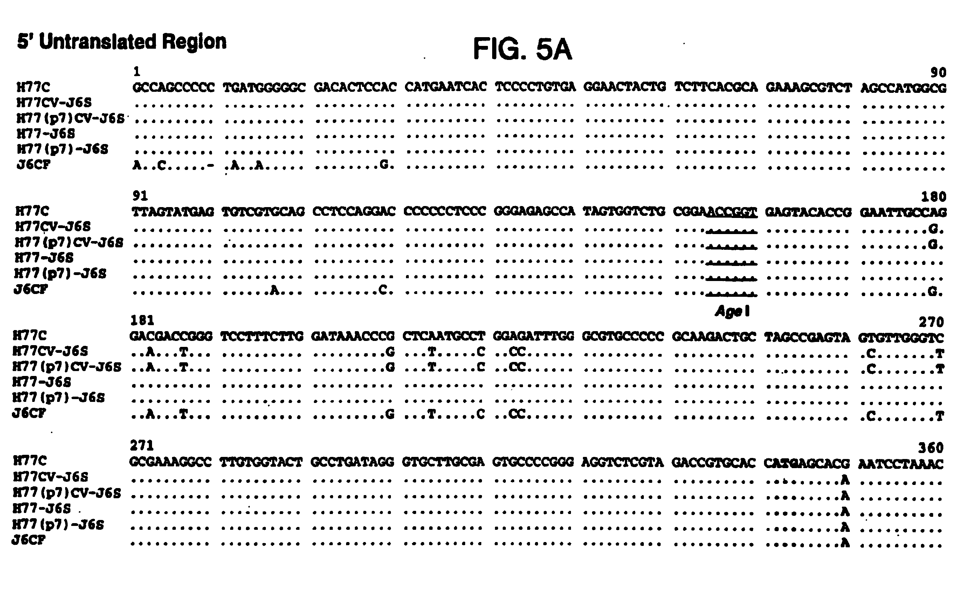

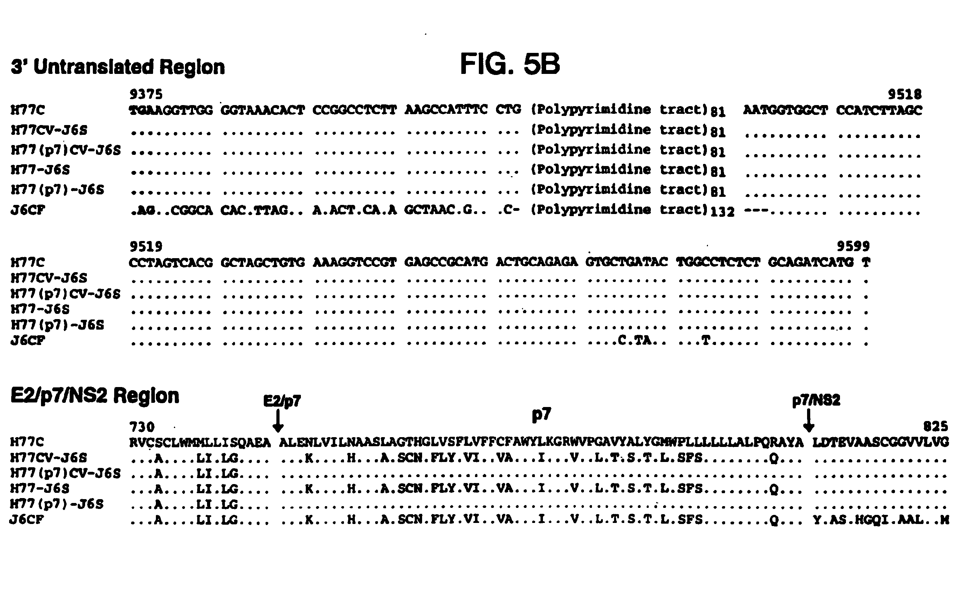

Figures 5A and 5B show the alignment of the nucleotide sequences of the 5' (Fig. 5A) and 3' UTRs (Fig. 5B) and the amino acid sequences of E2/p7/NS2 junctions (Fig. 5B) in the intertypic 1a, 2a chimeric cDNA clones. In the 5' UTR alignment, the first 39 nts of core believed to be important for the IRES function were included (Lemon and Honda, 1997). Top line: the sequence of the infectious genotype la clone pCV-H77C (Yanagi *et al*., 1997). Bottom line: the sequence of the infectious genotype 2a clone pJ6CF. Dot: identity with the sequence of H77C. Capital letter: different from the sequence of H77C. Dash: deletion. Bold face: initiation or stop codon of the ORF. Underlined: *Age*I cleavage site. Arrow: putative sites in the HCV polyprotein cleaved by host signal peptidases. Numbering corresponds to the sequence of pCV-H77C.

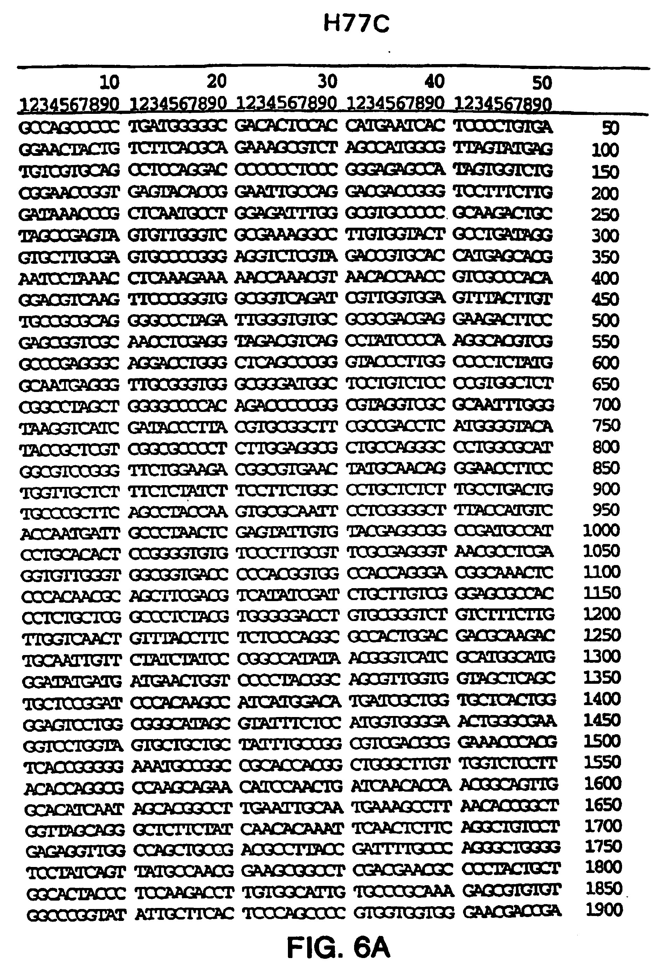

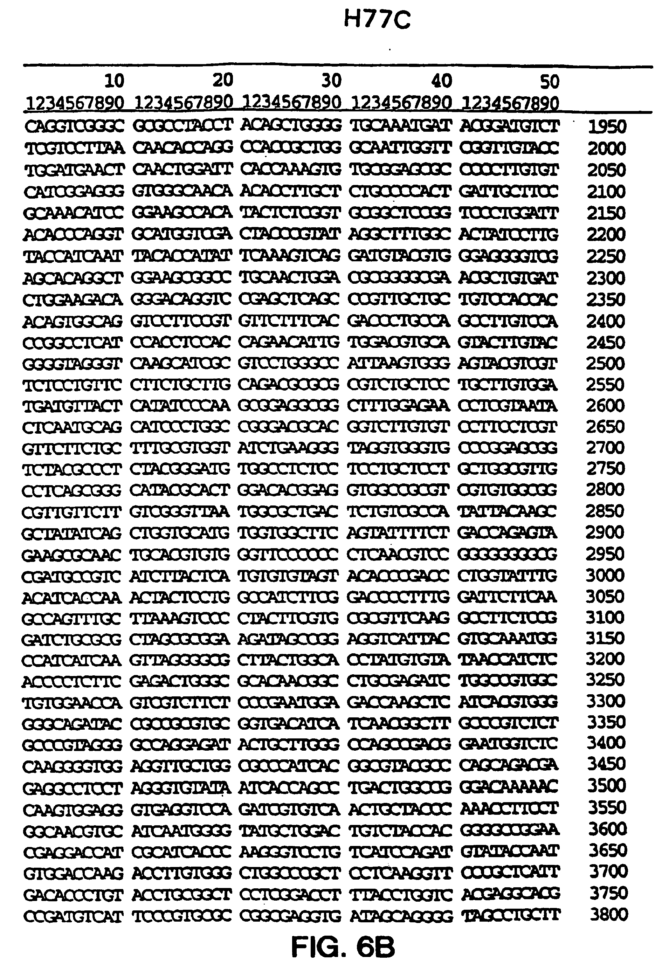

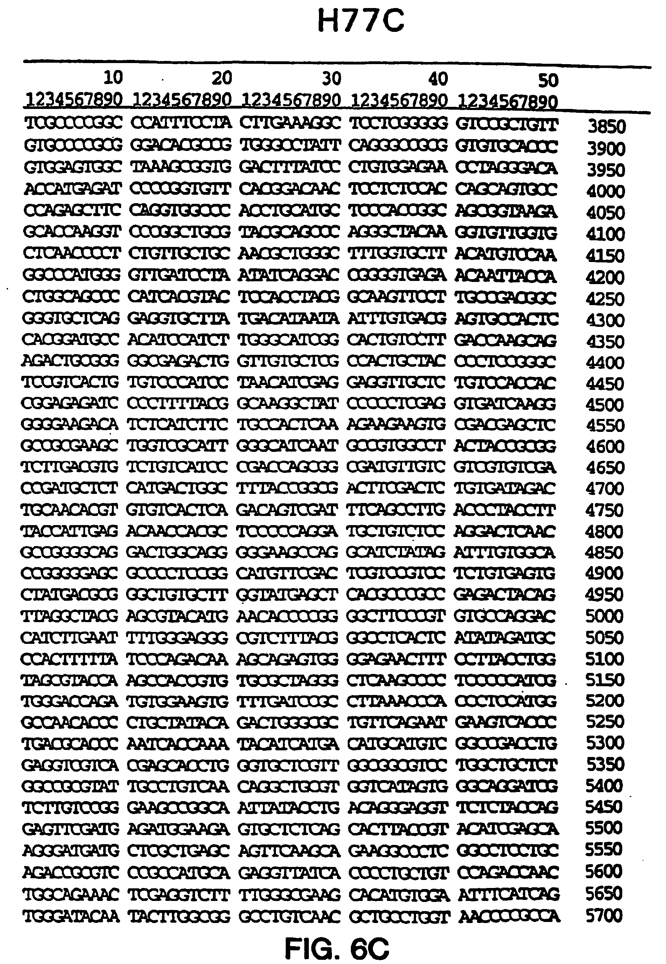

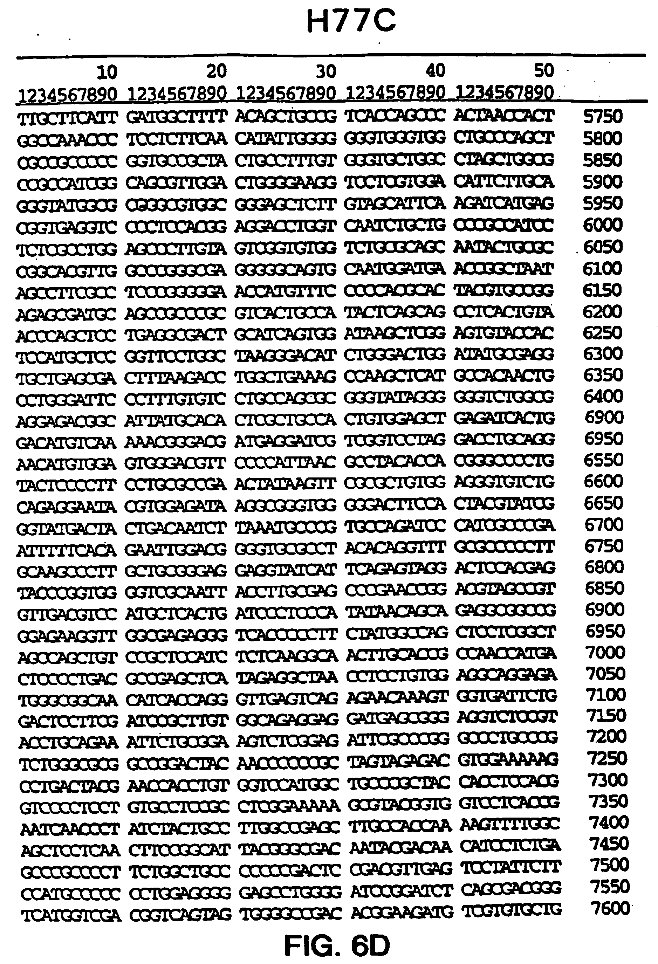

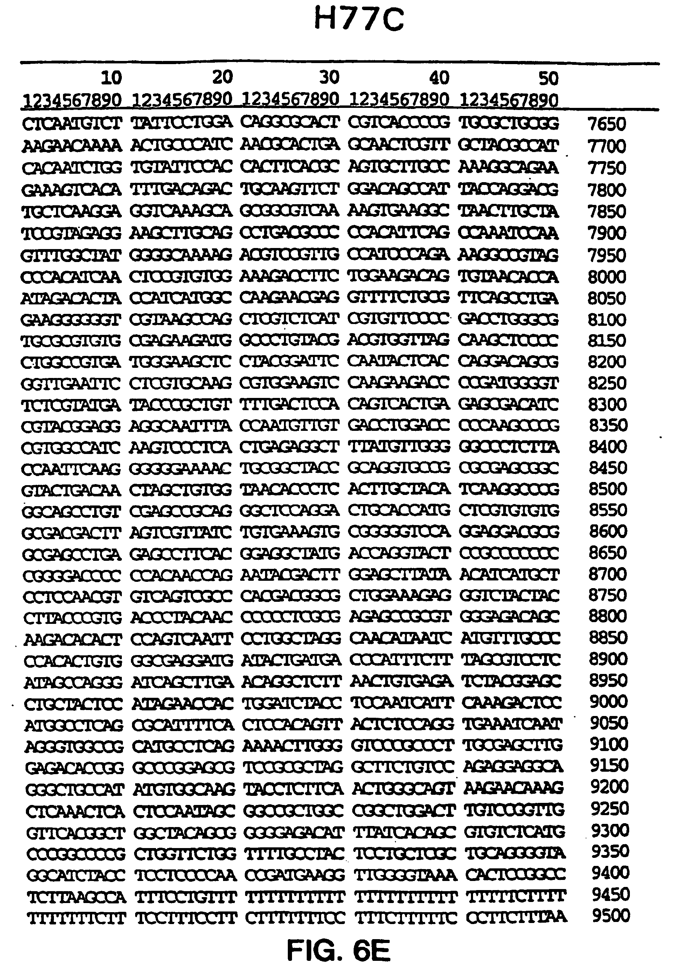

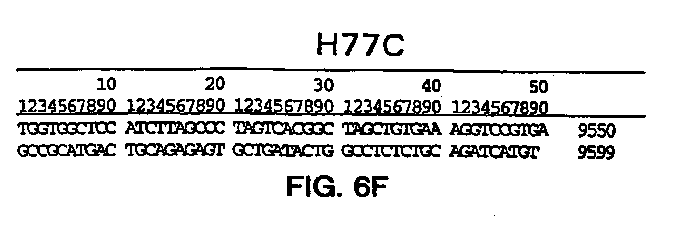

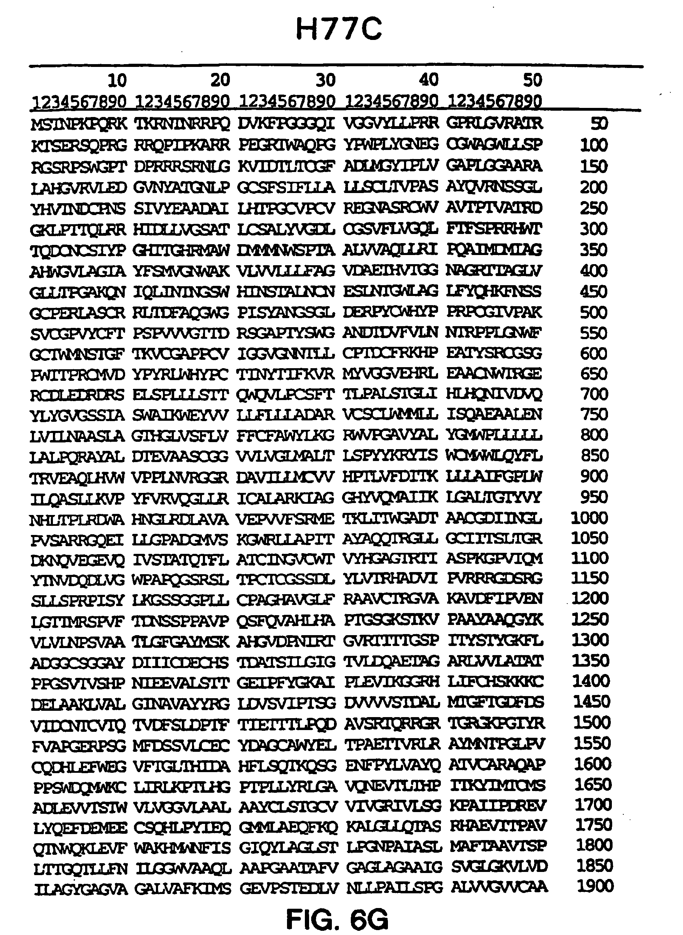

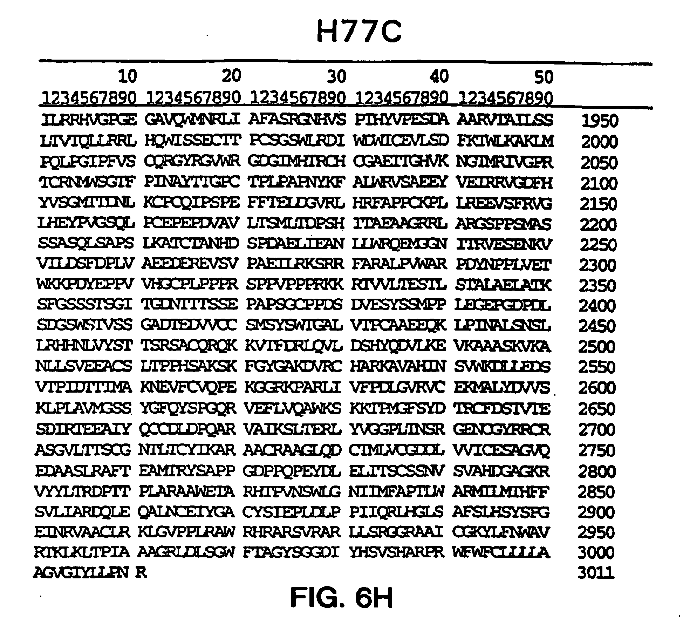

Figures 6A-6F show the nucleotide sequence of the infectious hepatitis C virus clone of genotype la strain H77C and Figures 6G-6H show the amino acid sequence encoded by the clone.

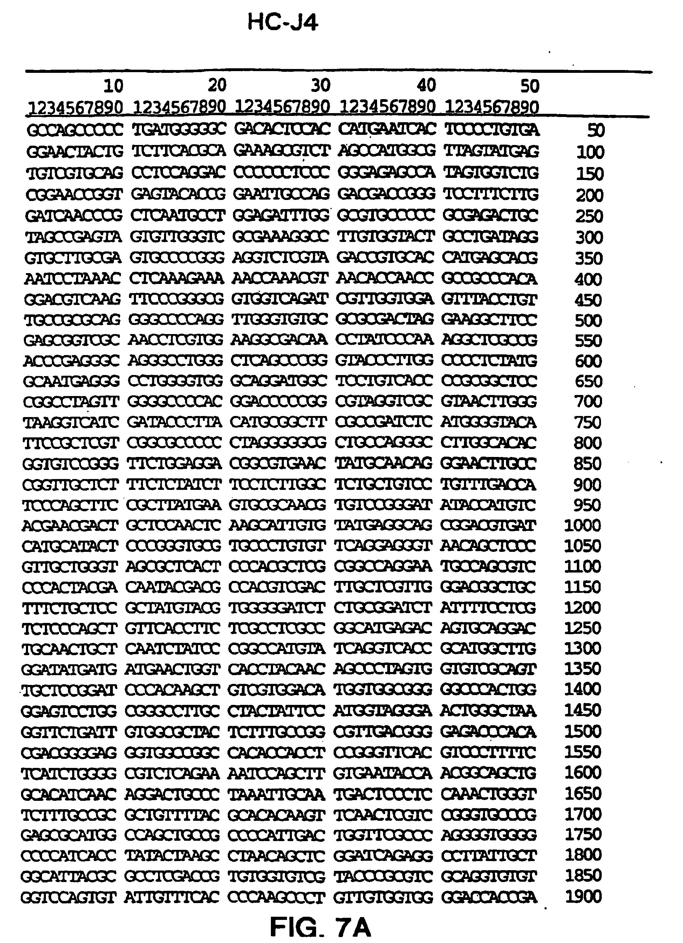

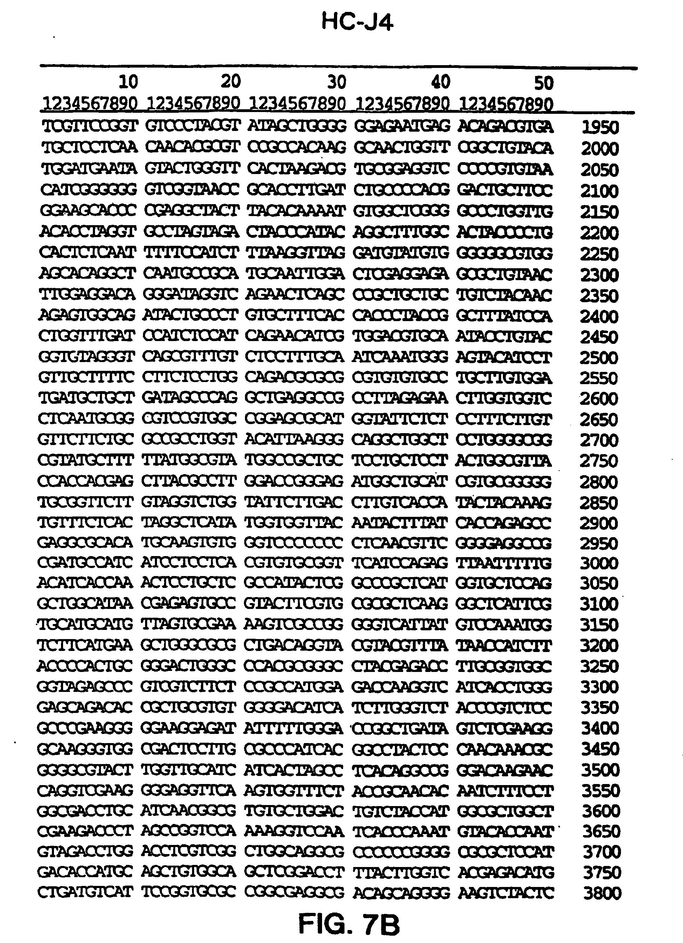

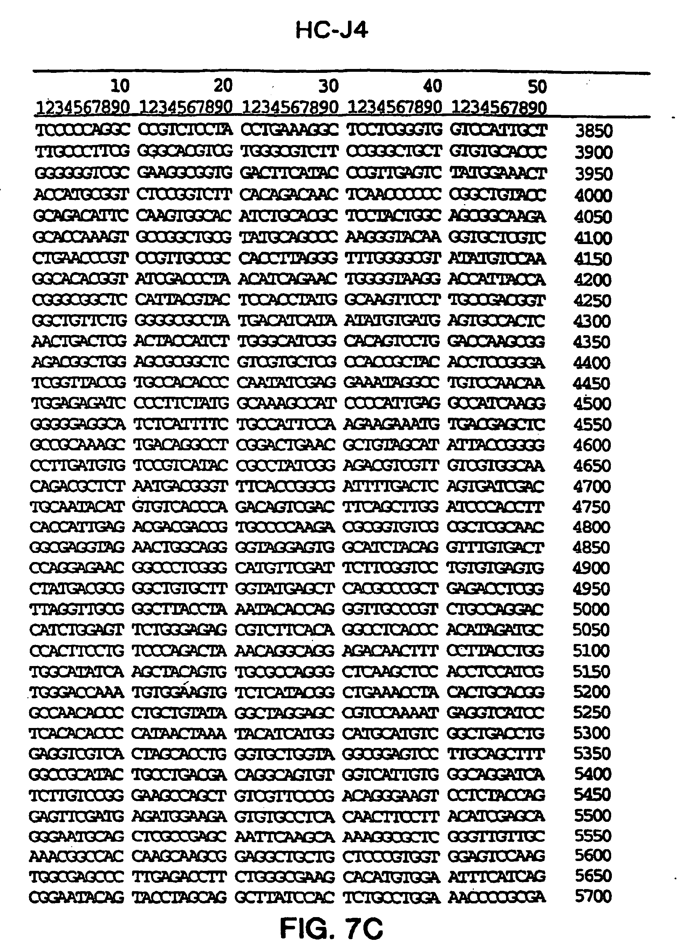

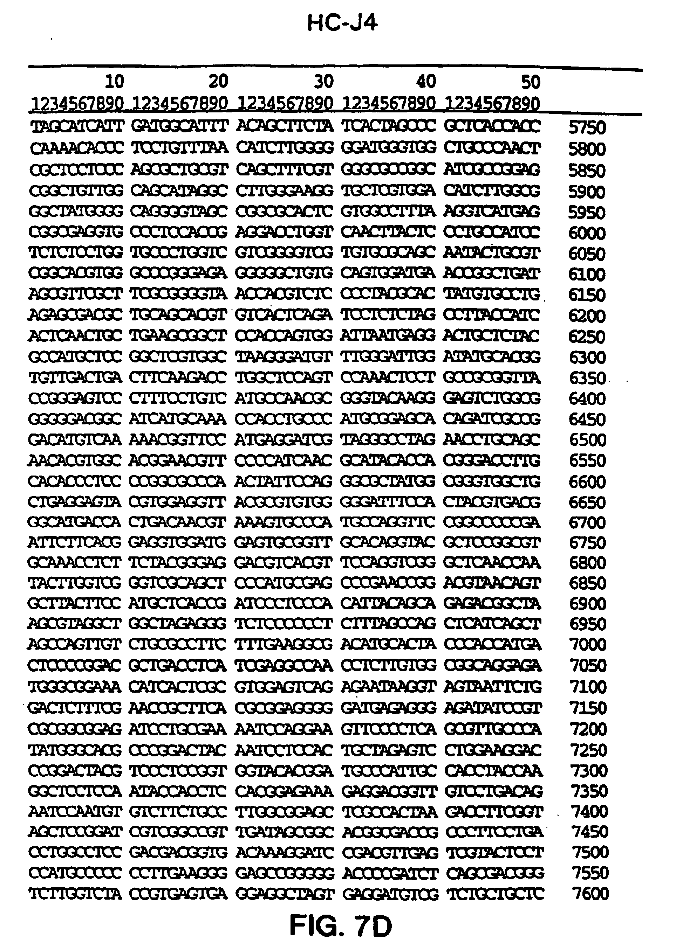

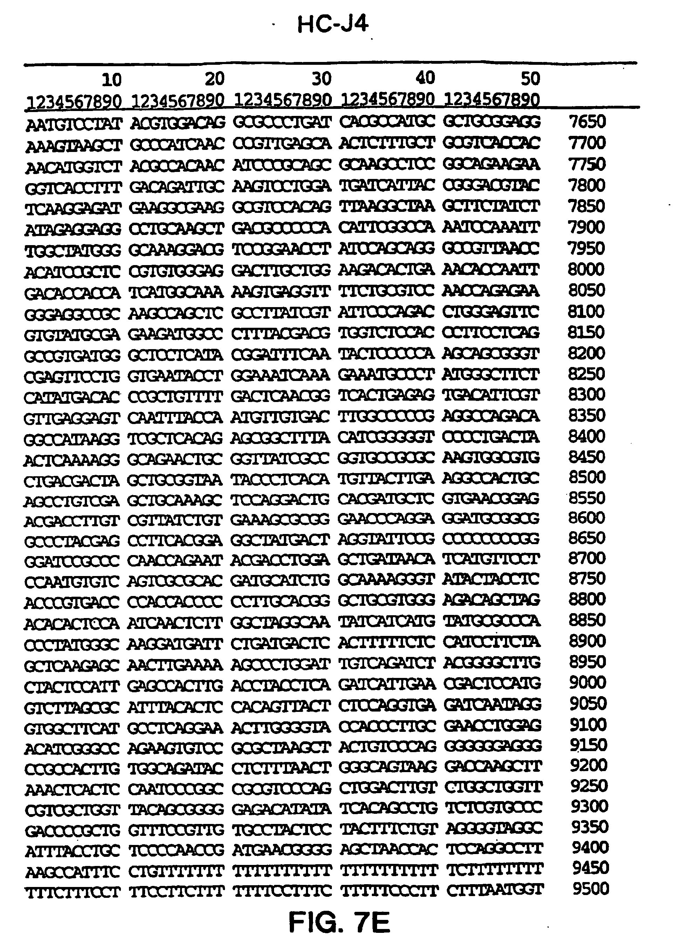

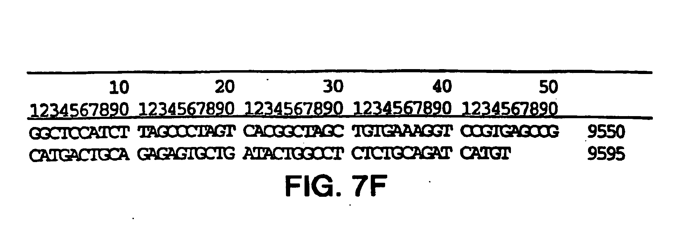

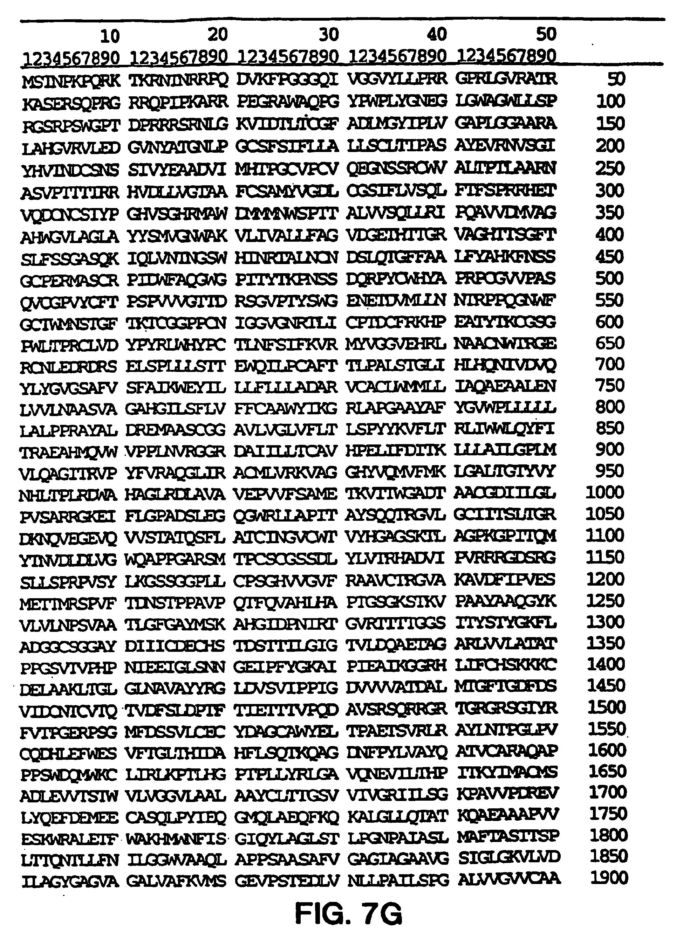

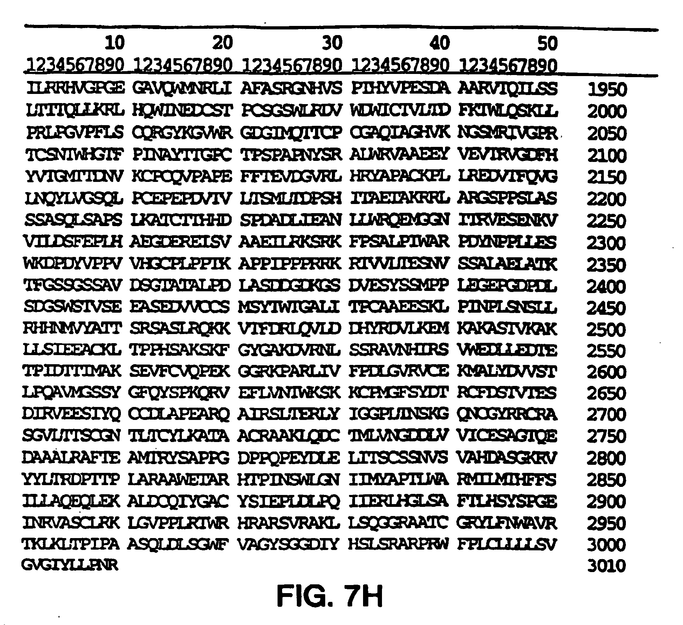

Figures 7A-7F show the nucleotide sequence of the infectious hepatitis C virus clone of genotype 1b strain HC-J4 and Figures 7G-H show the amino acid sequence encoded by the clone.

### DESCRIPTION OF THE INVENTION

The present invention relates to nucleic acid sequence which comprises the genome of an infectious hepatitis C virus. More specifically, the invention relates to nucleic acid sequence which encodes infectious hepatitis C virus of strain HC-J6_{CH}, genotype 2a. The infectious nucleic acid sequence of the invention is shown in SEQ ID NO:1 and is contained in a plasmid construct deposited with the American Type Culture Collection (ATCC) on May 28, 1999 and having ATCC accession number PTA-153.

The nucleic acid sequence from the genome of the infectious HCV clone of genotype 1a (deposited with the ATCC on June 2, 1999 ; Figures 6A-6F), or the nucleic acid sequence from the genome of the infectious HCV clone of genotype 1b (ATCC accession number 209596; Figures 7A-7F) can be used to construct the chimeric nucleic acid sequence with the HCV of genotype 2a of the invention.

It is believed that the construction of such chimeric nucleic acid sequences will be of importance in studying the growth and virulence properties of hepatitis C virus and in the production of candidate hepatitis C virus vaccines suitable to confer protection against multiple genotypes of HCV. For example, one might produce a "multivalent" vaccine by putting epitopes from several genotypes or subtypes into one clone. Alternatively one might replace just a single gene from an infectious sequence with the corresponding gene from the genomic sequence of a strain from another genotype or subtype or create a chimeric gene which contains portions of a gene from two genotypes or subtypes. Examples of genes which could be replaced or which could be made chimeric, include, but are not limited to, the E1, E2 and NS4 genes.

The invention further relates to mutations of the infectious nucleic acid sequences where "mutations" include, but are not limited to, point mutations, deletions and insertions. Of course, one of ordinary skill in the art would recognize that the size of the insertions would be limited by the ability of the resultant nucleic acid sequence to be properly packaged within the virion. Such mutations could be produced by techniques known to those of skill in the art such as site-directed mutagenesis, fusion PCR, and restriction digestion followed by religation.

In one embodiment, mutagenesis might be undertaken to determine sequences that are important for viral properties such as replication or virulence. For example, one may introduce a mutation into the infectious nucleic acid sequence which eliminates the cleavage site between the NS4A and NS4B polypeptides to examine the effects on viral replication and processing of the polypeptide.

Alternatively, one may delete all or part of a gene or of the 5' or 3' nontranslated region contained in an infectious nucleic acid sequence and then transfect a host cell (animal or cell culture) with the mutated sequence and measure viral replication in the host by methods known in the art such as RT-PCR. Preferred genes include, but are not limited to, the P7, NS4B and NS5A genes. Of course, those of ordinary skill in the art will understand that deletion of part of a gene, preferably the central portion of the gene, may be preferable to deletion of the entire gene in order to conserve the cleavage site boundaries which exist between proteins in the HCV polyprotein and which are necessary for proper processing of the polyprotein.

In the alternative, if the transfection is into a host animal such as a chimpanzee, one can monitor the virulence phenotype of the virus produced by transfection of the mutated infectious nucleic acid sequence by methods known in the art such as measurement of liver enzyme levels (alanine aminotransferase (ALT) or isocitrate dehydrogenase (ICD)) or by histopathology of liver biopsies. Thus, mutations of the infectious nucleic acid sequences may be useful in the production of attenuated HCV strains suitable for vaccine use.

The invention also relates to the use of the infectious nucleic acid sequence of the present invention to produce attenuated viral strains via passage in vitro or in vivo of the virus produced by transfection with the infectious nucleic acid sequence.

The present invention therefore relates to the use of the nucleic acid sequence of the invention to identify cell lines capable of supporting the replication of HCV.

In particular, it is contemplated that the mutations of the infectious nucleic acid sequence of the invention and the production of chimeric sequences as discussed above may be useful in identifying sequences critical for cell culture adaptation of HCV and hence, may be useful in identifying cell lines capable of supporting HCV replication.

Transfection of tissue culture cells with the nucleic acid sequences of the invention may be done by methods of transfection known in the art such as electroporation, precipitation with DEAE-Dextran or calcium phosphate or liposomes.

In one such embodiment, the method comprises the growing of animal cells, especially human cells, in vitro and transfecting the cells with the nucleic acid of the invention, then determining if the cells show indicia of HCV infection. Such indicia include the detection of viral antigens in the cell, for example, by immunofluorescence procedures well known in the art; the detection of viral polypeptides by Western blotting using antibodies specific therefor; and the detection of newly transcribed viral RNA within the cells via methods such as RT-PCR. The presence of live, infectious virus particles following such tests may also be shown by injection of cell culture medium or cell lysates into healthy, susceptible animals, with subsequent exhibition of the signs and symptoms of HCV infection.

Suitable cells or cell lines for culturing HCV include, but are not limited to, lymphocyte and hepatocyte cell lines known in the art.

Alternatively, primary hepatocytes can be cultured, and then infected with HCV; or, the hepatocyte cultures could be derived from the livers of infected chimpanzees. In addition, various immortalization methods known to those of ordinary skill in the art can be used to obtain cell lines derived from hepatocyte cultures. For example, primary hepatocyte cultures may be fused to a variety of cells to maintain stability.

The present invention further relates to the in vitro production of hepatitis C viruses from the nucleic acid sequences of the invention.

In one embodiment, the sequences of the invention can be inserted into an expression vector that functions in eukaryotic cells. Eukaryotic expression vectors suitable for producing high efficiency gene transfer in vivo are well known to those of ordinary skill in the art and include, but are not limited to, plasmids, vaccinia viruses, retroviruses, adenoviruses and adeno-associated viruses.

In another embodiment, the sequences contained in the recombinant expression vector can be transcribed in vitro by methods known to those of ordinary skill in the art in order to produce RNA transcripts which encode the hepatitis C viruses of the invention. The hepatitis C viruses of the invention may then be produced by transfecting cells by methods known to those of ordinary skill in the art with either the in vitro transcription mixture containing the RNA transcripts or with the recombinant expression vectors containing the nucleic acid sequences described herein.

The hepatitis C viruses produced from the sequences of the invention may be purified or partially purified from the transfected cells by methods known to those of ordinary skill in the art. In a preferred embodiment, the viruses are partially purified prior to their use as immunogens in the pharmaceutical compositions and vaccines of the present invention.

The present invention therefore relates to the use of the hepatitis C viruses produced from the nucleic acid sequences of the invention as immunogens in live or killed (e.g., formalin inactivated) vaccines to prevent hepatitis C in a mammal.

In an alternative embodiment, the immunogen of the present invention may be an infectious nucleic acid sequence, or a mutated infectious nucleic acid sequence which encodes a hepatitis C virus.

Alternatively, direct gene transfer may be accomplished via administration of a eukaryotic expression vector containing a nucleic acid sequence of the invention.

In yet another embodiment, the immunogen may be a polypeptide encoded by the nucleic acid sequences of the invention. The present invention therefore also relates to polypeptides produced from the nucleic acid sequences of the invention or fragments thereof. In one embodiment, polypeptides of the present invention can be recombinantly produced by synthesis from the nucleic acid sequences of the invention or isolated fragments thereof, and purified, or partially purified, from transfected cells using methods already known in the art. In an alternative embodiment, the polypeptides may be purified or partially purified from viral particles produced via transfection of a host cell with the nucleic acid sequences of the invention. Such polypeptides might, for example, include either capsid or envelope polypeptides prepared from the sequences of the present invention.

When used as immunogens, the nucleic acid sequences of the invention, or the polypeptides or viruses produced therefrom, are preferably partially purified prior to use as immunogens in pharmaceutical compositions and vaccines of the present invention. When used as a vaccine, the sequences and the polypeptide and virus products thereof, can be administered alone or in a suitable diluent, including, but not limited to, water, saline, or some type of buffered medium. The vaccine according to the present invention may be administered to an animal, especially a mammal, and most especially a human, by a variety of routes, including, but not limited to, intradermally, intramuscularly, subcutaneously, or in any combination thereof.

Suitable amounts of material to administer for prophylactic and therapeutic purposes will vary depending on the route selected and the immunogen (nucleic acid, virus, polypeptide) administered. One skilled in the art will appreciate that the amounts to be administered for any particular treatment protocol can be readily determined without undue experimentation. The vaccines of the present invention may be administered once or periodically until a suitable titer of anti-HCV antibodies appear in the blood. For an immunogen consisting of a nucleic acid sequence, a suitable amount of nucleic acid sequence to be used for prophylactic purposes might be expected to fall in the range of from about 100 µg to about 5 mg and most preferably in the range of from about 500 µg to about 2mg. For a polypeptide, a suitable amount to use for prophylactic purposes is preferably 100 ng to 100 µg and for a virus 10² to 10⁶ infectious doses. Such administration will, of course, occur prior to any sign of HCV infection.

A vaccine of the present invention may be employed in such forms as capsules, liquid solutions, suspensions or elixirs for oral administration, or sterile liquid forms such as solutions or suspensions. An inert carrier is preferably used, such as saline or phosphate-buffered saline, or any such carrier in which the HCV of the present invention can be suitably suspended. The vaccines may be in the form of single dose preparations or in multi-dose flasks which can be utilized for mass-vaccination programs of both animals and humans. For purposes of using the vaccines of the present invention reference is made to Remington's Pharmaceutical Sciences, Mack Publishing Co., Easton, Pa., Osol (Ed.) (1980); and New Trends and Developments in Vaccines, Voller et al. (Eds.), University Park Press, Baltimore, Md. (1978), both of which provide much useful information for preparing and using vaccines. Of course, the polypeptides of the present invention, when used as vaccines, can include, as part of the composition or emulsion, a suitable adjuvant, such as alum (or aluminum hydroxide) when humans are to be vaccinated, to further stimulate production of antibodies by immune cells. When nucleic acids, viruses or polypeptides are used for vaccination purposes, other specific adjuvants such as CpG motifs (Krieg, A.K. et al.(1995) and (1996)), may prove useful.

When the nucleic acids, viruses and polypeptides of the present invention are used as vaccines or inocula, they will normally exist as physically discrete units suitable as a unitary dosage for animals, especially mammals, and most especially humans, wherein each unit will contain a predetermined quantity of active material calculated to produce the desired immunogenic effect in association with the required diluent. The dose of said vaccine or inoculum according to the present invention is administered at least once. In order to increase the antibody level, a second or booster dose may be administered at some time after the initial dose. The need for, and timing of, such booster dose will, of course, be determined within the sound judgment of the administrator of such vaccine or inoculum and according to sound principles well known in the art. For example, such booster dose could reasonably be expected to be advantageous at some time between about 2 weeks to about 6 months following the initial vaccination. Subsequent doses may be administered as indicated.

The nucleic acid sequences, viruses and polypeptides of the present invention can also be administered for purposes of therapy, where a mammal, especially a primate, and most especially a human, is already infected, as shown by well known diagnostic measures. When the nucleic acid sequences, viruses or polypeptides of the present invention are used for such therapeutic purposes, much of the same criteria will apply as when it is used as a vaccine, except that inoculation will occur post-infection. Thus, when the nucleic acid sequences, viruses or polypeptides of the present invention are used as therapeutic agents in the treatment of infection, the therapeutic agent comprises a pharmaceutical composition containing a sufficient amount of said nucleic acid sequences, viruses or polypeptides so as to elicit a therapeutically effective response in the organism to be treated. Of course, the amount of pharmaceutical composition to be administered will, as for vaccines, vary depending on the immunogen contained therein (nucleic acid, polypeptide, virus) and on the route of administration.

The therapeutic agent according to the present invention can thus be administered by subcutaneous, intramuscular or intradermal routes. One skilled in the art will certainly appreciate that the amounts to be administered for any particular treatment protocol can be readily determined without undue experimentation. Of course, the actual amounts will vary depending on the route of administration as well as the sex, age, and clinical status of the subject which, in the case of human patients, is to be determined with the sound judgment of the clinician.

The therapeutic agent of the present invention can be employed in such forms as capsules, liquid solutions, suspensions or elixirs, or sterile liquid forms such as solutions or suspensions. An inert carrier is preferably used, such as saline, phosphate-buffered saline, or any such carrier in which the HCV of the present invention can be suitably suspended. The therapeutic agents may be in the form of single dose preparations or in the multi-dose flasks which can be utilized for mass-treatment programs of both animals and humans. Of course, when the nucleic acid sequences, viruses or polypeptides of the present invention are used as therapeutic agents they may be administered as a single dose or as a series of doses, depending on the situation as determined by the person conducting the treatment.

The nucleic acids, polypeptides and viruses of the present invention can also be utilized in the production of antibodies against HCV. The term "antibody" is herein used to refer to immunoglobulin molecules and immunologically active portions of immunoglobulin molecules. Examples of antibody molecules are intact immunoglobulin molecules, substantially intact immunoglobulin molecules and portions of an immunoglobulin molecule, including those portions known in the art as Fab, F(ab')₂ and F(v) as well as chimeric antibody molecules.

Thus, the polypeptides, viruses and nucleic acid sequences of the present invention can be used in the generation of antibodies that immunoreact (i.e., specific binding between an antigenic determinant-containing molecule and a molecule containing an antibody combining site such as a whole antibody molecule or an active portion thereof) with antigenic determinants on the surface of hepatitis C virus particles.

The present invention therefore also relates to antibodies produced following immunization with the nucleic acid sequences, viruses or polypeptides of the present invention. These antibodies are typically produced by immunizing a mammal with an immunogen or vaccine to induce antibody molecules having immunospecificity for polypeptides or viruses produced in response to infection with the nucleic acid sequences of the present invention. When used in generating such antibodies, the nucleic acid sequences, viruses, or polypeptides of the present invention may be linked to some type of carrier molecule. The resulting antibody molecules are then collected from said mammal. Antibodies produced according to the present invention have the unique advantage of being generated in response to authentic, functional polypeptides produced according to the actual cloned HCV genome.

The antibody molecules of the present invention may be polyclonal or monoclonal. Monoclonal antibodies are readily produced by methods well known in the art. Portions of immunoglobin molecules, such as Fabs, as well as chimeric antibodies, may also be produced by methods well known to those of ordinary skill in the art of generating such antibodies.

The antibodies according to the present invention may also be contained in blood, plasma, serum, hybridoma supernatants, and the like. Alternatively, the antibody of the present invention is isolated to the extent desired by well known techniques such as, for example, using DEAE Sephadex. The antibodies produced according to the present invention may be further purified so as to obtain specific classes or subclasses of antibody such as IgM, IgG, IgA, and the like. Antibodies of the IgG class are preferred for purposes of passive protection.

The antibodies of the present invention are useful in the prevention and treatment of diseases caused by hepatitis C virus in animals, especially mammals, and most especially humans.

In providing the antibodies of the present invention to a recipient mammal, preferably a human, the dosage of administered antibodies will vary depending on such factors as the mammal's age, weight, height, sex, general medical condition, previous medical history, and the like.

In general, it will be advantageous to provide the recipient mammal with a dosage of antibodies in the range of from about 1 mg/kg body weight to about 10 mg/kg body weight of the mammal, although a lower or higher dose may be administered if found desirable. Such antibodies will normally be administered by intravenous or intramuscular route as an inoculum. The antibodies of the present invention are intended to be provided to the recipient subject in an amount sufficient to prevent, lessen or attenuate the severity, extent or duration of any existing infection.

The antibodies prepared by use of the nucleic acid sequences, viruses or polypeptides of the present invention are also highly useful for diagnostic purposes. For example, the antibodies can be used as in vitro diagnostic agents to test for the presence of HCV in biological samples taken from animals, especially humans. Such assays include, but are not limited to, radioimmunoassays, EIA, fluorescence, Western blot analysis and ELISAs. In one such embodiment, the biological sample is contacted with antibodies of the present invention and a labeled second antibody is used to detect the presence of HCV to which the antibodies are bound.

Such assays may be, for example, direct where the labeled first antibody is immunoreactive with the antigen, such as, for example, a polypeptide on the surface of the virus; indirect where a labeled second antibody is reactive with the first antibody; a competitive protocol such as would involve the addition of a labeled antigen; or sandwich where both labeled and unlabeled antibody are used, as well as other protocols well known and described in the art.

In one embodiment, an immunoassay method would utilize an antibody specific for HCV envelope determinants and would further comprise the steps of contacting a biological sample with the HCV-specific antibody and then detecting the presence of HCV material in the test sample using one of the types of assay protocols as described above. Polypeptides and antibodies produced according to the present invention may also be supplied in the form of a kit, either present in vials as purified material, or present in compositions and suspended in suitable diluents as previously described.

For all prophylactic, therapeutic and diagnostic uses, the antibodies of the invention and other reagents, plus appropriate devices and accessories, may be provided in the form of a kit so as to facilitate ready availability and ease of use.

The present invention also relates to the use of nucleic acid sequences and polypeptides of the present invention to screen potential antiviral agents for antiviral activity against HCV. Such screening methods are known by those of skill in the art. Generally, the antiviral agents are tested at a variety of concentrations, for their effect on preventing viral replication in cell culture systems which support viral replication, and then for an inhibition of infectivity or of viral pathogenicity (and a low level of toxicity) in an animal model system.

In one embodiment, animal cells (especially human cells) transfected with the nucleic acid sequences of the invention are cultured in vitro and the cells are treated with a candidate antiviral agent (a chemical, peptide etc.) by adding the candidate agent to the medium. The treated cells are then exposed, possibly under transfecting or fusing conditions known in the art, to the nucleic acid sequences of the present invention. A sufficient period of time would then be allowed to pass for infection to occur, following which the presence or absence of viral replication would be determined versus untreated control cells by methods known to those of ordinary skill in the art. Such methods include, but are not limited to, the detection of viral antigens in the cell, for example, by immunofluorescence procedures well known in the art; the detection of viral polypeptides by Western blotting using antibodies specific therefor; the detection of newly transcribed viral RNA within the cells by RT-PCR; and the detection of the presence of live, infectious virus particles by injection of cell culture medium or cell lysates into healthy, susceptible animals, with subsequent exhibition of the signs and symptoms of HCV infection. A comparison of results obtained for control cells (treated only with nucleic acid sequence) with those obtained for treated cells (nucleic acid sequence and antiviral agent) would indicate, the degree, if any, of antiviral activity of the candidate antiviral agent. Of course, one of ordinary skill in the art would readily understand that such cells can be treated with the candidate antiviral agent either before or after exposure to the nucleic acid sequence of the present invention so as to determine what stage, or stages, of viral infection and replication said agent is effective against.

In an alternative embodiment, viral enzyme such as NS3 protease, NS2-NS3 protease, NS3 helicase or NS5B RNA polymerase may be produced from a nucleic acid sequence of the invention and used to screen for inhibitors which may act as antiviral agents. The structural and nonstructural regions of the HCV genome, including nucleotide and amino acid locations, have been determined, for example, as depicted in Houghton, M. (1996), Fig. 1; and Major, M.E. et al. (1997), Table 2.

Such above-mentioned protease inhibitors may take the form of chemical compounds or peptides which mimic the known cleavage sites of the protease and may be screened using methods known to those of skill in the art (Houghton, M. (1996) and Major, M.E. et al. (1997)). For example, a substrate may be employed which mimics the protease's natural substrate, but which provides a detectable signal (e.g. by fluorimetric or colorimetric methods) when cleaved. This substrate is then incubated with the protease and the candidate protease inhibitor under conditions of suitable pH, temperature etc. to detect protease activity. The proteolytic activities of the protease in the presence or absence of the candidate inhibitor are then determined.

In yet another embodiment, a candidate antiviral agent (such as a protease inhibitor) may be directly assayed in vivo for antiviral activity by administering the candidate antiviral agent to a chimpanzee transfected with a nucleic acid sequence of the invention or infected with a virus of the invention and then measuring viral replication in vivo via methods such as RT-PCR. Of course, the chimpanzee may be treated with the candidate agent either before or after transfection with the infectious nucleic acid sequence or infected with a virus of the invention so as to determine what stage, or stages, of viral infection and replication the agent is effective against.

The invention also provides that the nucleic acid sequences, viruses and polypeptides of the invention may be supplied in the form of a kit, alone or in the form of a pharmaceutical composition.

All scientific publication and/or patents cited herein are specifically incorporated by reference. The following examples illustrate various aspects of the invention but are in no way intended to limit the scope thereof.

### EXAMPLES

### Materials and Methods

### Source of HCV

An infectious plasma pool of HCV genotype 2a (HC-J6_{CH}) prepared from acute phase plasma of a chimpanzee experimentally inoculated with plasma from a Japanese patient infected with strain HC-J6 (Okamoto *et al.*, 1991) was used for cloning. An infectious cDNA clone of HCV strain H77, genotype 1a was also used (pCV-H77C; Yanagi *et al*., 1997).

### Amplification, cloning and sequence analysis

Viral RNA was extracted from 100 µl aliquots of the HC-J6_{CH} plasma pool with the TRIzol system (GIBCO/BRL) (Yanagi *et al.*, 1997). Primers used in cDNA synthesis and PCR amplification were based on the genomic sequence of strain HC-J6 (Okamoto *et al*., 1991) and from the conserved region (3'X) of the 3' UTR of HCV genotype 2a (Tanaka et al., 1996) (Table 1). The RNA was denatured at 65°C for 2 min, and cDNA was synthesized at 42°C for 1 hour with Superscript II reverse transcriptase (GIBCO/BRL) and specific reverse primers in 20 µl reaction volumes. The cDNA mixtures were treated with RNase H and RNase T1 (GIBCO/BRL) at 37°C for 20 min.

**TABLE 1**

| Oligonucleotides used for amplification and cloning of strain HC-J6_{CH}, genotype 2a | |
|---|---|
| Designation | Sequence (5' → 3')a |
| 2427S-H77 | ACTGGACACGGAGGTGGCCGCGTC |
| 2426S-H77 | TTGTTCTTGTCGGGTTAATGGCGC |
| 2645R-H77 | GGGTGTACTACACACATGAGTAAG |
| 2832R-H77 | AAGCGCCCCTAACTGATGATG |
| H2751SII | CGTC**ATCGATA**CCTCAGCGGGC**ATATG**CACTGGACACGGA |
| H2786R | GTCCAGTGCATATGCCCGCTGAGG |
| H2870R | CATGCACCAGCTGATATAGCGCTTGTAATATG |
| H7851S | TCCGTAGAGGAAGCTTGCAGCCTGACGCCC |
| H9140S (M) | CAGAGGAGGCAGGGTGCTATATGTGTGGCAAGTAC |
| H9173R (M) | GTACTTGCCACATATAGCAGCCCTGCCTCCTCTG |
| H9471R | **CGTCTCTAGA**CAGGAAATGGCTTAAGAGGCCGGAGTGTTTACC |
| J6-H2556S | TTATGGATGCTCATCTTGTTGGGCCAGGCCGAAGCAGCTTTGGAGAACCTCGTAATACT CAATGC |
| 356RF-J6H | AGGATTTGTGCTCATGGTGCACGGTCTACGAG |
| 1S-J6F^{b} | **TTTTTTTTGCGGCCGC*TAATACGACGACTCACTATAG***ACCCGCCCTAATAGG |
| 333S-J6 | CCGTGCACCATGAGCACAAATCCTAAACCTC |
| 753R-J6 | GGATGTACCCCATGAGGTCGGCAAAG |
| 2543S-J6F | GTTTGCGCCTGCTTATGGATGCTCATCTTG |
| 2787R-J6(26) | GCGTCATAAGCATA**T**GCCTGTTGGGG |
| 3329R-J6 | CCCTCAGCACTGGAGTACATCTG |
| 5487-J6F | **CGTCATGCATA**CCCCTAGGGCGGCTCTCATTGAAGAGGG |
| 5518R-J6F | CGTCCCCTCTTCAATGAGAGCCGCTCTAGA |
| 9251S-J6F | GCGGTGAAGACCAAGCTCAAACTCACTC |
| 9305R-J6F | **AATCTAGA**AGGCGCGCTTCCGGCAATGGAGTGAGTTTGAGC |
| 9310R-J6F | CGTC**TCTAGA**GGATAAATCCAGGAGGCGCGCTTCCGGC |
| 9399S-J6F | TACTTTTTGTAGGGGTAGGCCTTTTCC |
| 9464-J6F | CG**T**C**TCTAGA**GTGTAGCTAATGTGTGCCGCTCTA |
| 9470(24)-J6 | CTATGGAGTGTAGCTAATGTGTGC |
| J6-3' XR | CG**TC****TCTA**GACATGATCTGCAGAGAGACCAGTTACGGCACTCTCTGFCAGTCATGCGGC TCACGGACCTTTCACAGCTAGCCGTGACTAGGGCTAAGATGGAGCCACC |

| | |
|---|---|
| *a* HCV-specific sequences are shown in plain text, non HCV-specific sequences are shown in bold face, and cleavage sites used for cDNA cloning are underlined. *b* The core sequence of the T7 promotor is shown in italics. | |

The strategy used to amplify and clone the full-length HC-J6_{CH} sequence is shown in Fig. 1. Nucleotide positions correspond to those of the 2a infectious clone (pJ6CF) that is described herein. The 5' end of HC-J6_{CH} (nts. 17-297, excluding primer sequences) was amplified from 2 µl of cDNA synthesized with primer a-2 (Yanagi et al., 1996). PCR was performed with *AmpliTaq Gold* DNA polymerase (Perkin-Elmer) as described previously (Yanagi *et al*., 1996) using primers 1S-J6F and a-2. After purification, the amplified products were cloned into pGEM-T Easy vector (Promega) using standard procedures and 5 clones (pJ6-5'UTR) were sequenced.

The 3' end of HC-J6_{CH} was amplified in 3 overlapping pieces. RT-PCR of a short fragment of NS5B (nts. 9279-9439) was performed with primers 9251S-J6F and 9464R-J6F as described above. The PCR products were cloned into pGEM-T Easy vector and sequence analysis was performed from 5 pJ6-3'F clones. A second region spanning from NS5B to the conserved region of the 3' UTR (nts. 9376-9629) was amplified in RT-nested PCR (external primers H9261F and H3'X58R, internal primers H9282F and H3'X45R) (Yanagi *et al*., 1997). The amplified products were cloned into pGEM-9zf(-) by using *Hind*III and *Xba*I sites and 14 pJ6-3'VR clones were sequenced. The third fragment, which included the 3' terminal sequence was amplified with primers 9399S-J6F and J6-3'XR from one of the pJ6-3'VR clones, and cloned into one of the pJ6-3'F clones by using *Stu*I and *Xba*I sites (pJ6-3'X).

The ORF of HCV HC-J6_{CH} was amplified by long RT-PCR in 3 overlapping pieces. The amplification was performed on 2 µl of the cDNA mixtures with the Advantage cDNA polymerase mix (Clontech) (Yanagi *et al.*, 1997). The J6S fragment (nts. 86-2761) was amplified with primers a-1 (Yanagi et al., 1996) and J6-2787R from cDNA synthesized with primer J6-3329R. A single PCR round was performed in a Robocycler thermal cycler (Stratagene), and consisted of denaturation at 99°C for 35 sec, annealing at 67°C for 30 sec and elongation at 68°C for 4 min 30 sec during the first 5 cycles, 5 min during the next 10 cycles, 5 min 30 sec during the following 10 cycles and 6 min during the last 10 cycles. The J6B fragment (nts. 2573-5488) was amplified with primers 2543S-J6F and 5518R-J6F from cDNA synthesized with primer 5518R-J6F. Finally, the J6A fragment (nts. 5515-9282) was amplified with primers 5487S-J6F and 9310R-J6F from cDNA synthesized with primer 9470R(24)-J6F. PCR amplifications of fragments J6B and J6A consisted of denaturation at 99°C for 35 sec, annealing at 67°C for 30 sec and elongation at 68°C for 6 min during the first 5 cycles, 7 min during the next 10 cycles, 8 min during the following 10 cycles and 9 min during the last 10 cycles.

After purification of the long PCR products with QIAquick PCR purification kit (QIAGEN), A-tailing reactions were performed with *AmpliTaq* DNA polymerase (Perkin Elmer) at 72 °C for 1 hour. The gel-purified A-tailed PCR products were cloned into pCR2.1 vector (Invitrogen) or pGEM-T Easy vector (Promega). DH5-alpha competent cells (GIBCO BRL) were transformed and selected on LB agar plates containing 100 µg/ml ampicillin (SIGMA) and amplified in LB liquid cultures at 30°C for 18 - 20 hrs (Yanagi *et al*., 1997). Midiprep was performed using Wizard Plus Midipreps DNA Purification System (Promega). Multiple clones of the J6S, J6A and the J6B fragments were sequenced.

The consensus sequence of strain HC-J6_{CH} (nts. 17-9629) was determined by direct sequencing of PCR products (nts. 297-3004 and nts. 4893-5762) and by sequence analysis of the TA clones (nts. 17-5488 and nts. 5515-9629)(Fig. 1). Both strands of DNA were sequenced in all cases. Analyses of genomic sequences, including multiple sequence alignments and tree analyses, were performed with GeneWorks (Oxford Molecular Group) (Bukh *et al.,* 1995).

### Construction of chimeric cDNA clones of genotypes 1a & 2a

Four full-length intertypic chimeric cDNA clones were constructed (Figs. 4, 5A, 5B). In each clone the C, E1 and E2 genes encoded the consensus amino acid sequence of HC-J6_{CH}. The p7 protein was encoded either by the HC-J6_{CH} or pCV-H77C consensus sequence, and the NS proteins were all encoded by pCV-H77C genes. To engineer these cDNA clones, an *Nde*I site from pCV-H77C was first eliminated by a silent substitution (C to T) at position 9158. In brief, two fragments were amplified from pCV-H77C with primers H7851S and H9173R(M) and with primers H9140S(M) and H9417R (Table 3), gel-purified and used for fusion PCR with primers H7851S and H9417R. The fusion PCR products were cloned into pCV-H77C by using *Hind*III and *Afl*II sites. A new artificial NdeI site was introduced by a silent substitution (C to T) at position 2765. PCR products, which were amplified from pCV-H77C with primer H2751SII containing artificial *Cla*I and *Nde*I sites and primer H2870R, were cloned into the modified pCV-H77C by using *ClaI* and *Eco*47III sites. The final construct (pH77CV) was used as a cassette vector to construct the intertypic chimeric HCV cDNA clones.

The four chimeric cDNA clones were constructed as follows. pH77CV-J6S (nucleotide sequence shown in SEQ ID No:3 and amino acid sequence shown in SEQ ID No:4): The *Age*I/*Bsm*I fragment of clone J6S2 and the *Bsm*I/*Nde*I fragment of clone J6S1, were cloned into pH77CV by using *Age*I and *Nde*I sites; pH77 (p7)CV-J6S (nucleotide sequence shown in SEQ ID No:5 and amino acid sequence shown in SEQ ID No:6): A fragment of pH77CV-J6S was replaced with a fragment amplified from pCV-H77C with primers J6-H2556S and H2786R by using *BsaB*I and *Nde*I sites; J6S (nucleotide sequence shown in SEQ ID No:7 and amino acid sequence shown in SEQ ID No:8): A fragment amplified from pH77pCV-H77C with primers a-1 and 356RF-J6H77 and another fragment amplified from pH77CV-J6S with primers 333S-J6 and 753R-J6 were gel-purified and a fusion-PCR was performed with primers a-1 and 753R-J6. The *Age*I/*Cla*I fragment of the subcloned fusion PCR products and the *Cla*I/*Nde*I fragment of pH77CV-J6S were cloned into pH77CV-J6S by using AgeI and *Nde*I sites; pH77(p7)-J6S (nucleotide sequence shown in SEQ ID No:9 and amino acid sequence shown in SEQ ID No:10): The AgeI/ClaI fragment of J6S and the *Cla*I/*Nde*I fragment of (p7)CV-J6S were cloned into pH77(p7)CV-J6S by using *Age*I and *Nde*I sites.

Each intertypic chimeric cDNA clone was retransformed to select a single clone, and large-scale preparation of plasmid DNA was performed with a QIAGEN plasmid Maxi kit as described previously (Yanagi *et al*., 1997). Each of the four cDNA clones was completely sequenced before inoculation. Each clone was genetically stable since the digestion pattern was as expected following retransformation and the complete sequence was the expected one.

### Construction of full-length cDNA clone HC-J6_{CH}

An overview of the full-length HC-J6_{CH} clone is presented in Fig. 1. In the final construct pJ6CF, which encodes the consensus polyprotein of HC-J6_{CH}, an *Xba*I site was eliminated by a silent substitution (A to G) at position 5494. Digested fragments containing the consensus sequence were purified from the appropriate subclones and ligated using the sites indicated. The full-length cDNA clone (pJ6CF) was retransformed to select a single clone, and large-scale preparation of plasmid DNA followed by the complete sequence analysis was performed. Clone pJ6CF was genetically stable.

### Intrahepatic transfection of chimpanzee with transcribed RNA

In duplicate 100 µl reactions, RNA was transcribed *in vitro* with T7 RNA polymerase (Promega) from 10 µg of template plasmid linearized with *Xba*I (Promega) as described previously (Yanagi *et al.*, 1997). The integrity of the RNA was checked by electrophoresis through agarose gel stained with ethidium bromide (Yanagi *et al*., 1997). Each transcription mixture was diluted with 400 µl of ice-cold phosphate-buffered saline without calcium or magnesium and then immediately frozen on dry ice and stored at -80°C. Within 24 hours, both transcription mixtures were injected into the same chimpanzee by percutaneous intrahepatic injection guided by ultrasound (Yanagi *et al*., 1998, 1999). If the chimpanzee did not become infected, the same transfection was repeated once. After two negative results, the next clone was inoculated into the same chimpanzee following the same protocol. Injections were performed at weeks 0 and 2 with pH77CV-J6S, at weeks 5 and 8 with pH77(p7)CV-J6S, at weeks 14 and 16 with pH77-J6S, at weeks 19 and 23 with pH77(p7)-J6S, at week 28 with.pJ6CF, and finally at week 34 with pCV-H77C. The chimpanzee was maintained under conditions that met or exceeded all requirements for its use in an approved facility.

Serum samples were collected weekly from the chimpanzee and monitored for liver enzyme levels by standard procedures, anti-HCV antibodies by the second-generation ELISA (Abbott) and HCV RNA by a sensitive RT-nested PCR assay with *AmpliTaq Gold* DNA polymerase using primers from the 5' UTR (Yanagi *et al*., 1996). Samples were scored as negative for HCV RNA if two independent tests on 100 µl of serum were negative. The genome equivalent (GE) titer of HCV in positive samples was determined by RT-nested PCR on 10-fold serial dilutions of the extracted RNA (Bukh *et al*., 1998). The consensus sequence of the complete ORF from the chimpanzee infected with RNA transcripts of pJ6CF was determined by direct sequencing of overlapping PCR products obtained by long RT-nested PCR as previously described (Yanagi *et al.,* 1997) with HC-J6 specific primers. After the intrahepatic transfection with RNA transcripts of pCV-H77C, we performed H77(genotype 1a)-specific RT-nested PCR with primers 2427S-H77 and 2832R-H77 for the 1st round and with primers 2462S-H77 and 2645R-H77 for the 2nd round (Table 3). The sensitivity of this assay was equivalent to that of the assay using 5' UTR primers when testing serum containing only H77, genotype 1a. The genome titer of genotype 1a was determined by using this specific RT- nested PCR on 10-fold serial dilutions of the extracted RNA.

### EXAMPLE 1

### Sequence analysis of HCV strain HC-J6_{CH}

As minor deviations from the consensus amino acid sequence were found previously to render full-length HCV cDNA clones noninfectious (Yanagi *et al*., 1997, 1998), the consensus sequence of the cloning source of genotype 2a (strain HC-J6_{CH}) was determined prior to constructing any full-length clones. In brief, a plasma pool containing strain HC-J6_{CH} was prepared from acute phase plasmapheresis units collected from a chimpanzee experimentally infected with HC-J6 (Okamoto *et al*., 1991). The HCV genome titer of this pool was 10^{5.4} genome equivalents (GE)/ml (Quantiplex HCV RNA bDNA 2.0, Chiron) and the infectivity titer was 10⁴ chimpanzee infectious doses/ml.

The consensus sequence of the 5' UTR of HC-J6_{CH} (nts. 17-340) was deduced from 5 clones containing nts. 17-297 and 8 clones containing nts. 86-340. The 5' UTR of the various clones was highly conserved, but the consensus sequence of HC-J6_{CH} differed by 2 nucleotides from that published previously for HC-J6 (Okamoto *et al*., 1991: C to T at position 36 and T to C at position 222).

The consensus sequence of 14 clones of the 3' UTR of HC-J6_{CH} indicated that the 39 nucleotide long variable region was highly conserved in this strain and was identical to that previously published for HC-J6 (Okamoto *et al*., 1991). The polypyrimidine tract varied greatly in length (84-164 nucleotides), and contained some conserved A residues. In the conserved region, the proximal 16 nucleotides were identical to those previously published for isolates of different HCV genotypes (Kolykhalov *et al.,* 1996; Tanaka *et al.,* 1996; Yamada *et al*., 1996). The remaining 82 nucleotides of the conserved region were determined for other genotype 2a strains (Tanaka *et al.*, 1996) but not for HC-J6 or HC-J6_{CH}.

The ORF of HC-J6_{CH} was amplified in 3 fragments by RT-PCR (Fig. 1). Eight clones of the J6S fragment (nts. 86-2761), 6 clones of the J6B fragment (nts. 2573-5488) and 6 clones of the J6A fragment (nts. 5515-9298) were sequenced. PCR fragments containing nts. 5489-5514 were sequenced directly. A quasispecies was found at 243 nucleotide (2.7%) and 69 amino acid (2.3%) positions, scattered throughout the 9099 nts (3033 aa) of the ORF. However, the majority, 231 nucleotide substitutions, were detected only once and 71.6 % of these represented silent mutations. The 12 remaining nucleotide substitutions were each restricted to 2 clones and only 4 of these resulted in amino acid changes. The nucleotide difference among the J6S clones ranged from 0.1 - 1.3%, among the J6B clones it ranged from 0.1 - 0.3%, and it ranged from 0.2 - 4.0% among the J6A clones (Fig. 2). Three of 8 J6S clones, 4 of 6 J6B clones, and all 6 J6A clones had defective polyproteins due to nucleotide deletions, insertions or substitutions.

The sequences of clones of strain HC-J6_{CH} were relatively homogeneous. This was highlighted by the high degree of conservation among clones of the HVR1 (Fig. 3), a region frequently used to study the quasispecies of HCV (Bukh *et al.*, 1995). An exception was the sequence of clone J6A1, which differed by about 4% from the other clones of this region (Fig. 2). Importantly, the consensus sequence of strain HC-J6_{CH} (nts. 17-9629) could be determined with no ambiguity at the nucleotide or deduced amino acid level. The difference between the consensus ORF sequence of HC-J6_{CH} from the experimentally infected chimpanzee and that of HC-J6 of the inoculum (Okamoto *et al*., 1991) was 4.1 % and 2.2 % at the nucleotide and deduced amino acid levels, respectively (Fig. 2, Table 2). Moreover, we found that 12 (44.4%) of the 27 amino acids constituting HVR1 differed between HC-J6_{CH} and HC-J6 (Fig. 3). Such diversities are greater than the < 2 % generally considered to comprise a quasispecies. In fact, these differences are equivalent to those found between the two prototype strains of HCV genotype la [strains HCV-1 (Choo *et al.,* 1991) and H77 (Yanagi *et al.,* 1997)]. These results indicated that HC-J6_{CH}, which represented the major species in the experimentally infected chimpanzee, was a minor species in the original inoculum.

**TABLE 2**

| Percent difference of nucleotide and predicted amino acid sequences between strain HC-J6 (Okamoto *et al.*, 1991) and strain HC-J6_{CH} from acute phase plasma pool of a chimpanzee inoculated with HC-J6 | | | |
|---|---|---|---|
| Genome Region | nt.position*^{a}* | % nt. difference | % a.a. difference |
| ORF | 341-9439 | 4.1 (373/9099)*^{b}* | 2.2 (66/3033)*^{b}* |
| 5' UTR | 17-340 | 0.6 (2/324) | |
| Core | 341-913 | 0.5 (3/573) | 0 (0/191) |
| E1. | 914-1489 | 4.3 (25/576) | 2.1 (4/192) |
| HVR1 | 1490-1570 | 24.7 (20/81) | 44.4 (12/27) |
| E2-_{HVR1} | 1571-2590 | 3.9 (40/1020) | 3.2 (11/340) |
| p7 | 2591-2779 | 3.7 (7/189) | 3.2 (2/63) |
| NS2 | 2780-3430 | 4.0 (26/651) | 2.8 (6/217) |
| NS3 | 3431-5323 | 4.0 (76/1893) | 0.8 (5/631) |
| NS4A | 5324-5485 | 4.3 (7/162) | 1.9 (1/54) |
| NS4B | 5486-6268 | 3.7 (29/783) | 0.4 (1/261) |
| NS5A | 6269-7666 | 5.4 (75/1398) | 3.4 (16/466) |
| NS5B | 7667-9439 | 3.7 (65/1773) | 1.4 (8/591) |
| 3' UTR | 9440-9481 | 0 (0/42) | |

| | | | |
|---|---|---|---|
| *^{a}* The nucleotide positions correspond to those of the infectious full-length genotype 2a clone (pJ6CF)*.* *^{b}* The numbers in parenthesis indicate the nucleotide or amino acid differences for each region. | | | |

### Example 2

### Chimeric molecular clones

As chimeric flaviviruses with substituted structural genes have been useful in defining the biological function of viral sequences or proteins, in analyzing immune responses and in generating attenuated vaccine candidates (Bray and Lai, 1991; Chambers *et al.,* 1999; Pletnev *et al.*, 1992, 1993, 1998). The consensus sequence of the 2a structural genes and surrounding region was substituted for that of the infectious 1a cDNA clone. In the genotype 1a backbone, two silent mutations were introduced for cloning purposes [at positions 2765 (p7) and 9158 (NS5B) of pCV-H77C] (Fig. 4). The complete sequence of each chimera was verified. Infectivity of RNA transcripts from four different intertypic chimeric clones (Figs. 4, 5A, 5B) was evaluated by consecutive intrahepatic transfections of a chimpanzee. Clones were considered not to be viable if viral RNA was not detected in the serum within two weeks of the repeat transfection. All chimeric clones contained the C, E1 and E2 genes of genotype 2a. The two chimeric clones tested initially differed from each other in that one had the p7 gene of 2a (pH77CV-J6S) and the other [pH77(p7)CV-J6S] the p7 gene of 1a. They differed from the two other clones in that the 186 nucleotides of the 5' UTR just upstream of the initiation codon were from the 2a genotype. Since neither clone containing the chimeric 5' UTR was infectious, the chimeric 5' UTR was replaced with the consensus genotype 1a 5' UTR to generate the two p7 varieties [pH77-J6S and pH77(p7)-J6S]. After consecutive transfection of the four clones, no HCV RNA, anti-HCV or ALT elevation was detected in the chimpanzee during 28 weeks of follow-up, suggesting that RNA transcripts from these intertypic chimeric clones were not viable *in vivo.*

This finding that the intertypic clones between genotypes 1a and 2a were not viable was surprising since flavivirus chimeras containing the structural region of dengue virus type 1 or 2 or of tick-borne encephalitis virus and the nonstructural region of an infectious dengue type 4 virus were viable (Bray and Lai, 1991; Pletnev *et al*., 1992, 1993). While considerable sequence variation exists between the infectious genotype 1a and 2a clones of HCV (Table 3), these viruses exhibit a higher degree of genetic heterogeneity than do the major genotypes of HCV. For other flaviviruses, however, it was possible to obtain infectious chimeric clones only if the capsid region was derived from the backbone cDNA clone (Chambers *et al.,* 1999; Pletnev and Men, 1998).

**TABLE 3**

| Percent difference of the amino acid sequences between the infectious clone of genotype 1a (pCV-H77C; Yanagi *et al.,* 1997) and the infectious clone of genotype 2a (pJ6CF) of hepatitis C virus | |
|---|---|
| Genome Region*^{a}* | % difference |
| Polyprotein | 27.9 (839/3007)*^{b}* |
| Core | 8.9 (17/191) |
| E1 | 37.0 (71/192) |
| HVR1 | 59.3 (16/27) |
| E2-_{HVR1} | 27.1 (91/336) |
| p7 | 38.1 (24/63) |
| NS2 | 41.9 (91/217) |
| NS3 | 19.2 (121/631) |
| NS4A | 33.3 (18/54) |
| NS4B | 26.8 (70/261) |
| NS5A | 38.5 (171/444) |
| NS5B | 25.2 (149/591) |

| | |
|---|---|
| *^{a}* Genome regions defined as in Table 1. *^{b}* The numbers in parenthesis indicate the amino acid differences for each region. Positions with deletions or insertions in E2 (4 aa positions) and NS5A (26 aa positions) were not considered. | |

Trivial explanations may account for the lack of viability of these intertypic chimeras. First, the two silent mutations introduced in the genotype 1a backbone (one in p7 and one in NS5B) for cloning purposes could potentially eliminate infectivity. This is, however, very unlikely since mutations at these positions exist among field isolates of HCV including strain HC-J6_{CH} (Bukh *et al*., 1998). Also, it is noteworthy that the three previously published infectious clones of strain H77 had numerous silent nucleotide differences (Hong et al., 1999; Kolykhalov **et al.**, 1997; Yanagi *et al*., 1997). Second, signal peptidases might not cleave the chimeric E2/p7 or p7/NS2 junction. This seems unlikely, however, since eukaryotic signal peptidases typically recognize the amino acid sequences upstream of the cleavage site [the (-3, -1) rule] (Nielsen *et al*., 1997) and the amino acids at these two sites are conserved between genotypes 1a and 2a (Fig. 5B). Finally, the E2/p7 and/or p7/NS2 gene junctions could differ between genotypes 1a and 2a. The junctions determined for genotypes 1a and 1b were used (Lin et al., 1994; Mizushima *et al.*, 1994; Selby **et al.**, 1994) because those for genotype 2a have not been identified. In the latter two cases, further analyses of genotype 2a should eventually provide sufficient data to overcome such potential problems and it would most likely be possible to construct a viable chimera.

More complicated explanations for the lack of viability of the chimeras might be required if critical genotype-specific interactions occur as regards the structural proteins, the nonstructural proteins and the genomic RNA. For instance, one cannot rule out that the chimeras were not viable because the IRES function was compromised. In *in vitro* studies the IRES activity depended on RNA sequences not only in the 5' UTR but also extending 3' of the translation initiation site (Hahm *et al*., 1998; Lemon and Honda, 1997; Reynolds *et al*., 1995). Although the 3' border of the HCV IRES is still controversial it is believed to involve at most the first 39 nts of the core gene (Lemon and Honda, 1997). The 5' UTR of the intertypic chimeras was either a chimera of genotype 1a and 2a sequences or the entire 5' UTR was derived from the 1a clone (Figs. 4, 5A). Importantly, the 5' end of core is conserved among genotypes 1a and 2a (Fig. 5A). Thus, the predicted IRES-like secondary structure is maintained in these chimeras, suggesting that the IRES activity most likely was maintained.

Possible interactions between the structural proteins and the nonstructural proteins and/or the genomic RNA, which involve RNA packaging, replication or translation are conceivable. In poliovirus, which is another positive-sense RNA virus, functional coupling of RNA packaging to RNA replication and of RNA replication to translation have been suggested (Novak and Kirkegaard, 1994 ; Nugent *et al*., 1999). Similar to other viruses of the *Flaviviridae* family, a membrane-associated replicase complex is thought to initiate replication at the 3' end of HCV and to synthesize a complementary negative-strand RNA (Rice, 1996). The putative cis-acting elements at the 5' and 3' termini which are believed to be important for viral genome replication (Rice 1996; Frolov et al., 1998) should be maintained in the intertypic HCV chimeras at least in the two constructs with the authentic 1a 5'UTR. However, it is conceivable that the viral packaging system was interrupted (Frolov *et al.*, 1998). Studies using a Kunjin flavivirus replicon system and providing the structural proteins *in trans* suggested that the essential encapsidation signals did not reside in the structural region of the genome (Khromykh *et al*., 1997, 1998). The location of the packaging signals of HCV is not known. However, if the structural proteins encapsidate viral RNA via genotype-specific sequences outside of the structural region, the chimeras would be unable to package the RNA and it might be extremely difficult to construct viable chimeras between highly divergent strains.

### Example 3

### A consensus molecular clone of genotype 2a is infectious in vivo

In order to prove that the genotype 2a portion used in the 4 intertypic chimeric cDNA clones indeed represented the infectious sequence, a consensus full-length cDNA clone of HC-J6_{CH} (pJ6CF) was constructed. The core sequence of the T7 promoter, a 5' guanosine residue and the full-length sequence of HC-J6_{CH} (9711 nts) were cloned into pGEM-9Zf vector using NotI/XbaI sites. Within the HCV sequence there were no deduced amino acid differences and only 4 nucleotide differences (at nucleotide positions 1822, 5494, 9247 and 9289) from the consensus sequence of HC-J6_{CH} as determined in the present study. The silent mutation at position 1822 was within the structural region and so was also present in the four intertypic chimeras. The 5' terminal 16 nts and the 3' terminal 82 nts were deduced from previously published HCV genotype 2a sequences (Okamoto *et al.*, 1991, Tanaka *et al.,* 1996). The full-length cDNA clone of genotype 2a contained a 5' UTR of 340 nts, an ORF of 9099 nts encoding 3033 amino acids and a 3' UTR consisting of a variable region of 39 nts followed by a 132 nucleotide-long polypyrimidine tract interrupted with 3 A residues and the 3' terminal conserved region of 98 nts.

RNA transcripts from pJ6CF were injected into the same chimpanzee used for injection of the 4 intertypic chimeras. The chimpanzee became infected at the first attempt with an HCV titer of 10² GE/ml at week 1 post inoculation (p.i.), and 10³-10⁴ GE/ml during weeks 2 to 6 p.i. The consensus sequence of PCR products of the complete ORF, amplified from serum obtained during week 5 p.i., was identical to the sequence of pJ6CF and there was no evidence of a quasispecies. Since RNA transcripts of this infectious genotype 2a clone were infectious *in vivo*, and it shared an exact sequence with the non-infectious intertypic chimeric clones, their failure to replicate must have been the result of incompatibilities between the genotype 1a and 2a sequences.

To confirm that the chimpanzee used was susceptible also to infection by genotype 1a, which comprised most of the intertypic chimeras, the chimpanzee was subsequently inoculated with RNA transcripts from the infectious genotype 1a clone (pCV-H77C). Serum samples were tested in an H77-specific RT-PCR assay to identify super-infection with genotype 1a. At week 1 p.i. the total HCV genome titer was 10⁴ GE/ml and the H77-specific (1a) genome titer was 10² GE/ml. The H77-specific genome titer increased to 10³ GE/ml at week 2 p.i., and reached 10⁴ GE/ml during weeks 3-6 p.i. The consensus sequence of PCR products amplified with H77-specific primers at weeks 1-6 p.i. were found to be identical to that of pCV-H77C. However, the direct sequences of PCR products amplified with the 5' UTR primers at weeks 1-2 after inoculation of pCV-H77C were identical to that of pJ6CF indicating that the 2a genotype was still present and represented the majority species. These experiments confirmed that the inability of the intertypic 1a, 2a cDNA clones to infect the chimpanzee was not the result of protective immune responses in the chimpanzee but represented deficiencies intrinsic to the chimeras.

### Discussion

The published infectious cDNA clones of HCV represent the two most important subtypes of genotype 1 (Hong *et al*., 1999; Kolykhalov et al., 1997; Yanagi et al., 1997, 1998). However, 5 more major genotypes of HCV are recognized. In the above Examples, the infectivity of a cDNA clone of a second major HCV genotype was demonstrated. As in previous studies, the infectivity of RNA transcripts was demonstrated *in vivo* by intrahepatic transfection of a chimpanzee. This new infectious clone (pJ6CF) encodes the consensus polyprotein of HCV strain HC-J6_{CH}, genotype 2a. Its encoded polyprotein differs from those of the infectious clones of genotypes 1a and 1b by approximately 30% (Table 2). Genotype 2 strains, in particular subtypes 2a and 2b, have a worldwide distribution and important differences between genotypes 1 and 2 with respect to pathogenesis and treatment were indicated in previous studies. The availability of an infectious clone representing a second major genotype of HCV should permit new ways of studying the molecular biology and immunopathology of this important and genetically quite different human pathogen.

The 5' and 3' UTRs of HCV are believed to be critical for viral replication, translation and viral packaging (Rice, 1996). The 5' 203 terminal nucleotides and the 3' 101 terminal nucleotides of the published infectious clones of genotypes 1a and 1b were identical. However, the sequences of UTRs of the genotype 2a clone differ from those of the genotype 1 clones. Overall, the 5' UTR of the genotype 2a clone has 17 nt differences and a single nucleotide deletion compared with the infectious clones of genotype 1a (Fig. 5A). Five of these differences and the deletion are within the first 30 nucleotides, whereas the remainder are found within the predicted IRES structure. Differences also exist between the 3' UTR of the genotype 2a clone and the clones of genotype 1a (Fig. 5B). The sequences of the variable region are very different. Recent study has shown this region is not critical for infectivity *in vivo* (Yanagi et *al.,* 1999). Within the regions which are critical for infectivity *in vivo* (Yanagi *et al*., 1999), the 132 nucleotide-long polypyrimidine tract of the genotype 2a clone has 3 unique A residues interspersed and the 3' terminal conserved region of 98 nts has 4 nt differences within the 3' terminal stable stem-loop structure (Fig. 5B) (Kolykhalov *et al.,* 1996; Tanaka *et al.*, 1996). Since the 2a clone was infectious these sequence differences are apparently real and are compatible with infectivity. Further studies are required to determine whether these represent critical genotype-specific sequences.

### References

1. Alter, M. J. (1997). Hepatology 26, 62S-65S.
2. Blight, K. J. and Rice, C. M. (1997). J. Virol. 71, 7345-7352.
3. Brechot, C. (1997). Hepatology 25, 772-774.
4. Bray, M. and Lai, C.-J. (1991). Construction of intertypic chimeric dengue viruses by substitution of structural protein genes. *Proc. Natl. Acad. Sci. USA* 88, 10342-10346.
5. Bukh, J., Apgar, C. L., Engle, R., Govindarajan, S., Hegerich, P. A., Tellier, R., Wong, D. C., Elkins, R. & Kew, M. C. (1998). Experimental infection of chimpanzees with hepatitis C virus of genotype 5a: genetic analysis of the virus and generation of a standardized challenge pool. *J*. *Infect. Dis.* 178, 1193-1197.
6. Bukh, J., Emerson, S. U. and Purcell, R. H. (1997). Genetic heterogeneity of hepatitis C virus and related viruses. In "Viral Hepatitis and Liver Disease, Proceedings of IX Triennial International Symposium on Viral Hepatitis and Liver Disease, Rome, Italy, 1996" (M. Rizzetto, R. H. Purcell, J. L. Gerin and G. Verme, Eds.), pp. 167-175. Edizioni Minerva Medica, Turin.
7. Bukh, J., Miller, R. H. and Purcell, R. H. (1995). Genetic heterogeneity of hepatitis C virus: quasispecies and genotypes. *Semin. Liver Dis.* 15, 41-63.
8. Bukh, J., Purcell, R. H. and Miller, R. H. (1993). At least 12 genotypes of hepatitis C virus predicted by sequence analysis of the putative E1 gene of isolates collected worldwide. *Proc. Natl. Acad. Sci. USA* 90, 8234-8238.
9. Choo, Q.-L., Richman, K. H., Han, J. H., Berger, K., Lee, C., Dong, C., Gallegos, C., Coit, D., Medina-Selby, A., Barr, P. J., Weiner, A. J., Bradley, D. W., Kuo, G. and Houghton M. (1991). Genetic organization and diversity of the hepatitis C virus. *Proc. Natl. Acad. Sci. USA* 88, 2451-2455.
10. Chambers T. J., Nestorowicz A., Mason P. W. and Rice C. M. (1999). Yellow Fever/Japanese Encephalitis Chimeric Viruses: Construction and Biological Properties. *J*. *Virol.* 73: 3095-3101.
11. Dash, S., et al. (1997). Am. J. Pathol. 151, 363-373.
12. Davis, G. L., Esteban-Mur, R., Rustgi, V., Hoefs, J.,Gordon, S. C., Trepo, C., Shiffman, M. L., Zeuzem, S., Craxi, A., Ling, M.-H. and Albrecht, J., for the international hepatits interventional therapy group. (1998). Interferon alfa-2b alone or in combination with ribavirin for the treatment of relapse of chronic hepatitis C. *N. Engl.* J. *Med.* 339, 1493-1499.
13. Fausto, N. (1997). Am. J. Pathol. 151, 361.
14. Forns, X., Bukh, J., Purcell, R. H., Emerson, S. U. (1997). How Escherichia coli can bias the results of molecular cloning: preferential selection of defective genomes of hepatitis C virus during the cloning procedure. *Proc. Natl. Acad. Sci. USA* 94, 13909-13914.
15. Forns, X. and Bukh, J. (1998). Methods for determining the hepatitis C virus genotype. *Viral Hepatitis Reviews* 4, 1-19.
16. Fried, M. W. and Hoofnagle, J. H. (1995). Semin. Liver Dis. 15, 82-91.
17. Frolov, I., McBride, M. S. and Rice, C. M. (1998). Cis-acting RNA elements required for replication of bovine viral diarrhea virus-hepatits C virus 5' nontranslated region chimeras. *RNA* 4, 1418-1435.
18. Hahm, B., Kim, Y. K., Kim, J. H., Kim, T. Y. and Jang, S. K. (1998). Heterogeneous nuclear ribonucleoprotein L interacts with the 3' border of the internal ribosomal entry site of hepatits C virus. J. Virol. 72, 8782-8788.
19. Hijikata, M., Kato, N., Ootsuyama, Y., Nakagawa, M., Ohkoshi, S. and Shimotohno, K. (1991). Hypervariable regions in the putative glycoprotein of hepatitis C virus. *Biochem. Biophys. Res. Commun.* 175, 220-228.
20. Honda, M., et al. (1996). RNA 2, 955-968.
21. Hong, Z., Beaudet-Miller, M., Lanford, R. E., Guerra, B., Wright-Minogue, J., Skelton, A., Baroudy, B. M., Reyes, G. R. and Lau, J. Y. N. (1999). Generation of transmissible hepatitis C virions from a molecular clone in chimpanzees. *Virology* 256, 36-44.
22. Hoofnagle, J. H. (1997). Hepatitis C: the clinical spectrum of disease. *Hepatology* 26, 15S-20S.
23. Houghton, M. (1996). Hepatitis C viruses. In "Fields Virology" (B. N. Fields, D. M. Knipe, P. M. Howley, et al., Eds.), Third ed., pp. 1035-1058. Lippincott-Raven Publishers, Philadelphia.
24. Khromykh, A. A. and Westaway, E. G. (1997). Subgenomic replicons of the flavivirus Kunjin: construction and applications. *J. Virol.* 71, 1497-1505.
25. Ito, T. and Lai, M. M. C. (1997). J. Virol. 71, 8698-8706.
26. Khromykh, A. A., Varnavski, A. N. and Westaway, E. G. (1998). Encapsidation of the flavivirus Kunjin replicon RNA by using a complementation system providing Kunjin virus structural proteins in *trans. J. Virol.* 72, 5967-5977.
27. Kolykhalov, A. A., Feinstone, S. M. and Rice, C. M. (1996). Identification of a highly conserved sequence element at the 3' terminus of hepatitis C virus genome RNA. *J. Virol.* 70, 3363-3371.
28. Kolykhalov, A. A., Agapov, E. V., Blight, K. J., Mihalik, K., Feinstone, S. M. and Rice, C. M. (1997). Transmission of hepatitis C by intrahepatic inoculation with transcribed RNA. *Science* 277, 570-574.
29. Lemon, S. M. and Honda, M. (1997). Internal ribosome entry sites within the RNA genomes of hepatits C virus and other flaviviruses. *Semin. Virol.* 8, 274-288.
30. Lin, C., Lindenbach, B. D., Pragai, B. M., McCourt, D. W. and Rice, C. M. (1994). Processing in the hepatitis C virus E2-NS2 region: identification of p7 and two distinct E2-specific products with different C termini. *J*. *Virol.* 68, 5063-5073.
31. Lu, H.-H. and Wimmer, E. (1996). Poliovirus chimeras replicating under the translational control of genetic elements of hepatitis C virus reveal unusual properties of the internal ribosomal entry site of hepatitis C virus. *Proc. Natl. Acad. Sci. USA* 93, 1412-1417.
32. McHutchison, J. G., Gordon, S. C., Schiff, E. R., Shiffman, M. L., Lee, W. M., Rustgi, V. K., Goodman, Z. D., Ling, M.-H., Cort, S. and Albrecht, J. K., for the hepatits interventional therapy group. (1998). Interferon alfa-2b alone or in combination with ribavirin as initial treatment for chronic hepatitis C. *N. Engl. J. Med.* 339, 1485-1492.
33. Mizushima, H., Hijikata, M., Asabe, S.-I., Hirota, M., Kimura, K. and Shimotohno, K. (1994). Two hepatitis C virus glycoprotein E2 products with different C termini. *J. Virol.* 68, 6215-6222.
34. Nakao, H., Okamoto, H., Tokita, H., Inoue, T., Iizuka, H., Pozzato, G. and Mishiro, S. (1996). Full-length genomic sequence of a hepatitis C virus genotype 2c isolate (BEBE1) and the 2c-specific PCR primers. *Arch. Virol.* 141, 701-704.
35. Nielsen, H., Engelbrecht, J., Brunak, S. and von Heijne, G. (1997). Identification of prokaryotic and eukaryotic signal peptides and prediction of their cleavage sites. *Protein Eng.* 10, 1-6.
36. Novak, J. E. and Kirkegaard, K. (1994). Coupling between genome translation and replication in an RNA virus. *Genes Dev.* 8, 1726-1737.
37. Nugent, C. I., Johnson, K. L., Sarnow, P. and Kirkegaard, K. (1999). Functional coupling between replication and packaging of poliovirus replicon RNA. *J*. *Virol.* 73, 427-435.
38. Okamoto, H., Kurai, K., Okada, S. I., Yamamoto, K., Iizuka, H., Tanaka, T., Fukuda, S., Tsuda, F. and Mishiro, S. (1992). Full-length sequence of hepatitis C virus genome having poor homology to reported isolates: comparative study of four distinct genotypes. *Virology* 188, 331-341.
39. Okamoto, H., Okada, S., Sugiyama, Y., Kurai, K., Iizuka, H., Machida, A., Miyakawa, Y. and
40. Mayumi, M. (1991). Nucleotide sequence of the genomic RNA of hepatitis C virus isolated from a human carrier: comparison with reported isolates for conserved and divergent regions. *J*. *Gen. Virol.* 72, 2697-2704.
41. Pletnev, A. G., Bray, M., Huggins, J. and Lai, C.-J. (1992). Construction and characterization of chimeric tick-borne encephalitis/dengue type 4 viruses. *Proc. Natl. Acad. Sci. USA* 89, 10532-10536.
42. Pletnev, A. G., Bray, M. and Lai, C.-J. (1993). Chimeric tick-borne encephalitis and dengue type 4 viruses: Effects of mutations on neurovirulence in mice. *J. Virol*. 67, 4956-4963.
43. Pletnev, A. G. and Men, R. (1998). Attenuation of the Langat tick-borne flavivirus by chimerization with mosquito-borne flavivirus dengue type 4. *Proc. Natl. Acad. Sci*. USA 95, 1746-1751.
44. Reynolds, J. E., Kaminski, A., Kettinen, H. J., Grace, K., Clarke, B. E., Carroll, A. R., Rowlands, D. J. and Jackson, R. J. (1995). Unique features of internal initiation of hepatitis C virus RNA translation. *EMBO J.* 14, 6010-6020.
45. Rice, C. M. (1996). Flaviviridae: The viruses and their replication, In "Fields Virology". (B. N. Fields, D. M. Knipe, P. M. Howley, et al., Eds.), Third ed., pp. 931-959. Lippincott-Raven Publishers, Philadelphia.
46. Robertson, B., Myers, G., Howard, C., Brettin, T., Bukh, J., Gaschen, B., Gojobori, T., Maertens, G., Mizokami, M., Nainan, O., Netesov, S., Nishioka, K., Shin-i, T., Simmonds, P., Smith, D., Stuyver, L. and Weiner, A. (1998). Classification, nomenclature, and database development for hepatitis C virus (HCV) and related viruses: proposals for standardization. *Arch. Virol.* 143, 2493-2503.
47. Selby, M. J., Glazer, E., Masiarz, F. and Houghton, M. (1994). Complex processing and protein:protein interactions in the E2:NS2 region of HCV. *Virology* 204, 114-122.
48. Simmonds, P., Holmes, E. C., Cha, T.-A., Chan, S.-W., McOmish, F., Irvine, B., Beall, E., Yap, P. L., Kolberg, J. and Urdea, M. S. (1993). Classification of hepatitis C virus into six major genotypes and a series of subtypes by phylogenetic analysis of the NS-5 region. *J*. *Gen. Virol.* 74, 2391-2399.
49. Tanaka, T., Kato, N., Cho, M.-J. and Shimotohno, K. (1995). A novel sequence found at the 3' terminus of hepatitis C virus genome. *Biochem. Biophys. Res. Commun.* 215, 744-749.
50. Tanaka, T., Kato, N., Cho, M.-J., Sugiyama, K. and Shimotohno, K. (1996). Structure of the 3' terminus of the hepatitis C virus genome. *J*. *Virol.* 70, 3307-3312.
51. Tsuchihara, K., et al. (1997) J. Virol. 71, 6720-6726.
52. Tsukiyama-Kohara, K., et al. (1992) J. Virol. 66, 1476-1483.
53. Vassilev, V. B., Collett, M. S. and Donis, R. O. (1997). Authentic and chimeric full-length genomic cDNA clones of bovine viral diarrhea virus that yield infectious transcripts. *J*. *Virol.* 71, 471-478.
54. Weiner, A. J., Brauer, M. J., Rosenblatt, J., Richman, K. H., Tung, J., Crawford, K., Bonino, F., Saracco, G., Choo, Q.-L., Houghton, M. and Han, J. H. (1991). Variable and hypervariable domains are found in the regions of HCV corresponding to the Flavivirus envelope and NS1 proteins and the Pestivirus envelope glycoproteins. *Virology* 180, 842-848.
55. World Health Organization (1997). Hepatitis C. *Weekly Epidemiol. Rec.* 72, 65-72.
56. Yamada, N., Tanihara, K., Takada, A., Yorihuzi, T., Tsutsumi, M., Shimomura, H., Tsuji, T. and Date, T. (1996). Genetic organization and diversity of the 3' noncoding region of the hepatitis C virus genome. *Virology* 223, 255-261.
57. Yanagi, M., Bukh, J., Emerson, S. U. and Purcell, R. H. (1996). Contamination of commercially available fetal bovine sera vith bovine viral diarrhea virus genomes: implications for the study of hepatitis C virus in cell cultures. *J*. *Infect. Dis.* 174, 1324-1327.
58. Yanagi, M., Purcell, R. H., Emerson, S. U. and Bukh, J. (1997). Transcripts from a single full-length cDNA clone of hepatitis C virus are infectious when directly transfected into the liver of a chimpanzee.. *Proc. Natl. Acad. Sci. USA* 94, 8738-8743.
59. Yanagi, M., St. Claire, M., Shapiro, M., Emerson, S. U., Purcell, R. H. and Bukh, J. (1998). Transcripts of a chimeric cDNA clone of hepatitis C virus genotype 1b are infectious *in vivo. Virology* 244, 161-172.
60. Yanagi, M., St. Claire, M., Emerson, S. U., Purcell, R. H. and Bukh, J. (1999). *In vivo* analysis of the 3' untranslated region of hepatitis C virus after *in vitro* mutagenesis of an infectious cDNA clone. *Proc. Natl. Acad. Sci. USA* 96, 2291-2295.
61. Yoo, B. J., et al. (1995). J. Virol. 69, 32-38.
62. Zhao, W. D., Wimmer, E. and Lahser, F. C. (1999). Poliovirus/hepatitis C virus (internal ribosomal entry site-core) chimeric viruses: improved growth properties through modification of a proteolytic cleavage site and requirement for core RNA sequences but not for core-related polypeptides. *J*. *Virol.* 73, 1546-1554.

## Claims

1. A purified and isolated nucleic acid molecule encoding the genome of a human hepatitis C virus of genotype 2a quasispecies , said molecule capable of expressing said virus when transfected into cells and further capable of infectivity in vivo, wherein said molecule encodes the amino acid sequence SEQ ID NO: 2 or encodes an amino acid sequence that differs from that of SEQ ID NO: 2 by less than 2.2% at the amino acid level.

2. The nucleic acid molecule of claim 1, wherein said molecule comprises the nucleic acid sequence of SEQ ID NO: 1 or comprises a nucleic acid sequence that differs from that of SEQ ID NO: 1 from nucleotide 341 to 9439, which corresponds to the ORF, by less than 4.1% at the nucleotide level.

3. A DNA construct comprising a nucleic acid molecule according to claims 1-2.

4. An RNA transcript of the DNA construct of claim 3.

5. An *in vitro* cell transfected with the DNA construct of claim 3.

6. An *in vitro* cell transfected with RNA transcript of claim 4

7. A hepatitis C virus polypeptide produced by the cell of claim 5.

8. A hepatitis C virus polypeptide produced by the cell of claim 6.

9. A hepatitis C virus produced by the cell of claim 5.

10. A hepatitis C virus produced by the cell of claim 6.

11. A hepatitis C virus whose genome comprises a nucleic acid molecule according to anyone of claims 1-2.

12. A method for producing a hepatitis C virus comprising transfecting a host cell with the RNA transcript of claim 4.

13. A polypeptide encoded by the nucleic acid sequence according to claims 1-2

14. The polypeptide of claim 13, wherein said polypeptide is selected from the group consisting of NS3 protease, E1 protein, E2 protein or NS4 protein.

15. A method for assaying candidate antiviral agents for activity against HCV, comprising:
a) exposing a cell containing the hepatitis C virus of claim 11 to the candidate antiviral agent; and
b) measuring the presence or absence of hepatitis C virus replication in the cell of step (a).

16. The method of claim 15, wherein said replication in step (b) is measured by at least one of the following: negative strand RT-PCR, quantitative RT-PCR, Western blot, immunofluorescence, or infectivity in a susceptible animal.

17. A method for assaying candidate antiviral agents for activity against HCV, comprising:
a) exposing an HCV protease encoded by a nucleic acid sequence according to anyone of claims 1-2 or a fragment thereof to the candidate antiviral agent in the presence of a protease substrate; and
b) measuring the protease activity of said protease.

18. The method of claim 17, wherein said HCV protease is selected from the group consisting of an NS3 domain protease, an NS3-NS4A fusion polypeptide, or an NS2-NS3 protease.

19. Antibody to the polypeptide of claims 13-14.

20. Antibody to the hepatitis C virus of claims 9-11.

21. A method for determining the susceptibility of cells in vitro to support HCV infection, comprising the steps of:
a) growing animal cells in vitro;
b) transfecting into said cells the nucleic acid of claims 1-2-; and
c) determining if said cells show indicia of HCV replication.

22. The method according to claim 21, wherein said cells are human cells.

23. A composition comprising the polypeptide of claims 13-14 suspended in a suitable amount of a pharmaceutically acceptable diluent or excipient.

24. A composition comprising a nucleic acid molecule of claims 1-2 suspended in a suitable amount of a pharmaceutically acceptable diluent or excipient.

## Patentansprüche

1. Gereinigtes und isoliertes Nukleinsäuremolekül, kodierend das Genom eines menschlichen Hepatitis C-Virus der Genotyp 2a Quasispecies, wobei das Molekül dazu in der Lage ist, das Virus zu exprimieren, wenn es in Zellen transfiziert wird und weiterhin zu einer Infektivität in vivo in der Lage ist, wobei das Molekül die Aminosäuresequenz SEQ ID NO. 2 kodiert oder eine Aminosäuresequenz kodiert, die sich von der von SEQ ID NO. 2 um weniger als 2,2% auf dem Aminosäureniveau unterscheidet.

2. Nukleinsäuremolekül gemäß Anspruch 1, wobei das Molekül die Nukleinsäuresequenz von SEQ ID NO. 1 umfasst oder eine Nukleinsäuresequenz umfasst, die sich von der von SEQ ID NO. 1 von Nukleotid 341 bis 9439 unterscheidet, was zu dem offenen Leserahmen korrespondiert, und zwar um weniger als 4,1% auf Nukleotidniveau.

3. DNA-Konstrukt, umfassend ein Nukleinsäuremolekül gemäß einem der Ansprüche 1 und 2.

4. RNA-Transkript des DNA-Konstrukts von Anspruch 3.

5. In vitro-Zelle, transfiziert mit dem DNA-Konstrukt von Anspruch 3.

6. In vitro-Zelle, transfiziert mit dem RNA-Transkript von Anspruch 4.

7. Hepatitis C-Virus-Polypeptid, erzeugt durch die Zelle von Anspruch 5.

8. Hepatitis C-Virus-Polypeptid, erzeugt durch die Zelle von Anspruch 6.

9. Hepatitis C-Virus, erzeugt durch die Zelle von Anspruch 5.

10. Hepatitis C-Virus, erzeugt durch die Zelle von Anspruch 6.

11. Hepatitis C-Virus, dessen Genom ein Nukleinsäuremolekül gemäß einem der Ansprüche 1 und 2 umfasst.

12. Verfahren zur Erzeugung eines Hepatitis C-Virus, umfassend die Transfektion einer Wirtszelle mit dem RNA-Transkript von Anspruch 4.

13. Polypeptid, kodiert durch die Nukleinsäuresequenz gemäß den Ansprüchen 1 bis 2.

14. Polypeptid gemäß Anspruch 13, wobei das Polypeptid gewählt ist aus der Gruppe bestehend aus NS3-Protease, E1-Protein, E2-Protein oder NS4-Protein.

15. Verfahren zum Überprüfen von antiviralen Kandidatenmitteln im Hinblick auf eine Aktivität gegen HCV, umfassend:
a) Exponieren einer Zelle, enthaltend das Hepatitis C-Virus von Anspruch 11 gegenüber dem antiviralen Kandidatenmittel und
b) Messen der Gegenwart oder Abwesenheit der Hepatitis C-Virus-Applikation in der Zelle von Schritt (a).

16. Verfahren gemäß Anspruch 15, wobei die Replikation in Schritt (b) durch mindestens eines der Folgenden gemessen wird: Negativstrang-RT-PCR, quantitative RT-PCR, Western Blot, Immunfluoreszenz oder Infektivität in einem empfänglichen Tier.

17. Verfahren für einen Assay auf antivirale Kandidatenmittel im Hinblick auf eine Aktivität gegen HCV, umfassend:
a) Exponieren einer HCV-Protease, kodiert durch eine Nukleinsäuresequenz gemäß einem der Ansprüche 1 bis 2 oder einem Fragment davon gegenüber dem antiviralen Kandidatenmittel in Gegenwart eines Proteasesubstrats und
b) Messung der Proteaseaktivität der Protease.

18. Verfahren gemäß Anspruch 17, wobei die HCV-Protease gewählt wird aus der Gruppe, bestehend aus einer NS3-Domänenprotease, einem NS3-NS4A-Fusionspolypeptid oder einer NS2-NS3-Protease.

19. Antikörper gegen das Polypeptid der Ansprüche 13 bis 14.

20. Antikörper gegen das Hepatitis C-Virus der Ansprüche 9 bis 11.

21. Verfahren zur Bestimmung der Empfänglichkeit von Zellen in vitro, um eine HCV-Infektion zu unterstützen, umfassend die folgenden Schritte:
a) Züchten von tierischen Zellen in vitro;
b) Transfizieren der Zellen mit der Nukleinsäure der Ansprüche 1 bis 2; und
c) Bestimmung, ob die Zellen Anzeichen einer HCV-Replikation zeigen.

22. Verfahren gemäß Anspruch 21, wobei die Zellen menschliche Zellen sind.

23. Zusammensetzung, umfassend das Polypeptid der Ansprüche 13 bis 14, suspendiert in einer geeigneten Menge eines pharmazeutisch annehmbaren Verdünnungsmittels oder Exzipiens.

24. Zusammensetzung, umfassend ein Nukleinsäuremolekül der Ansprüche 1 bis 2, suspendiert in einer geeigneten Menge eines pharmazeutisch annehmbaren Verdünnungsmittels oder Exzipiens.

## Revendications

1. Molécule d'acide nucléique purifié et isolée codant pour le génome du virus de l'hépatite C humaine de la quasi-espèce du génotype 2a, ladite molécule étant capable d'exprimer ledit virus quand elle est transfectée dans des cellules et étant de plus capable d'infectivité in vivo, où ladite molécule code pour la séquence d'acides aminés SEQ ID NO:2 ou code pour une séquence d'acides aminés qui diffère de celle de SEQ ID NO: 2 de moins de 2,2% au niveau des acides aminés.

2. Molécule d'acide nucléique de la revendication 1, où ladite molécule comprend la séquence d'acide nucléique de SEQ ID N0:1 ou comprend une séquence d'acide nucléique qui diffère de celle de SEQ ID NO:1 du nucléotide 341 à 9439, qui correspond à ORF, de moins de 4,1% au niveau des nucléotides.

3. Construction d'ADN comprenant la molécule d'acide nucléique selon les revendications 1-2.

4. Transcription d'ARN de la construction d'ADN de la revendication 3.

5. Cellule *in vitro* transfectée par la construction d'ADN de la revendication 3.

6. Cellule *in vitro* transfectée par la transcription d'ARN de la revendication 4.

7. Polypeptide du virus de l'hépatite C produit par la cellule de la revendication 5.

8. Polypeptide du virus de l'hépatite C produit par la cellule de la revendication 6.

9. Virus de l'hépatite C produit par la cellule de la revendication 5.

10. Virus de l'hépatite C produit par la cellule de la revendication 6.

11. Virus de l'hépatite C dont le génome comprend une molécule d'acide nucléique selon l'une quelconque des revendications 1-2.

12. Méthode de production d'un virus de l'hépatite C comprenant la transfection d'une cellule hôte avec la transcription d'ARN de la revendication 4.

13. Polypeptide codé par la séquence d'acide nucléique selon les revendications 1-2.

14. Polypeptide de la revendication 13 où ledit polypeptide est sélectionné dans le groupe consistant en protéase de NS3, protéine E1, protéine de E2 ou protéine NS4.

15. Méthode pour analyser des agents candidats antiviraux pour une activité contre VHC, comprenant:
a) l'exposition d'une cellule contenant le virus de l'hépatite C de la revendication 11 à l'agent antiviral candidat; et
b) la mesure de la présence ou de l'absence de la réplication du virus de l'hépatite C dans la cellule de l'étape (a).

16. Méthode de la revendication 15, où ladite réplication à l'étape (b) est mesurée par au moins l'un de ce qui suit: RT-PCR brin négatif, RT-PCR quantitative, Western blot, immunofluorescence, ou infectivité chez un mammifère sensible.

17. Méthode pour analyser des agents antiviraux candidats pour une activité contre VHC, comprenant:
a) l'exposition d'une protéase de VHC codée par une séquence d'acide nucléique selon l'une quelconque des revendications 1-2 ou son fragment à l'agent antiviral candidat en présence d'un substrat de protéase; et
b) la mesure de l'activité de protéase de ladite protéase.

18. Méthode de la revendication 17, où ladite protéase de VHC est sélectionnée dans le groupe consistant en une protéase du domaine NS3, un polypeptide de fusion NS3-NS4A ou une protéase NS2-NS3.

19. Anticorps au polypeptide des revendications 13-14.

20. Anticorps au virus de l'hépatite C des revendications 9-11.

21. Méthode pour déterminer la sensibilité de cellules in vitro pour supporter une infection par VHC, comprenant les étapes de:
a) croissance de cellules animales in vitro;
b) transfection dans lesdites cellules de l'acide nucléique des revendications 1-2; et
c) détermination du fait que lesdites cellules montrent des indices de réplication de VHC.

22. Méthode selon la revendication 21, où lesdites cellules sont des cellules humaines.

23. Composition comprenant le polypeptide de la revendication 13-14 en suspension dans une quantité appropriée d'un diluant ou excipient pharmaceutiquement acceptable.

24. Composition comprenant une molécule d'acide nucléique des revendications 1-2 en suspension dans une quantité appropriée d'un diluant ou excipient pharmaceutiquement acceptable.
